(19) Europäisches Patentamt
European Patent Office
Office européen des brevets

(11) EP 2 868 197 A1

(12) **EUROPEAN PATENT APPLICATION**

(43) Date of publication:
**06.05.2015 Bulletin 2015/19**

(51) Int Cl.:
***A01N 43/84** (2006.01)* ***C07D 413/04** (2006.01)*

(21) Application number: **14190204.9**

(22) Date of filing: **24.10.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **05.11.2013 EP 13191534**

(71) Applicant: **BASF SE**
**67056 Ludwigshafen (DE)**

(72) Inventors:
- **Witschel, Matthias**
  **67098 Bad Dürkheim (DE)**
- **Simon, Anja**
  **69469 Weinheim (DE)**
- **Steinbrenner, Ulrich**
  **67435 Neustadt (DE)**
- **Chiodo, Tiziana**
  **68159 Mannheim (DE)**
- **Maywald, Volker**
  **67071 Ludwigshafen (DE)**

(54) **Herbicidal compositions**

(57) The present invention relates to compositions comprising the crystalline form B of 1,5-dimethyl-6-thioxo-3-(2,2,7-trifluoro-3-oxo-4-(prop-2-ynyl)-3,4-dihydro-2H-benzo[b][1,4]oxazin-6-yl)-1,3,5-triazinane-2,4-dione, and at least one further active compound selected from herbicides B and safeners C; and their use as herbicides, i.e. for controlling harmful plants, and also a method for controlling unwanted vegetation.

EP 2 868 197 A1

## Description

The present invention relates to herbicidal compositions comprising the crystalline form B of 1,5-dimethyl-6-thioxo-3-(2,2,7-trifluoro-3-oxo-4-(prop-2-ynyl)-3,4-dihydro-2H-benzo[b][1,4]oxazin-6-yl)-1,3,5-triazinane-2,4-dione and at least one further compound selected from herbicides B and safeners C.

In the case of crop protection compositions, it is desirable in principle to increase the specific activity of an active compound and the reliability of the effect. It is particularly desirable for the crop protection composition to control the harmful plants effectively, but at the same time to be compatible with the useful plants in question. Also desirable is a broad spectrum of activity allowing the simultaneous control of harmful plants. Frequently, this cannot be achieved using a single herbicidally active compound.

With many highly effective herbicides, there is the problem that their compatibility with useful plants, in particular dicotyledonous crop plants, such as cotton, oilseed rape and graminaceous plants, such as barley, millet, corn, rice, wheat and sugar cane, is not always satisfactory, i.e. in addition to the harmful plants, the crop plants, too, are damaged on a scale which cannot be tolerated. By reducing the application rates, the useful plants are spared; however, naturally, the extent of the control of harmful plants decreases, too.

Frequently, it is a problem that herbicides can only be applied within a narrow time frame in order to achieve the desired herbicidal action, which time frame may be unpredictably influenced by weather conditions.

It is known that special combinations of different specifically active herbicides result in enhanced activity of an herbicide component in the sense of a synergistic effect. In this manner, it is possible to reduce the application rates of herbicidally active compounds required for controlling the harmful plants.

Furthermore, it is known that in some cases joint application of specifically acting herbicides with organic active compounds, some of which may also have herbicidal activity, allows better crop plant compatibility to be achieved. In these cases, the active compounds act as antidotes or antagonists and are also referred to as safeners, since they reduce or even prevent damage to the crop plants.

1,5-dimethyl-6-thioxo-3-(2,2,7-trifluoro-3-oxo-4-(prop-2-ynyl)-3,4-dihydro-2H-benzo[b][1,4]oxazin-6-yl)-1,3,5-triazinane-2,4-dione is the herbicidal active substance of the formula I:

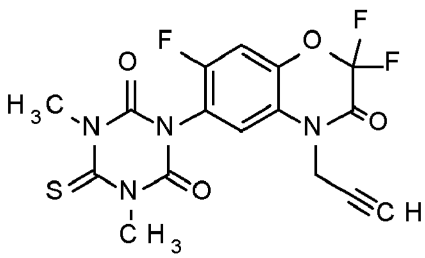

(I)

1,5-dimethyl-6-thioxo-3-(2,2,7-trifluoro-3-oxo-4-(prop-2-ynyl)-3,4-dihydro-2H-benzo[b][1,4]oxazin-6-yl)-1,3,5-triazinane-2,4-dione, which hereinafter is also termed as benzoxazinone (I), and a process for its production are known from WO 2010/145992. This process yields benzoxazinone (I) as an amorphous solid.

By applying suitable crystallization conditions crystalline, stable modification of 1,5-dimethyl-6-thioxo-3-(2,2,7-trifluoro-3-oxo-4-(prop-2-ynyl)-3,4-dihydro-2H-benzo[b][1,4]oxazin-6-yl)-1,3,5-triazinane-2,4-dione can be prepared, which does not display the disadvantages of the amorphous benzoxazinone (I). This crystalline form is hereinafter described and termed "the crystalline form B of benzoxazinone (I)" or "form B".

The form B according to the invention can be identified by X-ray powder diffractometry on the basis of its diffraction diagram. Thus an X-ray powder diffraction diagram of form B recorded using Cu-K$\alpha$ radiation (1.54178 Å) at 25 °C shows at least 3, often at least 5, in particular at least 7, and especially all of the reflections quoted in the following table as 2$\theta$ values or as interplanar spacings d:

| 2$\theta$ values | d [Å] |
|---|---|
| 9,0$\pm$0,2° | 9,85 |
| 10,9$\pm$0,2° | 8,10 |
| 11,5$\pm$0,2° | 7,69 |
| 12,9$\pm$0,2° | 6,87 |
| 13,5$\pm$0,2° | 6,56 |

(continued)

| 2θ values | d [Å] |
|---|---|
| 14,9±0,2° | 5,96 |
| 16,4±0,2° | 5,42 |
| 16,5±0,2° | 5,36 |
| 17,5±0,2° | 5,06 |
| 20,3±0,2° | 4,39 |

It is an object of the present invention to provide herbicidal compositions which are highly active against unwanted harmful plants. At the same time, the compositions should have good compatibility with useful plants. In addition, the compositions according to the invention should have a broad spectrum of activity.

This and further objects are achieved by the herbicidal compositions below.

Accordingly, the present invention relates to herbicidal compositions comprising

A) the crystalline form B of benzoxazinone (I);
and at least one further active compound selected from

B) herbicides of class b1) to b15):

- b1) lipid biosynthesis inhibitors;
- b2) acetolactate synthase inhibitors (ALS inhibitors);
- b3) photosynthesis inhibitors;
- b4) protoporphyrinogen-IX oxidase inhibitors,
- b5) bleacher herbicides;
- b6) enolpyruvyl shikimate 3-phosphate synthase inhibitors (EPSP inhibitors);
- b7) glutamine synthetase inhibitors;
- b8) 7,8-dihydropteroate synthase inhibitors (DHP inhibitors);
- b9) mitose inhibitors;
- b10) inhibitors of the synthesis of very long chain fatty acids (VLCFA inhibitors);
- b11) cellulose biosynthesis inhibitors;
- b12) decoupler herbicides;
- b13) auxin herbicides;
- b14) auxin transport inhibitors; and
- b15) other herbicides selected from the group consisting of bromobutide, chlorflurenol, chlorflurenol-methyl, cinmethylin, cumyluron, dalapon, dazomet, difenzoquat, difenzoquat-metilsulfate, dimethipin, DSMA, dymron, endothal and its salts, etobenzanid, flamprop, flamprop-isopropyl, flamprop-methyl, flamprop-M-isopropyl, flamprop-M-methyl, flurenol, flurenol-butyl, flurprimidol, fosamine, fosamine-ammonium, indanofan, indaziflam, maleic hydrazide, mefluidide, metam, methyl azide, methyl bromide, methyl-dymron, methyl iodide, MSMA, oleic acid, oxaziclomefone, pelargonic acid, pyributicarb, quinoclamine, triaziflam, tridiphane and 6-chloro-3-(2-cyclopropyl-6-methylphenoxy)-4-pyridazinol (CAS 499223-49-3) and its salts and esters;

and

C) safeners;
including the agriculturally acceptable salts or derivatives of herbicides B and safeners C having a carboxyl group.

The invention relates in particular to compositions in the form of herbicidal active crop protection compositions comprising a herbicidally effective amount of an active compound combination comprising the crystalline form B of benzoxazinone (I) and at least one further compound selected from the herbicides B and the safeners C, as defined above, and also at least one liquid and/or solid carrier and/or one or more surfactants and, if desired, one or more further auxiliaries customary for crop protection compositions.

The invention also relates to compositions in the form of a crop protection composition formulated as a 1-component composition comprising an active compound combination comprising the crystalline form B of benzoxazinone (I) and at least one further active compound selected from the herbicides B and the safeners C, and at least one solid

or liquid carrier and/or one or more surfactants and, if desired, one or more further auxiliaries customary for crop protection compositions.

The invention also relates to compositions in the form of a crop protection composition formulated as a 2-component composition comprising a first component comprising the crystalline form B of benzoxazinone (I), a solid or liquid carrier and/or one or more surfactants, and a second component comprising at least one further active compound selected from the herbicides B and safeners C, a solid or liquid carrier and/or one or more surfactants, where additionally both components may also comprise further auxiliaries customary for crop protection compositions.

Surprisingly, the compositions according to the invention comprising the crystalline form B of benzoxazinone (I) and at least one herbicide B have better herbicidal activity, i.e. better activity against harmful plants, than would have been expected based on the herbicidal activity observed for the individual compounds, or a broader activity spectrum. The herbicidal activity to be expected for mixtures based on the individual compound can be calculated using Colby's formula (see below). If the activity observed exceeds the expected additive activity of the individual compounds, synergism is said to be present.

Moreover, the time frame, within which the desired herbicidal action can be achieved, may be expanded by the compositions according to the invention comprising the crystalline form B of benzoxazinone (I) and at least one herbicide B and optionally a safener C. This allows a more flexibly timed application of the compositons according to the present invention in comparison with the single compounds.

The compositions according to the invention comprising both the crystalline form B of benzoxazinone (I) and at least one of the safeners mentioned under C also have good herbicidal activity against harmful plants and better compatibility with useful plants.

Surprisingly, the compositions according to the invention comprising the crystalline form B of benzoxazinone (I), at least one herbicide B and at least one of the safeners mentioned under C have better herbicidal activity, i.e. better activity against harmful plants, than would have been expected based on the herbicidal activity observed for the individual compounds, or a broader activity spectrum, and show better compatibility with useful plants than compositions comprising only the crystalline form B of benzoxazinone (I) and one herbicide B.

The invention furthermore relates to a method for controlling unwanted vegetation, in particular where crop plants are cultivated.

The invention also relates to a method for the desiccation or defoliation of plants.

As used herein, the terms "controlling" and "combating" are synonyms.
As used herein, the terms "undesirable vegetation" and "harmful plants" are synonyms.

Further embodiments of the present invention are evident from the claims, the description and the examples. It is to be understood that the features mentioned above and still to be illustrated below of the subject matter of the invention can be applied not only in the combination given in each particular case but also in other combinations, without leaving the scope of the invention.

For the production of active substances on the industrial scale but also for the formulation of active substances, in many cases knowledge concerning the possible existence of crystalline modifications (also described as crystalline forms) or of solvates of the active substance in question, and knowledge of the specific properties of such modifications and solvates and of methods for their preparation are of decisive importance. A range of active substances can exist in different crystalline but also in amorphous modifications. Polymorphism is the term used in these cases. A polymorph is a solid, crystalline phase of a compound which is characterized by a specific, uniform packing and arrangement of the molecules in the solid.

Different modifications of one and the same active substance can sometimes have different properties, for example differences in the following properties: solubility, vapor pressure, dissolution rate, stability against a phase change into a different modification, stability during grinding, suspension stability, optical and mechanical properties, hygroscopicity, crystal form and size, filterability, density, melting point, stability to decomposition, color and sometimes even chemical reactivity or biological activity.

The applicant's own attempts to convert benzoxazinone (I) into a crystalline solid by crystallization at first resulted in amorphous products or in complex mixtures of different crystal modifications, which could only be handled with difficulty and whose stability against uncontrolled phase change was unsatisfactory.

By applying suitable crystallization conditions crystalline, stable modification of 1,5-dimethyl-6-thioxo-3-(2,2,7-trifluoro-3-oxo-4-(prop-2-ynyl)-3,4-dihydro-2H-benzo[b][1,4]oxazin-6-yl)-1,3,5-triazinane-2,4-dione can be prepared, which does not display the disadvantages of the amorphous benzoxazinone (I). This crystalline form is hereinafter described and termed "the crystalline form B of benzoxazinone (I)" or "form B".

In addition, the crystalline form B according to the invention is easier to handle than the previously known amorphous benzoxazinone (I), since during production they are obtained in the form of discrete crystals. Compared to mixtures of different crystal modifications of benzoxazinone (I), the pure form B displays increased stability with regard to conversion into another modification. The term "pure form B" should be understood to mean that the proportion of the form B, based on the total quantity of benzoxazinone (I), is at least 90 wt.% and in particular at least 95 wt.%.

Accordingly, a first object of the present invention relates to the crystalline form B of benzoxazinone (I). Also an object is a benzoxazinone (I), which at least 90 wt.% in particular at least 95 % consists of the crystalline form B.

The form B according to the invention can be identified by X-ray powder diffractometry on the basis of its diffraction diagram. Thus an X-ray powder diffraction diagram of form B recorded using Cu-Kα radiation (1.54178 Å) at 25 °C shows at least 3, often at least 5, in particular at least 7, and especially all of the reflections quoted in the following table as 2θ values or as interplanar spacings d:

| 2θ values | d [Å] |
|---|---|
| 9,0±0,2° | 9,85 |
| 10,9±0,2° | 8,10 |
| 11,5±0,2° | 7,69 |
| 12,9±0,2° | 6,87 |
| 13,5±0,2° | 6,56 |
| 14,9±0,2° | 5,96 |
| 16,4±0,2° | 5,42 |
| 16,5±0,2° | 5,36 |
| 17,5±0,2° | 5,06 |
| 20,3±0,2° | 4,39 |

Form B displays a thermogram with a characteristic melting peak in the range from 190 to 220°C. The melting point, determined as the onset of the melting peak, typically lies in the range from about 200°C to 210°C, in particular in the range from 203 to 208°C. The melting enthalpy is preferably in the range from 30 to 40 J/g. The values quoted here relate to values determined by differential calorimetry (differential scanning calorimetry: DSC, aluminum closed and vented cup, nitrogen flow 150 ml/min, heating rate 5 K/min).

The production of the modification B can be principally effected by running the crystallization at temperatures exceeding 60°C, in particular at temperatures of at least 80°C or at least 90°C, e.g. from 80 to 130°C or from 90 to 120°C.

Form B can bee obtained e.g. by crystallization from a solution or slurry of benzoxazinone (I) in an organic solvent selected from toluene, monochlorobenzen or dichlorobenzene at temperatures of at least 80°C or at least 90°C, e.g. from 80 to 130°C or from 90 to 120°C.

Form B can bee obtained e.g. by crystallization from a slurry of benzoxazinone (I) in a mixture of water and a water-miscible solvent, selected from $C_1$-$C_3$-alkanols, in particular methanol or isopropanol, $C_2$-$C_4$-alkandiols, such as 1,3-propanediol, $C_1$-$C_4$-dialkylketones, such as acetone and cyclic ethers having preferably 4 to 6 carbon atoms and 1 or 2 oxygen atoms such as tetra-hydrofurane and 1,4-dioxane at temperatures of at least 80°C or at least 90°C, e.g. from 80 to 130°C or from 90 to 120°C. Apart from that crystallization from a slurry of benzoxazinone (I) to obtain form B can be performed by analogy to the crystallization of form A, in particular regarding preparation of the slurry, concentrations and measures of effecting crystallization, provided that crystallization is effected in the above temperature range.

Pure form B is also obtained by heating the crystalline benzoxazinone (I), e.g. form A of benzoxazinone (I) or mixtures of forms A + B + C to temperatures of at least 160°C, in particular at least 170°C, e.g. temperatures in the range from 160°C to 210°C or in the range from 170 to 200°C.

In order to obtain form B of benzoxazinone (I), the crystallization is effected at temperatures of at least 80°C or at least 90°C, e.g. from 80 to 130°C or from 90 to 120°C. Crystallization of form B is preferably effected under controlled conditions, i.e. the conditions of the crystallization are chosen to achieve a slow crystallization rate.

For this, in a first step i) a solution or slurry of benzoxazinone (I) in one of the aforesaid solvents or solvent mixtures is prepared, and then in a second step ii) crystallization of the benzoxazinone (I) is effected at temperatures of at least 80°C or at least 90°C, e.g. from 80 to 130°C or from 90 to 120°C.

The concentration of benzoxazinone (I) in the solution or slurry used for the crystallization naturally depends on the nature of the solvent and the dissolution temperature and often lies in the range from 100 to 800 g/l. Suitable conditions can be determined by the person skilled in the art by routine experiments.

Preferably the solution or slurry used for the crystallization contains benzoxazinone (I) in a purity of at least 85%, often at least 90%, in particular at least 95%, i.e. the content of organic impurities which are not organic solvents is not more than 15 wt.%, often not more than 10 wt.%, and in particular not more than 5 wt.%, based on the benzoxazinone (I) present dissolved in the solvent.

The solution or slurry used for the crystallization is preferably essentially free from solvents other than those stated. In this context, "essentially free" means that the concentration of other solvents in the benzoxazinone (I) containing solution or slurry does not exceed 10 wt.%, often 5 wt.%, based on the total quantity of solvent.

The solution of benzoxazinone (I) can for example be prepared by the following methods:

(1) Dissolution of the benzoxazinone (I), preferably in a form different from form B, in one of the aforesaid polar organic solvents, or

(2) Preparation of the benzoxazinone (I) by a chemical reaction and transfer of the reaction mixture, if necessary after removal of reagents and/or side products, into an organic solvent suitable according to the invention.

For the preparation of the solution by dissolution of the benzoxazinone (I), essentially any known form of benzoxazinone (I) can be used. Often amorphous benzoxazinone (I) or a mixture of different crystalline modifications or a mixture of amorphous and crystalline benzoxazinone (I) will be used. Also suitable are other crystalline forms of benzoxazinone (I) and mixtures thereof, for example the form A described below and the form C also described below, not according to the invention, and mixtures of these forms as well as mixtures of form B with form A or form C of benzoxazinone (I).

The dissolution of the benzoxazinone (I) is usually effected at temperatures in the range from 85 to 200°C, in particular from 90 to 150°C.

The solution of the benzoxazinone (I) can also be prepared by transferring a reaction mixture obtained by a chemical reaction, which contains the benzoxazinone (I), if necessary after removal of reagents and/or side products, into an organic solvent suitable according to the invention. This can be effected in such a manner that the reaction is performed in an organic solvent or solvent mixture which consists at least partly, preferably at least 50 wt.%, of a solvent suitable for the crystallization and, if necessary a workup is performed during which excess reagents and any catalysts present and any unsuitable solvents present, for example water and/or methanol, are removed. The preparation of a solution of the benzoxazinone (I) by chemical reaction of a suitable precursor of benzoxazinone (I) can be effected by analogy to the methods which are described in the state of the art cited at the beginning, to which full reference is hereby made.

For the preparation of a slurry of the benzoxazinone (I), essentially any known form of benzoxazinone (I) can be used. Of course, in the preparation of form B usually a form of benzoxazinone (I) which is different from pure form B. However, benzoxazinone (I) may be used in a form already containing form B, thereby achieving a form B having a higher content of form B. Often a mixture of different crystalline modifications or a mixture of amorphous and crystalline benzoxazinone (I) will be used. Also suitable are other crystalline forms of benzoxazinone (I) and mixtures thereof, for example the form A described below and the form C also described below, not according to the invention, and mixtures of these forms as well as mixtures of form B with form A and/or form C of benzoxazinone (I).

The crystallization of form B of benzoxazinone (I) can be effected as follows, for example

- by cooling of a hot saturated solution or slurry which contains the dissolved or suspended benzoxazinone (I), to a temperature in the range from 80 to 100°C
- by concentration of a hot saturated solution or slurry which contains the dissolved or dispersed benzoxazinone (I), or
- by a combination of the aforesaid measures.

The crystallization is as a rule carried out until at least 80 wt.%, preferably at least 90 wt.%, of the benzoxazinone (I) used crystallizes out.

If the crystallization of form B is effected by cooling, the cooling rate is preferably less than 10 K/min.

The crystallization of form B can be promoted or accelerated by seeding with seed crystals of form B, for example by adding seed crystals of form B before or during the crystallization.

If seed crystals are added during the crystallization, the quantity thereof is typically 0.001 to 10 wt.%, often 0.005 to 5 wt.%, in particular 0.01 to 1 wt.% and especially 0.05 to 0.5 wt.%, based on the dissolved benzoxazinone (I).

If the crystallization is performed in the presence of seed crystals of form B, these are preferably only added at a temperature at which the saturation concentration of the benzoxazinone (I) in the solvent in question has been reached, i.e. at or below that temperature at which the dissolved quantity of benzoxazinone (I) forms a saturated solution in the solvent in question. The person skilled in the art can determine the temperature dependence of the saturation concentration in a solvent in routine experiments.

The isolation of the form B from the crystallization product, i.e. the separation of the form B from the mother liquor, is effected by usual techniques for the separation of solid components from liquids, for example by filtration, centrifugation or by decantation. As a rule, the isolated solid will be washed, for example with the solvent used for the crystallization, with water or with a mixture of the organic solvent used for the crystallization with water. The washing

can be effected in one or more steps, washing with water often being used in the last washing step. The washing is typically effected at temperatures below 30°C, often below 25°C and in particular below 20°C, in order to keep the loss of valuable product as small as possible. Next, the form B obtained can be dried and then supplied for further processing. Often, however, the moist active substance obtained after washing, in particular an active substance moist with water, will be supplied directly for the further processing.

By means of the crystallization according to the invention, form B is obtained with a benzoxazinone (I) content of as a rule at least 90 wt.%, often 94 wt.%, in particular at least 96 wt.%. The content of form B, based on the total quantity of benzoxazinone (I), is typically at least 90% and often at least 95 % or at least 96%.

Therefore, a particular embodiment of the invention relates to 1,5-dimethyl-6-thioxo-3-(2,2,7-trifluoro-3-oxo-4-(prop-2-ynyl)-3,4-dihydro-2H-benzo[b][1,4]oxazin-6-yl)-1,3,5-triazinane-2,4-dione, which consists of at least 90 wt.% and often at least 95 % or at least 96% of the crystalline form B.

The crystalline form B may be mixed with other forms of 1,5-dimethyl-6-thioxo-3-(2,2,7-trifluoro-3-oxo-4-(prop-2-ynyl)-3,4-dihydro-2H-benzo[b][1,4]oxazin-6-yl)-1,3,5-triazinane-2,4-dione, e.g. form B and/or form C, without loosing the benefits achieved by form B. Therefore, the invention also relates to a mixture of the crystalline form B of 1,5-dimethyl-6-thioxo-3-(2,2,7-trifluoro-3-oxo-4-(prop-2-ynyl)-3,4-dihydro-2H-benzo[b][1,4]oxazin-6-yl)-1,3,5-triazinane-2,4-dione as described herein and 1,5-dimethyl-6-thioxo-3-(2,2,7-trifluoro-3-oxo-4-(prop-2-ynyl)-3,4-dihydro-2H-benzo[b][1,4]oxazin-6-yl)-1,3,5-triazinane-2,4-dione in a form which is different from form B, where the total amount of 1,5-dimethyl-6-thioxo-3-(2,2,7-trifluoro-3-oxo-4-(prop-2-ynyl)-3,4-dihydro-2H-benzo[b][1,4]oxazin-6-yl)-1,3,5-triazinane-2,4-dione in the mixture is at least 90 % by weight, preferably at least 94 % by weight, based on the total weight of the mixture. The mixture can likewise be used for preparing formulations as described hereinafter and can likewise be used as form B itself. In the mixture, the amout of form B will generally be at least 50 % by weight, in particular at least 60 % by weight, e.g. form 50 to 95 % by weight, in particular from 60 to 90 % by weight, based on the total amount of 1,5-dimethyl-6-thioxo-3-(2,2,7-trifluoro-3-oxo-4-(prop-2-ynyl)-3,4-dihydro-2H-benzo[b][1,4]oxazin-6-yl)-1,3,5-triazinane-2,4-dione contained in the mixture.

The preparation of benzoxazinone (I) used for the production of the form B can be effected by the process described in WO 2010/145992, to which full reference is hereby made.

In connection with the study on the crystallization of benzoxazinone (I), two further crystalline modifications A and C were found. While modification A can be obtained in pure form, modification C was occasionally obtained as a mixture with forms A and B. Form A can be stably formulated and is part of another patent application.

The form A can be identified by X-ray powder diffractometry on the basis of its diffraction diagram. Thus an X-ray powder diffraction diagram of form A recorded using Cu-K$\alpha$ radiation (1.54178 Å) at 25 °C shows at least 3, often at least 5, in particular at least 7, and especially all of the reflections quoted in the following table as 2$\theta$ values or as interplanar spacings d:

| 2$\theta$ values | d [Å] |
|---|---|
| 8,6$\pm$0,2° | 10,28 |
| 10,9$\pm$0,2° | 8,16 |
| 12,9$\pm$0,2° | 6,86 |
| 13,4$\pm$0,2° | 6,63 |
| 14,0$\pm$0,2° | 6,33 |
| 14,4$\pm$0,2° | 6,14 |
| 15,5$\pm$0,2° | 5,72 |
| 16,9$\pm$0,2° | 5,25 |
| 18,2$\pm$0,2° | 4,88 |
| 20,5$\pm$0,2° | 4,33 |

Studies on single crystals of form A demonstrate that the underlying crystal structure is orthorhombic. The unit cell has the space group Pna2(1). The characteristic data of the crystal structure of form A (determined at -173 °C) are compiled in the following table.

Crystallographic characteristics of form A

| Parameter | Form A |
|---|---|
| Crystal system | Orthorhombic |
| Space group | P n a $2_1$ |
| a | 16.0815(4) Å |
| b | 13.1360(3) Å |
| c | 7.9675(2) Å |
| $\alpha$ | 90° |
| $\beta$ | 90° |
| $\gamma$ | 90° |
| Volume | 1683.11(7) Å3 |
| Z | 4 |
| Density (calculated) | 1.63 g/cm$^3$ |
| R-Factor (%) | 2.97 |
| a,b,c = Length of the edges of the unit cell<br>$\alpha$ ,$\beta$,$\gamma$ = Angles of the unit cell<br>Z = Number of molecules, in the unit cell | |

Form A displays a thermogram with a characteristic melting peak in the range from 150 to 185°C. The melting point, determined as the onset of the melting peak, typically lies in the range from about 170°C to 180°C, in particular in the range from 174 to 179°C.
The melting enthalpy is preferably in the range from 70 to 80 J/g. The values quoted here relate to values determined by differential calorimetry (differential scanning calorimetry: DSC, aluminum closed and vented cup, nitrogen flow 150 ml/min, heating rate 5 K/min).

The production of the form A of benzoxazinone (I) according to the invention may be effected by crystallization from a solution of benzoxazinone (I) in a suitable organic solvent. Suitable solvents for the crystallization of form A from a solution are organic solvents which are selected from $C_1$-$C_3$-alkanols, such as methanol, ethanol, n-propanol or isopropanol, $C_1$-$C_4$-dialkylketones, such as acetone, mono- or di-$C_1$-$C_4$-dialkylbenzenes such as ethylbenzene or xylenes, and mono- or dichlorobenzenes.

The production of the form A of benzoxazinone (I) according to the invention may be also be effected by crystallization from a slurry of benzoxazinone (I) in a suitable organic solvent. Suitable solvents for the crystallization of form A from a slurry are mixtures of water with water-miscible organic solvents which are selected from $C_1$-$C_3$-alkanols, in particular ethanol or isopropanol, $C_2$-$C_4$-alkandiols, such as 1,3-propanediol, $C_1$-$C_4$-dialkylketones, such as acetone and cyclic ethers having preferably 4 to 6 carbon atoms and 1 or 2 oxygen atoms, such as tetrahy-drofurane and 1,4-dioxane.

In order to obtain form A of benzoxazinone (I), the crystallization is effected at temperatures of below 60°C, in particular at most 50°C and more preferably at most 40°C. Crystallization of form A is preferably effected under controlled conditions, i.e. the conditions of the crystallization are chosen to achieve a slow crystallization rate.

For this, in a first step i) a solution or slurry of benzoxazinone (I) in one of the aforesaid solvents or solvent mixtures is prepared, and then in a second step ii) crystallization of the benzoxazinone (I) is effected at temperatures of below 60°C, in particular at most 50°C and more preferably at most 40°C, e.g. from -10 to 50°C, in particular from 0 to 40°C.

The concentration of benzoxazinone (I) in the solution or slurry used for the crystallization naturally depends on the nature of the solvent and the dissolution temperature and often lies in the range from 100 to 800 g/l. Suitable conditions can be determined by the person skilled in the art by routine experiments.

Apart from that crystallization from a slurry of benzoxazinone (I) to obtain form A can be performed by analogy to the crystallization of form B, in particular regarding preparation of the slurry, concentrations and measures of effecting crystallization, provided that crystallization is effected at temperatures of below 60°C, in particular at most 50°C and more preferably at most 40°C, e.g. from -10 to 50°C, in particular from 0 to 40°C.

By means of the crystallization according to the invention, form A is obtained with a benzoxazinone (I) content of as a rule at least 90 wt.%, often 94 wt.%, in particular at least 96 wt.%. The content of form A, based on the total

quantity of benzoxazinone (I), is typically at least 90% and often at least 95 % or at least 96%.

In the mixture of forms A, B and C, form C can be identified by X-ray powder diffractometry on the basis of its diffraction diagram. Thus an X-ray powder diffraction diagram recorded using Cu-Kα radiation (1.54178 Å) at 25°C shows at least 3, often at least 5, and especially all of the reflections quoted in the following table as 2θ values or as interplanar spacings d:

| 2θ values | d [Å] |
|---|---|
| 7,6±0,2° | 11,64 |
| 9,6±0,2° | 9,17 |
| 11,8±0,2° | 7,48 |
| 12,4±0,2° | 7,11 |
| 15,2±0,2° | 5,81 |
| 15,9±0,2° | 5,57 |
| 16,1±0,2° | 5,52 |
| 19,1±0,2° | 4,64 |

To broaden the spectrum of action and to achieve synergistic effects, the crystalline form B of benzoxazinone (I) can be mixed with a large number of representatives of other herbicidal or growth-regulating active ingredient groups and then applied concomitantly. Suitable components for mixtures are, for example, 1,2,4-thiadiazoles, 1,3,4-thiadiazoles, amides, aminophosphoric acid and its derivatives, aminotriazoles, anilides, (het)aryloxyalkanoic acids and their derivatives, benzoic acid and its derivatives, benzothiadiazinones, 2-aroyl-1,3-cyclohexanediones, 2-hetaroyl-1,3-cyclohexanediones, hetaryl aryl ketones, benzylisoxazolidinones, meta-$CF_3$-phenyl derivatives, carbamates, quinolinecarboxylic acid and its derivatives, chloroacetanilides, cyclohexenone oxime ether derivatives, diazines, dichloropropionic acid and its derivatives, dihydrobenzofurans, dihydrofuran-3-ones, dinitroanilines, dinitrophenols, diphenyl ethers, dipyridyls, halocarboxylic acids and their derivatives, ureas, 3-phenyluracils, imidazoles, imidazolinones, N-phenyl-3,4,5,6-tetrahydrophthalimides, oxadiazoles, oxiranes, phenols, aryloxy- and hetaryloxyphenoxypropionic esters, phenylacetic acid and its derivatives, 2-phenylpropionic acid and its derivatives, pyrazoles, phenylpyrazoles, pyridazines, pyridinecarboxylic acid and its derivatives, pyrimidyl ethers, sulfonamides, sulfonylureas, triazines, triazinones, triazolinones, triazolecarboxamides, uracils, phenyl pyrazolines and isoxazolines and derivatives thereof.

It is furthermore be beneficial to apply the crystalline form B of benzoxazinone (I) alone or in combination with other herbicides, or else in the form of a mixture with other crop protection agents, for example together with agents for controlling pests or phytopathogenic fungi or bacteria.

Also of interest is the miscibility with mineral salt solutions, which are employed for treating nutritional and trace element deficiencies. Other additives such as non-phytotoxic oils and oil concentrates may also be added.

In one embodiment of the present invention the compositions according to the present invention comprise the crystalline form B of benzoxazinone (I) and at least one further herbicide B.

The further herbicide B is selected from the herbicides of class b1) to b15):

b1) lipid biosynthesis inhibitors;
b2) acetolactate synthase inhibitors (ALS inhibitors);
b3) photosynthesis inhibitors;
b4) protoporphyrinogen-IX oxidase inhibitors,
b5) bleacher herbicides;
b6) enolpyruvyl shikimate 3-phosphate synthase inhibitors (EPSP inhibitors);
b7) glutamine synthetase inhibitors;
b8) 7,8-dihydropteroate synthase inhibitors (DHP inhibitors);
b9) mitose inhibitors;
b10) inhibitors of the synthesis of very long chain fatty acids (VLCFA inhibitors);
b11) cellulose biosynthesis inhibitors;
b12) decoupler herbicides;
b13) auxin herbicides;
b14) auxin transport inhibitors; and
b15) other herbicides selected from the group consisting of bromobutide, chlorflurenol, chlorflurenol-methyl, cinmethylin, cumyluron, dalapon, dazomet, difenzoquat, difenzoquat-metilsulfate, dimethipin, DSMA, dymron, endothal and its salts, etobenzanid, flamprop, flamprop-isopropyl, flamprop-methyl, flamprop-M-isopropyl, flamprop-M-methyl, flurenol, flurenol-butyl, flurprimidol, fosamine, fosamine-ammonium, indanofan, indaziflam, maleic hydrazide, mefluidide, metam, methyl azide, methyl bromide, methyl-dymron, methyl iodide, MSMA, oleic acid, oxaziclomefone, pelargonic acid, pyributicarb, quinoclamine, triaziflam, tridiphane and 6-chloro-3-(2-cyclopropyl-6-methylphenoxy)-4-pyridazinol (CAS 499223-49-3) and its salts and esters.

Preference is given to those compositions according to the present invention comprising at least one herbicide B selected from herbicides of class b2, b3, b4, b5, b6, b9 and b10.

Specific preference is given to those compositions according to the present invention which comprise at least one herbicide B selected from the herbicides of class b4, b6, b9 and b10.

Particular preference is given to those compositions according to the present invention which comprise at least one herbicide B selected from the herbicides of class b4, b6 and b10.

According to a first embodiment of the invention the compositions contain at least one inhibitor of the lipid biosynthesis (herbicide b1). These are compounds which inhibit lipid biosynthesis. Inhibition of the lipid biosynthesis can be affected either through inhibition of acetyl CoA carboxylase (hereinafter termed ACC herbicides) or through a different mode of action (hereinafter termed non-ACC herbicides). The ACC herbicides belong to the group A of the HRAC classification system whereas the non-ACC herbicides belong to the group N of the HRAC classification.

According to a second embodiment of the invention the compositions contain at least one ALS inhibitor (herbicide b2). The herbicidal activity of these compounds is based on the inhibition of acetolactate synthase und thus on the inhibiton of the branched chain aminoacid biosynthesis. These inhibitors belong to the group B of the HRAC classification system.

According to a third embodiment of the invention the compositions contain at least one inhibitor of photosynthesis (herbicide b3). The herbicidal activity of these compounds is based either on the inhibition of the photosystem II in plants (so-called PSII inhibitors, groups C1, C2 and C3 of HRAC classification) or on diverting the electron transfer in photosystem I in plants (so-called PSI inhibitors, group D of HRAC classification) and thus on an inhibition of photosynthesis. Amongst these, PSII inhibitors are preferred.

According to a fourth embodiment of the invention the compositions contain at least one inhibitor of protoporphyrinogen-IX-oxidase (herbicide b4). The herbicidal activity of these compounds is based on the inhibition of the protoporphyrinogen-IX-oxidase. These inhibitors belong to the group E of the HRAC classification system.

According to a fifth embodiment of the invention the compositions contain at least one bleacher-herbicide (herbicide b5). The herbicidal activity of these compounds is based on the inhibition of the carotinoid biosynthesis. These include compounds which inhibit carotinoid biosynthesis by inhibition of phytoene desaturase (so-called PDS inhibitors, group F1 of HRAC classification), compounds which inhibit the 4-hydroxyphenylpyruvat-dioxygenase (HPPD inhibitors, group F2 of HRAC classification) and compounds which inhibit carotinoid biosynthesis by an unknown mode of action (bleacher - unknown target, group F3 of HRAC classification).

According to a sixth embodiment of the invention the compositions contain at least one EPSP synthase inhibitor (herbicide b6). The herbicidal activity of these compounds is based on the inhibition of enolpyruvyl shikimate 3-phosphate synthase und thus on the inhibition of the aminoacid biosynthesis in plants. These inhibitors belong to the group G of the HRAC classification system.

According to a seventh embodiment of the invention the compositions contain at least one glutamin synthetase inhibitor (herbicide b7). The herbicidal activity of these compounds is based on the inhibition of glutamin synthetase und thus on the inhibition of the aminoacid biosynthesis in plants. These inhibitors belong to the group H of the HRAC classification system.

According to an eighth embodiment of the invention the compositions contain at least one DHP synthase inhibitor (herbicide b8). The herbicidal activity of these compounds is based on the inhibition of 7,8-dihydropteroate synthetase. These inhibitors belong to the group I of the HRAC classification system.

According to a ninth embodiment of the invention the compositions contain at least one mitose inhibitor (herbicide b9). The herbicidal activity of these compounds is based on the disturbance or inhibition of microtubule formation or organization and thus on the mitosis inhibition. These inhibitors belong to the groups K1 and K2 of the HRAC classification system. Among these, compounds of the group K1, in particular dinitroanilines, are preferred.

According to a tenth embodiment of the invention the compositions contain at least one VLCFA inhibitor (herbicide b10). The herbicidal activity of these compounds is based on the inhibition of the synthesis of very long chain fatty acids and thus on the disturbance or inhibition of cell division in plants. These inhibitors belong to the group K3 of the HRAC classification system.

According to an eleventh embodiment of the invention the compositions contain at least one cellulose biosynthesis inhibitor (herbicide b11). The herbicidal activity of these compounds is based on the inhibition of the biosynthesis of cellulose and thus on the inhibition of the synthesis of cell walls in plants. These inhibitors belong to the group L of

the HRAC classification system.

According to a twelfth embodiment of the invention the compositions contain at least one decoupler herbicide (herbicide b12). The herbicidal activity of these compounds is based on the disruption of the cell membrane. These inhibitors belong to the group M of the HRAC classification system.

According to a thirteenth embodiment of the invention the compositions contain at least one auxin herbicide (herbicide b13). These include compounds which act like auxins, i.e. plant hormones, and inhibit the growth of the plants. These compounds belong to the group O of the HRAC classification system.

According to a fourteenth embodiment of the invention the compositions contain at least one auxin transport inhibitor (herbicide b14). The herbicidal activity of these compounds is based on the inhibition of the auxin transport in plants. These compounds belong to the group P of the HRAC classification system.

As to the given mechanisms of action and classification of the active substances, see e.g. "HRAC, Classification of Herbicides According to Mode of Action",
http://www.plantprotection.org/hrac/MOA.html).

Examples of herbicides B which can be used in combination with the crystalline form B of benzoxazinone (I) according to the present invention are:

b1) from the group of the lipid biosynthesis inhibitors:

ACC-herbicides such as alloxydim, alloxydim-sodium, butroxydim, clethodim, clodinafop, clodinafop-propargyl, cycloxydim, cyhalofop, cyhalofop-butyl, diclofop, diclofop-methyl, fenoxaprop, fenoxaprop-ethyl, fenoxaprop-P, fenoxaprop-P-ethyl, fluazifop, fluazifop-butyl, fluazifop-P, fluazifop-P-butyl, haloxyfop, haloxyfop-methyl, haloxyfop-P, haloxyfop-P-methyl, metamifop, pinoxaden, profoxydim, propaquizafop, quizalofop, quizalofop-ethyl, quizalofop-tefuryl, quizalofop-P, quizalofop-P-ethyl, quizalofop-P-tefuryl, sethoxydim, tepraloxydim and tralkoxydim, and non ACC herbicides such as benfuresate, butylate, cycloate, dalapon, dimepiperate, EPTC, esprocarb, ethofumesate, flupropanate, molinate, orbencarb, pebulate, prosulfocarb, TCA, thiobencarb, tiocarbazil, triallate and vernolate;

b2) from the group of the ALS inhibitors:

Sulfonylureas such as amidosulfuron, azimsulfuron, bensulfuron, bensulfuron-methyl, chlorimuron, chlorimuron-ethyl, chlorsulfuron, cinosulfuron, cyclosulfamuron, ethametsulfuron, ethametsulfuron-methyl, ethoxysulfuron, flazasulfuron, flucetosulfuron, flupyrsulfuron, flupyrsulfuron-methyl-sodium, foramsulfuron, halosulfuron, halosulfuron-methyl, imazosulfuron, iodosulfuron, iodosulfuron-methyl-sodium, mesosulfuron, metazosulfuron, metsulfuron, metsulfuron-methyl, nicosulfuron, orthosulfamuron, oxasulfuron, primisulfuron, primisulfuron-methyl, propy-risulfuron, prosulfuron, pyrazosulfuron, pyrazosulfuron-ethyl, rimsulfuron, sulfometuron, sulfometuron-methyl, sulfosulfuron, thifensulfuron, thifensulfuron-methyl, triasulfuron, tribenuron, tribenuron-methyl, trifloxysulfuron, triflusulfuron, triflusulfuron-methyl and tritosulfuron, imidazolinones such as imazamethabenz, imazamethabenz-methyl, imazamox, imazapic, imazapyr, imazaquin and imazethapyr, triazolopyrimidine herbicides and sulfonanilides such as cloransulam, cloransulam-methyl, diclosulam, flumetsulam, florasulam, metosulam, penoxsulam, pyrimisulfan and pyroxsulam, pyrimidinylbenzoates such as bispyribac, bispyribac-sodium, pyribenzoxim, pyriftalid, pyriminobac, pyriminobac-methyl, pyrithiobac, pyrithiobac-sodium, 4-[[[2-[(4,6-dimethoxy-2-pyrimidinyl)oxy]phenyl]methyl]amino]-benzoic acid-1-methylethyl ester (CAS 420138-41-6), 4-[[[2-[(4,6-dimethoxy-2-pyrimidinyl)oxy]phenyl]methyl]amino]-benzoic acid propyl ester (CAS 420138-40-5), N-(4-bromophenyl)-2-[(4,6-dimethoxy-2-pyrimidinyl)oxy]benzenemethanamine (CAS 420138-01-8), and sulfonylaminocarbonyl-triazolinone herbicides such as flucarbazone, flucarbazone-sodium, propoxycarbazon, propoxycarbazon- sodium, thiencarbazone and thiencarbazone-methyl. Among these, a preferred embodiment of the invention relates to those compositions comprising at least one imidazolinone herbicide;

b3) from the group of the photosynthesis inhibitors:

amicarbazone, inhibitors of the photosystem II, e.g. triazine herbicides, including of chlorotriazine, triazinones, triazindiones, methylthiotriazines and pyridazinones such as ametryn, atrazine, chloridazone, cyanazine, desmetryn, dimethametryn,hexazinone, metribuzin, prometon, prometryn, propazin, simazin, simetryn, terbumeton, terbuthylazin, terbutryn and trietazin, aryl urea such as chlorobromuron, chlorotoluron, chloroxuron, dimefuron, diuron, fluometuron, isoproturon, isouron, linuron, metamitron, methabenzthiazuron, metobenzuron, metoxuron, monolinuron, neburon, siduron, tebuthiuron and thiadiazuron, phenyl carbamates such as desmedipham, karbutilat, phenmedipham, phenmedipham-ethyl, nitrile herbicides such as bromofenoxim, bromoxynil and its salts and esters, ioxynil and its salts and esters, uraciles such as bromacil, lenacil and terbacil, and bentazon and

bentazon-sodium, pyridatre, pyridafol, pentanochlor and propanil and inhibitors of the photosystem I such as diquat, diquat-dibromide, paraquat, paraquat-dichloride and paraquat-dimetilsulfate. Among these, a preferred embodiment of the invention relates to those compositions comprising at least one aryl urea herbicide. Among these, likewise a preferred embodiment of the invention relates to those compositions comprising at least one triazine herbicide. Among these, likewise a preferred embodiment of the invention relates to those compositions comprising at least one nitrile herbicide;

b4) from the group of the protoporphyrinogen-IX oxidase inhibitors:

acifluorfen, acifluorfen-sodium, azafenidin, bencarbazone, benzfendizone, bifenox, butafenacil, carfentrazone, carfentrazone-ethyl, chlomethoxyfen, cinidon-ethyl, fluazolate, flufenpyr, flufenpyr-ethyl, flumiclorac, flumiclorac-pentyl, flumioxazin, fluoroglycofen, fluoroglycofen-ethyl, fluthiacet, fluthiacet-methyl, fomesafen, halosafen, lactofen, oxadiargyl, oxadiazon, oxyfluorfen, pentoxazone, profluazol, pyraclonil, pyraflufen, pyraflufen-ethyl, saflufenacil, sulfentrazone, thidiazimin, ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100), N-ethyl-3-(2,6-dichloro-4-trifluoromethylphenoxy)-5-methyl-1*H*-pyrazole-1-carboxamide (CAS 452098-92-9), N-tetrahydrofurfuryl-3-(2,6-dichloro-4-trifluoromethylphenoxy)-5-methyl-1*H*-pyrazole-1-carboxamide (CAS 915396-43-9), N-ethyl-3-(2-chloro-6-fluoro-4-trifluoromethylphenoxy)-5-methyl-1*H*-pyrazole-1-carboxamide (CAS 452099-05-7), N-tetrahydrofurfuryl-3-(2-chloro-6-fluoro-4-trifluoromethylphenoxy)-5-methyl-1*H*-pyrazole-1-carboxamide (CAS 45100-03-7) and 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione;

b5) from the group of the bleacher herbicides:

PDS inhibitors: beflubutamid, diflufenican, fluridone, flurochloridone, flurtamone, norflurazon, picolinafen, and 4-(3-trifluoromethylphenoxy)-2-(4-trifluoromethylphenyl)pyrimidine (CAS 180608-33-7), HPPD inhibitors: benzobicyclon, benzofenap, isoxaflutole, mesotrione, pyrasulfotole, pyrazolynate, pyrazoxyfen, sulcotrione, tefuryltrione, tembotrione, topramezone and bicyclopyrone, bleacher, unknown target: aclonifen, amitrole, clomazone and flumeturon;

b6) from the group of the EPSP synthase inhibitors:

glyphosate, glyphosate-isopropylammonium and glyphosate-trimesium (sulfosate);

b7) from the group of the glutamine synthase inhibitors:

bilanaphos (bialaphos), bilanaphos-sodium, glufosinate, glufosinate-P and glufosinate-ammonium;

b8) from the group of the DHP synthase inhibitors:

asulam;

b9) from the group of the mitose inhibitors:

compounds of group K1: dinitroanilines such as benfluralin, butralin, dinitramine, ethalfluralin, fluchloralin, oryzalin, pendimethalin, prodiamine and trifluralin, phosphoramidates such as amiprophos, amiprophos-methyl, and butamiphos, benzoic acid herbicides such as chlorthal, chlorthal-dimethyl, pyridines such as dithiopyr and thiazopyr, benzamides such as propyzamide and tebutam; compounds of group K2: chlorpropham, propham and carbetamide, among these, compounds of group K1, in particular dinitroanilines are preferred;

b10) from the group of the VLCFA inhibitors:

chloroacetamides such as acetochlor, alachlor, butachlor, dimethachlor, dimethenamid, dimethenamid-P, metazachlor, metolachlor, metolachlor-S, pethoxamid, pretilachlor, propachlor, propisochlor and thenylchlor, oxyacetanilides such as flufenacet and mefenacet, acetanilides such as diphenamid, naproanilide and napropamide, tetrazolinones such fentrazamide, and other herbicides such as anilofos, cafenstrole, fenoxasulfone, ipfencarbazone, piperophos, pyroxasulfone and
isoxazoline compounds of the formula II,

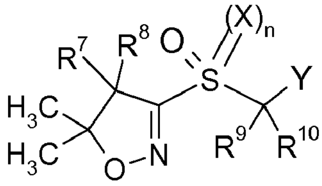

(II)

wherein $R^7$, $R^8$, $R^9$, $R^{10}$, W, Z and n have the following meanings:

| | |
|---|---|
| $R^7$, $R^8$, $R^9$, $R^{10}$ | independently of one another hydrogen, halogen or $C_1$-$C_4$-alkyl; |
| X | oxygen or NH; |
| Y | phenyl or monocyclic 5-, 6-, 7-, 8-, 9- or 10-membered heterocyclyl containing, in addition to carbon ring members one, two or three same or different heteroatoms selected from oxygen, nitrogen and sulfur as ring members, wherein phenyl and heterocyclyl are unsubstituted or carry 1, 2 or 3 substituents $R^{yy}$ selected from halogen, $C_1$-$C_4$-alkyl, $C_1$-$C_4$-alkoxy, $C_1$-$C_4$-haloalkyl and $C_1$-$C_4$-haloalkoxy; preferably phenyl or 5- or 6-membered aromatic heterocyclyl (hetaryl) which contains, in addition to carbon ring members, one, two or three nitrogen atoms as ring members, wherein phenyl and hetaryl are unsubstituted or carry 1, 2 or 3 substituents $R^{yy}$; and |
| n | zero or one; |

among the isoxazoline compounds of the formula II, preference is given to isoxazoline compounds of the formula II, wherein

| | |
|---|---|
| $R^7$, $R^8$, $R^9$, $R^{10}$ | independently of one another are H, F, Cl or methyl; |
| X | is oxygen; |
| n | is 0 or 1; and |
| Y | is phenyl, pyrazolyl or 1,2,3-triazolyl, wherein the three last-mentioned radicals are unsubstituted or carry one, two or three substituents $R^{yy}$, especially one of the following radicals |

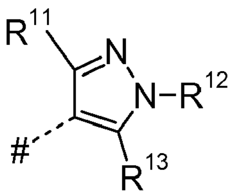
,
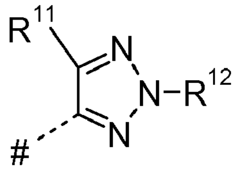
or
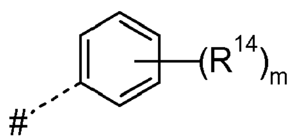

wherein

| | |
|---|---|
| $R^{11}$ | is halogen, $C_1$-$C_4$-alkyl or $C_1$-$C_4$-haloalkyl; |
| $R^{12}$ | is $C_1$-$C_4$-alkyl; |
| $R^{13}$ | is halogen, $C_1$-$C_4$-alkoxy or $C_1$-$C_4$-haloalkoxy; |
| $R^{14}$ | is halogen, $C_1$-$C_4$-alkyl, $C_1$-$C_4$-haloalkyl or $C_1$-$C_4$-haloalkoxy; |
| m | is 0, 1, 2 or 3; and |
| # | denotes the point of attachment to the group $CR^{13}R^{14}$; |

among the isoxazoline compounds of the formula II, particular preference is given to those isoxazoline compounds of the formula II, wherein

| | |
|---|---|
| $R^7$ | is hydrogen; |
| $R^8$ | is fluorine; |
| $R^9$ | is hydrogen or fluorine; |
| $R^{10}$ | is hydrogen or fluorine; |
| X | is oxygen; |
| Y | is one of the radicals of the formulae $Y^1$, $Y^2$, $Y^3$ or $Y^4$ |

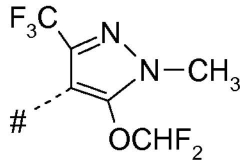 , 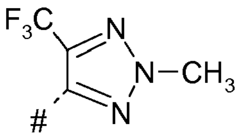 , 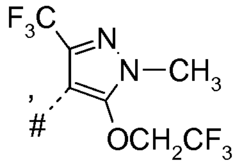 or 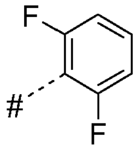

$Y^1$ $Y^2$ $Y^3$ $Y^4$

wherein # denotes the point of attachment to the group $CR^9R^{10}$;

n is zero or 1, in particular 1; and

among these, especially prefered are the isoxazoline compounds of the formulae II.1, II.2, II.3, II.4, II.5, II.6, II.7, II.8 and II.9

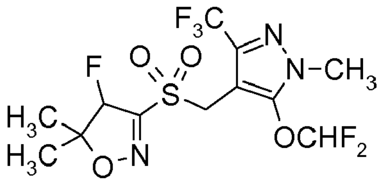

II.1

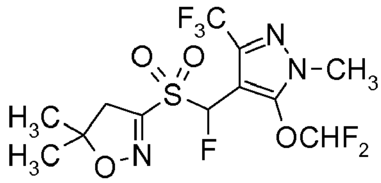

II.2

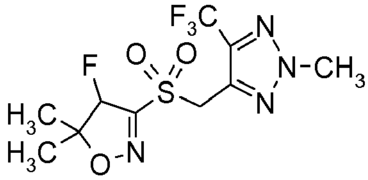

II.3

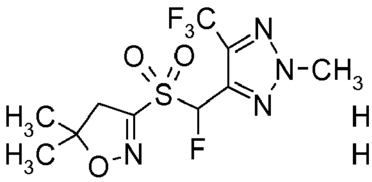

II.4

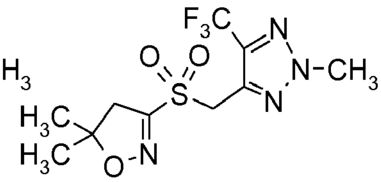

II.5

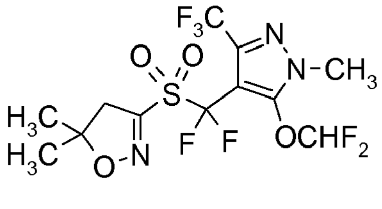

II.6

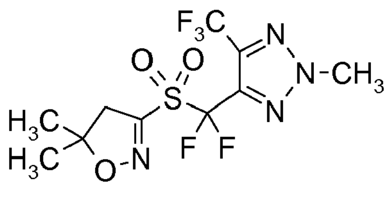

II.7

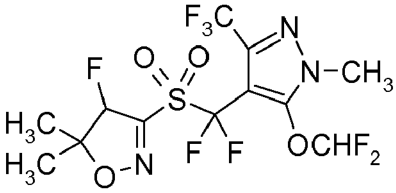

II.8

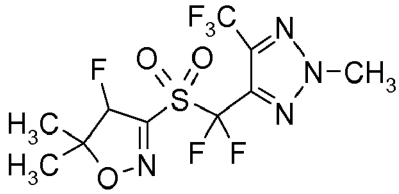

II.9

the isoxazoline compounds of the formula II are known in the art, e.g. from WO 2006/024820, WO 2006/037945, WO 2007/071900 and WO 2007/096576;

among the VLCFA inhibitors, preference is given to chloroacetamides and oxyacetamides, especially to pyroxasulfone;

b11) from the group of the cellulose biosynthesis inhibitors:

chlorthiamid, dichlobenil, flupoxam, isoxaben, 1-Cyclohexyl-5-pentafluorphenyloxy-$1^4$-[1,2,4,6]thiatriazin-3-ylamine and piperazine compounds of formula III,

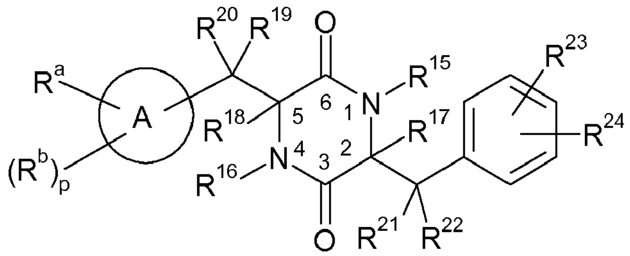

III,

in which

| | |
|---|---|
| A | is phenyl or pyridyl where $R^a$ is attached in the ortho-position to the point of attachment of A to a carbon atom; |
| $R^a$ | is CN, $NO_2$, $C_1$-$C_4$-alkyl, D-$C_3$-$C_6$-cycloalkyl, $C_1$-$C_4$-haloalkyl, $C_1$-$C_4$-alkoxy, $C_1$-$C_4$-haloalkoxy, O-D-$C_3$-$C_6$-cycloalkyl, $S(O)_qR^y$, $C_2$-$C_6$-alkenyl, D-$C_3$-$C_6$-cycloalkenyl, $C_3$-$C_6$-alkenyloxy, $C_2$-$C_6$-alkynyl, $C_3$-$C_6$-alkynyloxy, $NR^AR^B$, tri-$C_1$-$C_4$-alkylsilyl, D-C(=O)-$R^{a1}$, D-P(=O)$(R^{a1})_2$, phenyl, naphthyl, a 3- to 7-membered monocyclic or 9- or 10-membered bicyclic saturated, unsaturated or aromatic heterocycle which is attached via carbon or nitrogen, which contains 1, 2, 3 or 4 heteroatoms selected from the group consisting of O, N and S, and which may be partially or fully substituted by groups $R^{aa}$ and/or $R^{a1}$, and, if $R^a$ is attached to a carbon atom, additionally halogen;<br>$R^y$ is $C_1$-$C_6$-alkyl, $C_3$-$C_4$-alkenyl, $C_3$-$C_4$-alkynyl, $NR^AR^B$ or $C_1$-$C_4$-haloalkyl and q is 0, 1 or 2;<br>$R^A$,$R^B$ independently of one another are hydrogen, $C_1$-$C_6$-alkyl, $C_3$-$C_6$-alkenyl and $C_3$-$C_6$-alkynyl; together with the nitrogen atom to which they are attached, $R^A$,$R^B$ may also form a five- or six-membered saturated, partially or fully unsaturated ring which, in addition to carbon atoms, may contain 1, 2 or 3 heteroatoms selected from the group consisting of O, N and S, which ring may be substituted by 1 to 3 groups $R^{aa}$;<br>D is a covalent bond, $C_1$-$C_4$-alkylene, $C_2$-$C_6$-alkenyl or $C_2$-$C_6$-alkynyl;<br>$R^{a1}$ is hydrogen, OH, $C_1$-$C_8$-Alkyl, $C_1$-$C_4$-haloalkyl, $C_3$-$C_6$-cycloalkyl, $C_2$-$C_8$-alkenyl, $C_5$-$C_6$-cycloalkenyl, $C_2$-$C_8$-alkynyl, $C_1$-$C_6$-alkoxy, $C_1$-$C_4$-haloalkoxy, $C_3$-$C_8$-alkenyloxy, $C_3$-$C_8$-alkynyloxy, $NR^AR^B$, $C_1$-$C_6$-alkoxyamino, $C_1$-$C_6$-alkylsulfonylamino, $C_1$-$C_6$-alkylaminosulfonylamino, [di-($C_1$-$C_6$)alkylamino]sulfonylamino, $C_3$-$C_6$-alkenylamino, $C_3$-$C_6$-alkynylamino, N-($C_2$-$C_6$-alkenyl)-N-($C_1$-$C_6$-alkyl)amino, N-($C_2$-$C_6$-alkynyl)-N-($C_1$-$C_6$-alkyl)amino, N-($C_1$-$C_6$-alkoxy)-N-($C_1$-$C_6$-alkyl)amino, N-($C_2$-$C_6$-alkenyl)-N-($C_1$-$C_6$-alkoxy)amino, N-($C_2$-$C_6$-alkynyl)-N-($C_1$-$C_6$-alkoxy)-amino, $C_1$-$C_6$-alkylsulfonyl, tri-$C_1$-$C_4$-alkylsilyl, phenyl, phenoxy, phenylamino or a 5- or 6-membered monocyclic or 9- or 10-membered bicyclic heterocycle which contains 1, 2, 3 or 4 heteroatoms selected from the group consisting of O, N and S, where the cyclic groups are unsubstituted or substituted by 1, 2, 3 or 4 groups $R^{aa}$;<br>$R^{aa}$ is halogen, OH, CN, $NO_2$, $C_1$-$C_4$-alkyl, $C_1$-$C_4$-haloalkyl, $C_1$-$C_4$-alkoxy, $C_1$-$C_4$-haloalkoxy, $S(O)_qR^y$, D-C(=O)-$R^{a1}$ and tri-$C_1$-$C_4$-alkylsilyl; |
| $R^b$ | independently of one another are hydrogen, CN, $NO_2$, halogen, $C_1$-$C_4$-alkyl, $C_1$-$C_4$-haloalkyl, $C_2$-$C_4$-alkenyl, $C_3$-$C_6$-alkynyl, $C_1$-$C_4$-alkoxy, $C_1$-$C_4$-haloalkoxy, benzyl or $S(O)_qR^y$,<br>$R^b$ together with the group $R^a$ or $R^b$ attached to the adjacent ring atom may also form a five- or six-membered saturated or partially or fully unsaturated ring which, in addition to carbon atoms, may contain 1, 2 or 3 heteroatoms selected from the group consisting of O, N and S, which ring may be partially or fully substituted by $R^{aa}$; |
| p | is 0, 1, 2 or 3; |
| $R^{15}$ | is hydrogen, OH, CN, $C_1$-$C_{12}$-alkyl, $C_3$-$C_{12}$-alkenyl, $C_3$-$C_{12}$-alkynyl, $C_1$-$C_4$-alkoxy, $C_3$-$C_6$-cycloalkyl, $C_5$-$C_6$-cycloalkenyl, $NR^AR^B$, $S(O)_nR^y$, $S(O)_nNR^AR^B$, $C(=O)R^{25}$, CON- |

$R^AR^B$, phenyl or a 5- or 6-membered monocyclic or 9- or 10-membered bicyclic aromatic heterocycle which contains 1, 2, 3 or 4 heteroatoms selected from the group consisting of O, N and S, where the cyclic groups are attached via $D^1$ and are unsubstituted or substituted by 1, 2, 3 or 4 groups $R^{aa}$, and also the following partially or fully $R^{aa}$-substituted groups: $C_1$-$C_4$-alkyl, $C_3$-$C_4$-alkenyl, $C_3$-$C_4$-alkynyl, $C_1$-$C_4$-alkoxy, $C_3$-$C_6$-cycloalkyl, $C_5$-$C_6$-cycloalkenyl, $NR^AR^B$, $S(O)_nR^y$, $S(O)_nNR^AR^B$, $C(=O)R^{25}$, $CONR^AR^B$;

preferably is hydrogen, OH, CN, $C_1$-$C_{12}$-alkyl, $C_3$-$C_{12}$-alkenyl, $C_3$-$C_{12}$-alkynyl, $C_1$-$C_4$-alkoxy, $C_3$-$C_6$-cycloalkyl, $C_5$-$C_6$-cycloalkenyl, $NR^AR^B$, $S(O)_nR^y$, $S(O)_nNR^AR^B$, $C(=O)R^{25}$, $CONR^AR^B$, phenyl or a 5- or 6-membered monocyclic or 9- or 10-membered bicyclic aromatic heterocycle which contains 1, 2, 3 or 4 heteroatoms selected from the group consisting of O, N and S, where the cyclic groups are attached via $D^1$ and are unsubstituted or substituted by 1, 2, 3 or 4 groups $R^{aa}$, and also the following partially or fully $R^{aa}$-substituted groups: $C_1$-$C_4$-alkyl, $C_3$-$C_4$-alkenyl and $C_3$-$C_4$-alkynyl;

$R^{25}$ is hydrogen, $C_1$-$C_4$-alkyl, $C_1$-$C_4$-haloalkyl, $C_1$-$C_4$-alkoxy or $C_1$-$C_4$-haloalkoxy;

$D^1$ is carbonyl or a group D;

where in groups $R^{15}$, $R^a$ and their sub-substituents the carbon chains and/or the cyclic groups may carry 1, 2, 3 or 4 substituents $R^{aa}$ and/or $R^{a1}$;

| | |
|---|---|
| $R^{16}$ | is $C_1$-$C_4$-alkyl, $C_3$-$C_4$-alkenyl or $C_3$-$C_4$-alkynyl; |
| $R^{17}$ | is OH, $NH_2$, $C_1$-$C_4$-alkyl, $C_3$-$C_6$-cycloalkyl, $C_3$-$C_6$-alkenyl, $C_3$-$C_6$-alkynyl, $C_1$-$C_4$-hydroxyalkyl, $C_1$-$C_4$-cyanoalkyl, $C_1$-$C_4$-haloalkyl, $C_1$-$C_4$-alkoxy-$C_1$-$C_4$-alkyl or $C(=O)R^{25}$; |
| $R^{18}$ | is hydrogen, halogen, $C_1$-$C_4$-alkyl or $C_1$-$C_4$-haloalkyl, or $R^{18}$ and $R^{19}$ together are a covalent bond; |
| $R^{19}$, $R^{20}$, $R^{21}$, $R^{21}$ | independently of one another are hydrogen, halogen, OH, CN, $NO_2$, $C_1$-$C_4$-alkyl, $C_1$-$C_4$-haloalkyl, $C_2$-$C_6$-alkenyl, $C_2$-$C_6$-alkynyl, $C_1$-$C_4$-alkoxy, $C_1$-$C_4$-haloalkoxy, $C_3$-$C_6$-cycloalkyl, $C_3$-$C_6$-cycloalkenyl and $C_3$-$C_6$-cycloalkynyl; |
| $R^{23}$, $R^{24}$ | independently of one another are hydrogen, halogen, OH, haloalkyl, $NR^AR^B$, $NR^AC(O)R^{26}$, CN, $NO_2$, $C_1$-$C_4$-alkyl, $C_1$-$C_4$-haloalkyl, $C_2$-$C_4$-alkenyl, $C_3$-$C_6$-alkynyl, $C_1$-$C_4$-alkoxy, $C_1$-$C_4$-haloalkoxy, O-C(O)$R^{26}$, phenoxy or benzyloxy, where in groups $R^{23}$ and $R^{24}$ the carbon chains and/or the cyclic groups may carry 1, 2, 3 or 4 substituents $R^{aa}$; |
| $R^{26}$ | is $C_1$-$C_4$-alkyl or $NR^AR^B$; |

among the isoxazoline compounds of the piperazin compounds of formula III, preference is given to the piperazine compounds of the formula III, wherein

| | |
|---|---|
| A | is phenyl or pyridyl where $R^a$ is attached in the ortho-position to the point of attachment of A to a carbon atom; |
| $R^a$ | is CN, $NO_2$, $C_1$-$C_4$-alkyl, $C_1$-$C_4$-haloalkyl, $C_1$-$C_4$-alkoxy, $C_1$-$C_4$-haloalkoxy or D-C(=O)-$R^{a1}$;<br>$R^y$ is $C_1$-$C_6$-alkyl, $C_3$-$C_4$-alkenyl, $C_3$-$C_4$-alkynyl, $NR^AR^B$ or $C_1$-$C_4$-haloalkyl and q is 0, 1 or 2;<br>$R^A$,$R^B$ independently of one another are hydrogen, $C_1$-$C_6$-alkyl, $C_3$-$C_6$-alkenyl and $C_3$-$C_6$-alkynyl; together with the nitrogen atom to which they are attached, $R^A$,$R^B$ may also form a five- or six-membered saturated, partially or fully unsaturated ring which, in addition to carbon atoms, may contain 1, 2 or 3 heteroatoms selected from the group consisting of O, N and S, which ring may be substituted by 1 to 3 groups $R^{aa}$;<br>D is a covalent bond or $C_1$-$C_4$-alkylene;<br>$R^{a1}$ is hydrogen, OH, $C_1$-$C_8$-Alkyl, $C_1$-$C_4$-haloalkyl, $C_3$-$C_6$-cycloalkyl;<br>$R^{aa}$ is halogen, OH, CN, $NO_2$, $C_1$-$C_4$-alkyl, $C_1$-$C_4$-haloalkyl, $C_1$-$C_4$-alkoxy, $C_1$-$C_4$-haloalkoxy, $S(O)_qR^y$, D-C(=O)-$R^{a1}$ and tri-$C_1$-$C_4$-alkylsilyl; |
| $R^b$ | independently of one another is CN, $NO_2$, halogen, $C_1$-$C_4$-alkyl, $C_1$-$C_4$-haloalkyl, $C_2$-$C_4$-alkenyl, $C_3$-$C_6$-alkynyl, $C_1$-$C_4$-alkoxy, $C_1$-$C_4$-haloalkoxy, benzyl or $S(O)_qR^y$, $R^b$ together with the group $R^a$ or $R^b$ attached to the adjacent ring atom may also form a five- or six-membered saturated or partially or fully unsaturated ring which, in addition to carbon atoms, may contain 1, 2 or 3 heteroatoms selected from the group consisting of O, N and S, which ring may be partially or fully substituted by $R^{aa}$; |
| p | is 0 or 1; |
| $R^{15}$ | is hydrogen, $C_1$-$C_{12}$-alkyl, $C_3$-$C_{12}$-alkenyl, $C_3$-$C_{12}$-alkynyl, $C_1$-$C_4$-alkoxy or $C(=O)R^{25}$, which can be partially or fully substituted by $R^{aa}$-groups; preferably is hydrogen, $C_1$-$C_{12}$-alkyl, $C_3$-$C_{12}$-alkenyl, $C_3$-$C_{12}$-alkynyl, $C_1$-$C_4$-alkoxy or $C(=O)R^{25}$;<br>$R^{25}$ is hydrogen, $C_1$-$C_4$-alkyl, $C_1$-$C_4$-haloalkyl, $C_1$-$C_4$-alkoxy or $C_1$-$C_4$-haloalkoxy; where in groups |

| | |
|---|---|
| | $R^{15}$, $R^a$ and their sub-substituents the carbon chains and/or the cyclic groups may carry 1, 2, 3 or 4 substituents $R^{aa}$ and/or $R^{a1}$; |
| $R^{16}$ | is $C_1$-$C_4$-alkyl; |
| $R^{17}$ | is OH, $NH_2$, $C_1$-$C_4$-alkyl, $C_3$-$C_6$-cycloalkyl, $C_1$-$C_4$-haloalkyl or $C(=O)R^{25}$; |
| $R^{18}$ | is hydrogen, or $R^{18}$ and $R^{19}$ together are a covalent bond; |
| $R^{19}$, $R^{20}$, $R^{21}$, $R^{21}$ | independently of one another are hydrogen; |
| $R^{23}$, $R^{24}$ | independently of one another are hydrogen, halogen or OH; |

b12) from the group of the decoupler herbicides:

dinoseb, dinoterb and DNOC and its salts;

b13) from the group of the auxin herbicides:

2,4-D and its salts and esters, 2,4-DB and its salts and esters, aminopyralid and its salts such as aminopyralid-tris(2-hydroxypropyl)ammonium and its esters, benazolin, benazolin-ethyl, chloramben and its salts and esters, clomeprop, clopyralid and its salts and esters, dicamba and its salts and esters, dichlorprop and its salts and esters, dichlorprop-P and its salts and esters, fluroxypyr, fluroxypyr-butometyl, fluroxypyr-meptyl, MCPA and its salts and esters, MCPA-thioethyl, MCPB and its salts and esters, mecoprop and its salts and esters, mecoprop-P and its salts and esters, picloram and its salts and esters, quinclorac, quinmerac, TBA (2,3,6) and its salts and esters, triclopyr and its salts and esters, and aminocyclopyrachlor and its salts and esters;

b14) from the group of the auxin transport inhibitors: diflufenzopyr, diflufenzopyr-sodium, naptalam and naptalam-sodium;

b15) from the group of the other herbicides: bromobutide, chlorflurenol, chlorflurenol-methyl, cinmethylin, cumyluron, dalapon, dazomet, difenzoquat, difenzoquat-metilsulfate, dimethipin, DSMA, dymron, endothal and its salts, etobenzanid, flamprop, flamprop-isopropyl, flamprop-methyl, flamprop-M-isopropyl, flamprop-M-methyl, flurenol, flurenol-butyl, flurprimidol, fosamine, fosamine-ammonium, indanofan, indaziflam, maleic hydrazide, mefluidide, metam, methyl azide, methyl bromide, methyl-dymron, methyl iodide, MSMA, oleic acid, oxaziclomefone, pelargonic acid, pyributicarb, quinoclamine, triaziflam, tridiphane and 6-chloro-3-(2-cyclopropyl-6-methylphenoxy)-4-pyridazinol (CAS 499223-49-3) and its salts and esters.

Preferred herbicides B which can be used in combination with the crystalline form B of benzoxazinone (I) according to the present invention are:

b1) from the group of the lipid biosynthesis inhibitors:

clethodim, clodinafop-propargyl, cycloxydim, cyhalofop-butyl, diclofop-methyl, fenoxaprop-P-ethyl, fluazifop-P-butyl, haloxyfop-P-methyl, metamifop, pinoxaden, profoxydim, propaquizafop, quizalofop-P-ethyl, quizalofop-P-tefuryl, sethoxydim, tepraloxydim, tralkoxydim, benfuresate, dimepiperate, EPTC, esprocarb, ethofumesate, molinate, orbencarb, prosulfocarb, thiobencarb and triallate;

b2) from the group of the ALS inhibitors:

amidosulfuron, azimsulfuron, bensulfuron-methyl, bispyribac-sodium, chlorimuron-ethyl, chlorsulfuron, cloransulam-methyl, cyclosulfamuron, diclosulam, ethametsulfuron-methyl, ethoxysulfuron, flazasulfuron, florasulam, flucarbazone-sodium, flucetosulfuron, flumetsulam, flupyrsulfuron-methyl-sodium, foramsulfuron, halosulfuron-methyl, imazamethabenz-methyl, imazamox, imazapic, imazapyr, imazaquin, imazethapyr, imazosulfuron, iodosulfuron, iodosulfuron-methyl-sodium, mesosulfuron, metazosulfuron, metosulam, metsulfuron-methyl, nicosulfuron, orthosulfamuron, oxasulfuron, penoxsulam, primisulfuron-methyl, propoxycarbazon-sodium, propyrisul-furon, prosulfuron, pyrazosulfuron-ethyl, pyribenzoxim, pyrimisulfan, pyriftalid, pyriminobac-methyl, pyrithiobac-sodium, pyroxsulam, rimsulfuron, sulfometuron-methyl, sulfosulfuron, thiencarbazone-methyl, thifensulfuron-methyl, triasulfuron, tribenuron-methyl, trifloxysulfuron, triflusulfuron-methyl and tritosulfuron;

b3) from the group of the photosynthesis inhibitors:

ametryn, amicarbazone, atrazine, bentazone, bentazone-sodium, bromoxynil and its salts and esters, chloridazone, chlorotoluron, cyanazine, desmedipham, diquat-dibromide, diuron, fluometuron, hexazinone, ioxynil and its salts and esters, isoproturon, lenacil, linuron, metamitron, methabenzthiazuron, metribuzin, paraquat, paraquat-dichloride, phenmedipham, propanil, pyridate, simazine, terbutryn, terbuthylazine and thidiazuron;

b4) from the group of the protoporphyrinogen-IX oxidase inhibitors:

acifluorfen-sodium, bencarbazone, benzfendizone, butafenacil, carfentrazone-ethyl, cinidon-ethyl, flufenpyr-ethyl, flumiclorac-pentyl, flumioxazin, fluoroglycofen-ethyl, fomesafen, lactofen, oxadiargyl, oxadiazon, oxyfluorfen, pentoxazone, pyraflufen-ethyl, saflufenacil, sulfentrazone, ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100), N-ethyl-3-(2,6-dichloro-4-trifluoromethylphenoxy)-5-methyl-1*H*-pyrazole-1-carboxamide (CAS 452098-92-9), N-tetrahydrofurfuryl-3-(2,6-dichloro-4-trifluoromethylphenoxy)-5-methyl-1*H*-pyrazole-1-carboxamide (CAS 915396-43-9), N-ethyl-3-(2-chloro-6-fluoro-4-trifluoromethylphenoxy)-5-methyl-1*H*-pyrazole-1-carboxamide (CAS 452099-05-7), N-tetrahydrofurfuryl-3-(2-chloro-6-fluoro-4-trifluoromethylphenoxy)-5-methyl-1*H*-pyrazole-1-carboxamide (CAS 45100-03-7) and 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione;

b5) from the group of the bleacher herbicides:

aclonifen, beflubutamid, benzobicyclon, clomazone, diflufenican, flurochloridone, flurtamone, isoxaflutole, mesotrione, norflurazon, picolinafen, pyrasulfotole, pyrazolynate, sulcotrione, te-furyltrione, tembotrione, topramezone, bicyclopyrone, 4-(3-trifluoromethylphenoxy)-2-(4-trifluoromethylphenyl)pyrimidine (CAS 180608-33-7), amitrole and flumeturon;

b6) from the group of the EPSP synthase inhibitors:

glyphosate, glyphosate-isopropylammonium and glyphosate-trimesium (sulfosate);

b7) from the group of the glutamine synthase inhibitors:

glufosinate, glufosinate-P, glufosinate-ammonium;

b8) from the group of the DHP synthase inhibitors: asulam;

b9) from the group of the mitose inhibitors:

benfluralin, dithiopyr, ethalfluralin, oryzalin, pendimethalin, thiazopyr and trifluralin;

b10) from the group of the VLCFA inhibitors:

acetochlor, alachlor, anilofos, butachlor, cafenstrole, dimethenamid, dimethenamid-P, fentrazamide, flufenacet, mefenacet, metazachlor, metolachlor, S-metolachlor, naproanilide, napropamide, pretilachlor, fenoxasulfone, ipfencarbazone, pyroxasulfone thenylchlor and isoxazoline-compounds of the formulae II.1, II.2, II.3, II.4, II.5, II.6, II.7, II.8 and II.9 as mentioned above;

b11) from the group of the cellulose biosynthesis inhibitors: dichlobenil, flupoxam, isoxaben, 1-Cyclohexyl-5-pentafluorphenyloxy-1$^4$-[1,2,4,6]thiatriazin-3-ylamine and the piperazine compounds of formula III as mentioned above;

b13) from the group of the auxin herbicides:

2,4-D and its salts and esters, aminopyralid and its salts such as aminopyralid-tris(2-hydroxypropyl)ammonium and its esters, clopyralid and its salts and esters, dicamba and its salts and esters, dichlorprop-P and its salts and esters, fluroxypyr-meptyl, MCPA and its salts and esters, MCPB and its salts and esters, mecoprop-P and its salts and esters, picloram and its salts and esters, quinclorac, quinmerac, triclopyr and its salts and esters, and aminocyclopyrachlor and its salts and esters;

b14) from the group of the auxin transport inhibitors: diflufenzopyr and diflufenzopyr-sodium;

b15) from the group of the other herbicides: bromobutide, cinmethylin, cumyluron, dalapon, difenzoquat, difenzoquat-metilsulfate, DSMA, dymron (= daimuron), flamprop, flamprop-isopropyl, flamprop-methyl, flamprop-M-isopropyl, flamprop-M-methyl, indanofan, indaziflam, metam, methylbromide, MSMA, oxaziclomefone, pyributicarb, triaziflam, tridiphane and 6-chloro-3-(2-cyclopropyl-6-methylphenoxy)-4-pyridazinol (CAS 499223-49-3) and its salts and esters.

Particularly preferred herbicides B which can be used in combination with the crystalline form B of benzoxazinone (I) according to the present invention are:

b1) from the group of the lipid biosynthesis inhibitors: clodinafop-propargyl, cycloxydim, cyhalofop-butyl, fenoxaprop-P-ethyl, pinoxaden, profoxydim, tepraloxydim, tralkoxydim, esprocarb, prosulfocarb, thiobencarb and triallate;

b2) from the group of the ALS inhibitors: bensulfuron-methyl, bispyribac-sodium, cyclosulfamuron, diclosulam, flumetsulam, flupyrsulfuron-methyl-sodium, foramsulfuron, imazamox, imazapic, imazapyr, imazaquin, imazethapyr, imazosulfuron, iodosulfuron, iodosulfuron-methyl-sodium, mesosulfuron, metazosulfuron, nicosulfuron, penoxsulam, propoxycarbazon-sodium, pyrazosulfuron-ethyl, pyroxsulam, rimsulfuron, sulfosulfuron, thiencarbazon-methyl and tritosulfuron;

b3) from the group of the photosynthesis inhibitors: ametryn, atrazine, diuron, fluometuron, hexazinone, isoproturon, linuron, metribuzin, paraquat, paraquat-dichloride, propanil, terbutryn and terbuthylazine;

b4) from the group of the protoporphyrinogen-IX oxidase inhibitors: flumioxazin, oxyfluorfen, saflufenacil, sulfentrazone, ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100) and 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione;

b5) from the group of the bleacher herbicides: clomazone, diflufenican, flurochloridone, isoxaflutole, mesotrione, picolinafen, sulcotrione, tefuryltrione, tembotrione, topramezone, bicyclopyrone, amitrole and flumeturon;

b6) from the group of the EPSP synthase inhibitors: glyphosate, glyphosate-isopropylammonium and glyphosate-trimesium (sulfosate);

b7) from the group of the glutamine synthase inhibitors: glufosinate, glufosinate-P and glufosinate-ammonium;

b9) from the group of the mitose inhibitors: pendimethalin and trifluralin;

b10) from the group of the VLCFA inhibitors: acetochlor, cafenstrole, dimethenamid-P, fentrazamide, flufenacet, mefenacet, metazachlor, metolachlor, S-metolachlor, fenoxasulfone, ipfencarbazone and pyroxasulfone; likewise, preference is given to isoxazoline compounds of the formulae II.1, II.2, II.3, II.4, II.5, II.6, II.7, II.8 and II.9 as mentioned above;

b11) from the group of the cellulose biosynthesis inhibitors: isoxaben and the piperazine compounds of formula III as mentioned above;

b13) from the group of the auxin herbicides: 2,4-D and its salts and esters, aminopyralid and its salts and its esters, clopyralid and its salts and esters, dicamba and its salts and esters, fluroxypyr-meptyl, quinclorac, quinmerac and aminocyclopyrachlor and its salts and esters;

b14) from the group of the auxin transport inhibitors: diflufenzopyr and diflufenzopyr-sodium,

b15) from the group of the other herbicides: dymron (= daimuron), indanofan, indaziflam, oxaziclomefone and triaziflam.

Moreover, it may be useful to apply the crystalline form B of benzoxazinone (I) in combination with safeners. Safeners are chemical compounds which prevent or reduce damage on useful plants without having a major impact on the herbicidal action of the crystalline form B of benzoxazinone (I) towards unwanted plants. They can be applied either before sowings (e.g. on seed treatments, shoots or seedlings) or in the pre-emergence application or post-emergence application of the useful plant. The safeners and the crystalline form B of benzoxazinone (I) can be applied simultaneously

or in succession.

Furthermore, the safeners C, the crystalline form B of benzoxazinone (I) and/or the herbicides B can be applied simultaneously or in succession.

Suitable safeners are e.g. (quinolin-8-oxy)acetic acids, 1-phenyl-5-haloalkyl-1H-1,2,4-triazol-3-carboxylic acids, 1-phenyl-4,5-dihydro-5-alkyl-1 H-pyrazol-3,5-dicarboxylic acids, 4,5-dihydro-5,5-diaryl-3-isoxazol carboxylic acids, dichloroacetamides, alpha-oximinophenylacetonitriles, acetophenonoximes, 4,6-dihalo-2-phenylpyrimidines, N-[[4-(aminocarbonyl)phenyl]sulfonyl]-2-benzoic amides, 1,8-naphthalic anhydride, 2-halo-4-(haloalkyl)-5-thiazol carboxylic acids, phosphorthiolates and N-alkyl-O-phenylcarbamates and their agriculturally acceptable salts and their agriculturally acceptable derivatives such amides, esters, and thioesters, provided they have an acid group.

Examples of preferred safeners C are benoxacor, cloquintocet, cyometrinil, cyprosulfamide, dichlormid, dicyclonon, dietholate, fenchlorazole, fenclorim, flurazole, fluxofenim, furilazole, isoxadifen, mefenpyr, mephenate, naphthalic anhydride, oxabetrinil, 4-(dichloroacetyl)-1-oxa-4-azaspiro[4.5]decane (MON4660, CAS 71526-07-3) and 2,2,5-trimethyl-3-(dichloroacetyl)-1,3-oxazolidine (R-29148, CAS 52836-31-4).

Especially preferred safeners C are benoxacor, cloquintocet, cyprosulfamide, dichlormid, fenchlorazole, fenclorim, flurazole, fluxofenim, furilazole, isoxadifen, mefenpyr, naphthalic anhydride, oxabetrinil, 4-(dichloroacetyl)-1-oxa-4-azaspiro[4.5]decane (MON4660, CAS 71526-07-3) and 2,2,5-trimethyl-3-(dichloroacetyl)-1,3-oxazolidine (R-29148, CAS 52836-31-4).

Particularly preferred safeners C are benoxacor, cloquintocet, cyprosulfamide, dichlormid, fenchlorazole, fenclorim, furilazole, isoxadifen, mefenpyr, 4-(dichloroacetyl)-1-oxa-4-azaspiro-[4.5]decane (MON4660, CAS 71526-07-3) and 2,2,5-trimethyl-3-(dichloroacetyl)-1,3-oxazolidine (R-29148, CAS 52836-31-4).

The herbicides B of groups b1) to b15) and the safeners C are known herbicides and safeners, see, for example, The Compendium of Pesticide Common Names (http://www.alanwood.net/pesticides/); Farm Chemicals Handbook 2000 volume 86, Meister Publishing Company, 2000; B. Hock, C. Fedtke, R. R. Schmidt, Herbizide [Herbicides], Georg Thieme Verlag, Stuttgart 1995; W. H. Ahrens, Herbicide Handbook, 7th edition, Weed Science Society of America, 1994; and K. K. Hatzios, Herbicide Handbook, Supplement for the 7th edition, Weed Science Society of America, 1998. 2,2,5-Trimethyl-3-(dichloroacetyl)-1,3-oxazolidine [CAS No. 52836-31-4] is also referred to as R-29148. 4-(Dichloroacetyl)-1-oxa-4-azaspiro[4.5]decane [CAS No. 71526-07-3] is also referred to as AD-67 and MON 4660. Further herbicidally active compounds are known from WO 96/26202, WO 97/41116, WO 97/41117, WO 97/41118 and WO 01/83459 and also from W. Krämer et al. (ed.) "Modern Crop Protection Compounds", Vol. 1, Wiley VCH, 2007 and the literature cited therein.

The assignment of the active compounds to the respective mechanisms of action is based on current knowledge. If several mechanisms of action apply to one active compound, this substance was only assigned to one mechanism of action.

If the herbicides B and/or the safener C are capable of forming geometrical isomers, for example E/Z isomers, both the pure isomers and mixtures thereof may be used in the compositions according to the invention.

If the herbicides B and/or the safener C have one of more centers of chirality and are thus present as enantiomers or diastereomers, both the pure enantiomers and diastereomers and mixtures thereof may be used in the compositions according to the invention.

If the herbicides B and/or the safener C have ionizable functional groups, they can also be employed in the form of their agriculturally acceptable salts. Suitable are, in general, the salts of those cations and the acid addition salts of those acids whose cations and anions, respectively, have no adverse effect on the activity of the active compounds.

Preferred cations are the ions of the alkali metals, preferably of lithium, sodium and potassium, of the alkaline earth metals, preferably of calcium and magnesium, and of the transition metals, preferably of manganese, copper, zinc and iron, further ammonium and substituted ammonium in which one to four hydrogen atoms are replaced by $C_1$-$C_4$-alkyl, hydroxy-$C_1$-$C_4$-alkyl, $C_1$-$C_4$-alkoxy-$C_1$-$C_4$-alkyl, hydroxy-$C_1$-$C_4$-alkoxy-$C_1$-$C_4$-alkyl, phenyl or benzyl, preferably ammonium, methylammonium, isopropylammonium, dimethylammonium, diisopropylammonium, trimethylammonium, tetramethylammonium, tetraethylammonium, tetrabutylammonium, 2-hydroxyethylammonium, 2-(2-hydroxyeth-1-oxy)eth-1-ylammonium, di(2-hydroxyeth-1-yl)-ammonium, benzyltrimethylammonium, benzyltriethylammonium, furthermore phosphonium ions, sulfonium ions, preferably tri($C_1$-$C_4$-alkyl)sulfonium, such as trimethylsulfonium, and sulfoxonium ions, preferably tri($C_1$-$C_4$-alkyl)sulfoxonium.

Anions of useful acid addition salts are primarily chloride, bromide, fluoride, iodide, hydrogensulfate, methylsulfate, sulfate, dihydrogenphosphate, hydrogenphosphate, nitrate, bicarbonate, carbonate, hexafluorosilicate, hexafluorophosphate, benzoate and also the anions of $C_1$-$C_4$-alkanoic acids, preferably formate, acetate, propionate and butyrate.

Herbicides B and safeners C having a carboxyl group can be employed in the form of the acid, in the form of an agriculturally suitable salt or else in the form of an agriculturally acceptable derivative in the compositions according to the invention, for example as amides, such as mono- and di-$C_1$-$C_6$-alkylamides or arylamides, as esters, for example as allyl esters, propargyl esters, $C_1$-$C_{10}$-alkyl esters, alkoxyalkyl esters and also as thioesters, for example as $C_1$-$C_{10}$-alkylthio esters. Preferred mono- and di-$C_1$-$C_6$-alkylamides are the methyl and the dimethylamides. Preferred

arylamides are, for example, the anilides and the 2-chloroanilides. Preferred alkyl esters are, for example, the methyl, ethyl, propyl, isopropyl, butyl, isobutyl, pentyl, mexyl (1-methylhexyl) or isooctyl (2-ethylhexyl) esters. Preferred $C_1$-$C_4$-alkoxy-$C_1$-$C_4$-alkyl esters are the straight-chain or branched $C_1$-$C_4$-alkoxy ethyl esters, for example the methoxyethyl, ethoxyethyl or butoxyethyl ester. An example of a straight-chain or branched $C_1$-$C_{10}$-alkylthio ester is the ethylthio ester.

According to a preferred embodiment of the invention, the composition comprises as herbicide B at least one, prefereby exactly one herbicide B.

According to another preferred embodiment of the invention, the composition comprises as herbicide B, at least two, prefereby exactly two herbicides B different from each other.

According to another preferred embodiment of the invention, the composition comprises as herbicide B, at least three, prefereby exactly three herbicides B different from each other.

According to another preferred embodiment of the invention, the composition comprises as safener C at least one, prefereby exactly one safener C.

According to another preferred embodiment of the invention, the composition comprises as herbicide B, at least one, prefereby exactly one herbicide B, and at lest one, prefereby exactly one, safener C.

According to another preferred embodiment of the invention, the composition comprises as herbicide B, preferebly exactly two herbicides B different from each other, and at lest one, prefereby exactly one, safener C.

According to another preferred embodiment of the invention, the composition comprises as herbicide B, at least three, prefereby exactly three herbicides B different from each other, and at lest one, prefereby exactly one, safener C.

A first preferred embodiment of the invention relates to compositions according to the invention comprising, in addition to the crystalline form B of benzoxazinone (I), at least one and especially exactly one herbicide B from group b1), in particular selected from the group consisting of clodinafop-propargyl, cycloxydim, cyhalofop-butyl, fenoxaprop-P-ethyl, pinoxaden, profoxydim, tepraloxydim, tralkoxydim, esprocarb, prosulfocarb, thiobencarb and triallate.

A second preferred embodiment of the invention relates to compositions according to the invention comprising, in addition to the crystalline form B of benzoxazinone (I), at least one and especially exactly one herbicide B from group b2), in particular selected from the group consisting of bensulfuron-methyl, bispyribac-sodium, cyclosulfamuron, diclosulam, flumetsulam, flupyrsulfuron-methyl-sodium, foramsulfuron, imazamox, imazapic, imazapyr, imazaquin, imazethapyr, imazosulfuron, iodosulfuron, iodosulfuron-methyl-sodium, mesosulfuron, metazosulfuron, nicosulfuron, penoxsulam, propoxycarbazon-sodium, pyrazosulfuron-ethyl, pyroxsulam, rimsulfuron, sulfosulfuron, thiencarbazon-methyl and tritosulfuron.

A third preferred embodiment of the invention relates to compositions according to the invention comprising, in addition to the crystalline form B of benzoxazinone (I), at least one and especially exactly one herbicide B from group b3), in particular selected from the group consisting of ametryn, atrazine, diuron, fluometuron, hexazinone, isoproturon, linuron, metribuzin, paraquat, paraquat-dichloride, propanil, terbutryn and terbuthylazine.

A fourth preferred embodiment of the invention relates to compositions according to the invention comprising, in addition to the crystalline form B of benzoxazinone (I), at least one and especially exactly one herbicide B from group b4), in particular selected from the group consisting of flumioxazin, oxyfluorfen, saflufenacil, sulfentrazone, ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100) and 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione.

A fifth preferred embodiment of the invention relates to compositions according to the invention comprising, in addition to the crystalline form B of benzoxazinone (I), at least one and especially exactly one herbicide B from group b5), in particular selected from the group consisting of clomazone, diflufenican, flurochloridone, isoxaflutole, mesotrione, picolinafen, sulcotrione, tefuryltrione, tembotrione, topramezone, bicyclopyrone, amitrole and flumeturon.

A sixth preferred embodiment of the invention relates to compositions according to the invention comprising, in addition to the crystalline form B of benzoxazinone (I), at least one and especially exactly one herbicide B from group b6), in particular selected from the group consisting of glyphosate, glyphosate-isopropylammonium and glyphosate-trimesium (sulfosate).

A seventh preferred embodiment of the invention relates to compositions according to the invention comprising, in addition to the crystalline form B of benzoxazinone (I), at least one and especially exactly one herbicide B from group b7), in particular selected from the group consisting of glufosinate, glufosinate-P and glufosinate-ammonium.

An eighth preferred embodiment of the invention relates to compositions according to the invention comprising, in addition to the crystalline form B of benzoxazinone (I), at least one and especially exactly one herbicide B from group b9), in particular selected from the group consisting of pendimethalin and trifluralin.

A nineth preferred embodiment of the invention relates to compositions according to the invention comprising, in addition to the crystalline form B of benzoxazinone (I), at least one and especially exactly one herbicide B from group b10), in particular selected from the group consisting of acetochlor, cafenstrole, dimethenamid-P, fentrazamide, flufenacet, mefenacet, metazachlor, metolachlor, S-metolachlor, fenoxasulfone and pyroxasulfone. Likewise, preference is

given to compositions comprising in addition to the crystalline form B of benzoxazinone (I), at least one and especially exactly one herbicide B from group b10), in particular selected from the group consisting of isoxazoline compounds of the formulae II.1, II.2, II.3, II.4, II.5, II.6, II.7, II.8 and II.9, as defined above.

A tenth preferred embodiment of the invention relates to compositions according to the invention comprising, in addition to the crystalline form B of benzoxazinone (I), at least one and especially exactly one herbicide B from group b11), in particular isoxaben. Likewise, preference is given to compositions comprising in addition to the crystalline form B of benzoxazinone (I), at least one and especially exactly one herbicide B from group b11), in particular selected from the group consisting of piperazine compounds of formula III as defined above.

An eleventh preferred embodiment of the invention relates to compositions according to the invention comprising, in addition to the crystalline form B of benzoxazinone (I), at least one and especially exactly one herbicide B from group b13), in particular selected from the group consisting of 2,4-D and its salts and esters, aminopyralid and its salts such as aminopyralid-tris(2-hydroxypropyl)ammonium and its esters, clopyralid and its salts and esters, dicamba and its salts and esters, fluroxypyr-meptyl, quinclorac, quinmerac and aminocyclopyrachlor and its salts and esters.

A twelfth preferred embodiment of the invention relates to compositions according to the invention comprising, in addition to the crystalline form B of benzoxazinone (I), at least one and especially exactly one herbicide B from group b14), in particular selected from the group consisting of diflufenzopyr and diflufenzopyr-sodium.

A 13th preferred embodiment of the invention relates to compositions according to the invention comprising, in addition to the crystalline form B of benzoxazinone (I), at least one and especially exactly one herbicide B from group b15), in particular selected from the group consisting of dymron (= daimuron), indanofan, indaziflam, oxaziclomefone and triaziflam.

A 14th preferred embodiment of the invention relates to compositions according to the invention comprising, in addition to the crystalline form B of benzoxazinone (I), at least one and especially exactly one safener C, in particular selected from the group consisting of benoxacor, cloquintocet, cyprosulfamide, dichlormid, fenchlorazole, fenclorim, furilazole, isoxadifen, mefenpyr, 4-(dichloroacetyl)-1-oxa-4-azaspiro[4.5]decane (MON4660, CAS 71526-07-3) and 2,2,5-trimethyl-3-(dichloroacetyl)-1,3-oxazolidine (R-29148, CAS 52836-31-4).

Further preferred embodiments relate to ternary compositions which correspond to the binary compositions of embodiments 1 to 14 and additionally comprise a safener C, in particular selected from the group consisting of benoxacor, cloquintocet, cyprosulfamide, dichlormid, fenchlorazole, fenclorim, furilazole, isoxadifen, mefenpyr, 4-(dichloroacetyl)-1-oxa-4-azaspiro[4.5]decane (MON4660, CAS 71526-07-3) and 2,2,5-trimethyl-3-(dichloroacetyl)-1,3-oxazolidine (R-29148, CAS 52836-31-4).

Here and below, the term "binary compositions" includes compositions comprising the crystalline form B of benzoxazinone (I), and either one or more, for example 1, 2 or 3, herbicides B or one or more safeners C.

Correspondingly, the term "ternary compositions" includes compositions comprising the crystalline form B of benzoxazinone (I), one or more, for example 1, 2 or 3, herbicides B, and one or more, for example 1, 2 or 3, safeners C.

In binary compositions comprising the crystalline form B of benzoxazinone (I) and at least one herbicide B, the weight ratio of the crystalline form B of benzoxazinone (I) : herbicide B is generally in the range of from 1:1000 to 1000:1, preferably in the range of from 1:500 to 500:1, in particular in the range of from 1:250 to 250:1 and particularly preferably in the range of from 1:75 to 75:1.

In binary compositions comprising the crystalline form B of benzoxazinone (I) and at least one safener C, the weight ratio of the crystalline form B of benzoxazinone (I) : safener C is generally in the range of from 1:1000 to 1000:1, preferably in the range of from 1:500 to 500:1, in particular in the range of from 1:250 to 250:1 and particularly preferably in the range of from 1:75 to 75:1.

In ternary compositions comprising the crystalline form B of benzoxazinone (I), at least one herbicide B and at least one safener C, the relative proportions by weight of the crystalline form B of benzoxazinone (I) : herbicide B are generally in the range of from 1:1000 to 1000:1, preferably in the range of from 1:500 to 500:1, in particular in the range of from 1:250 to 250:1 and particularly preferably in the range of from 1:75 to 75:1, the weight ratio of the crystalline form B of benzoxazinone (I) : safener C is generally in the range of from 1:1000 to 1000:1, preferably in the range of from 1:500 to 500:1, in particular in the range of from 1:250 to 250:1 and particularly preferably in the range of from 1:75 to 75:1, and the weight ratio of the herbicide B : safener C is generally in the range of from 1:1000 to 1000:1, preferably in the range of from 1:500 to 500:1, in particular in the range of from 1:250 to 250:1 and particularly preferably in the range of from 1:75 to 75:1. The weight ratio of the crystalline form B of benzoxazinone (I) + herbicide B to safener C is preferably in the range of from 1:500 to 500:1, in particular in the range of from 1:250 to 250:1 and particularly preferably in the range of from 1:75 to 75:1.

Particularly preferred herbicides B are the herbicides B as defined above; in particular the herbicides B.1 - B.141 listed below in table B:

Table B:

| | Herbicide B |
|---|---|
| B.1 | clethodim |
| B.2 | clodinafop-propargyl |
| B.3 | cycloxydim |
| B.4 | cyhalofop-butyl |
| B.5 | fenoxaprop-P-ethyl |
| B.6 | metamifop |

| | Herbicide B |
|---|---|
| B.7 | pinoxaden |
| B.8 | profoxydim |
| B.9 | sethoxydim |
| B.10 | tepraloxydim |
| B.11 | tralkoxydim |
| B.12 | esprocarb |

| | Herbicide B |
|---|---|
| B.13 | ethofumesate |
| B.14 | molinate |
| B.15 | prosulfocarb |
| B.16 | thiobencarb |
| B.17 | triallate |
| B.18 | bensulfuron-methyl |
| B.19 | bispyribac-sodium |
| B.20 | cloransulam |
| B.21 | chlorsulfuron |
| B.22 | clorimuron |
| B.23 | cyclosulfamuron |
| B.24 | diclosulam |
| B.25 | florasulam |
| B.26 | flumetsulam |
| B.27 | flupyrsulfuron-methyl-sodium |
| B.28 | foramsulfuron |
| B.29 | imazamox |
| B.30 | imazapic |
| B.31 | imazapyr |
| B.32 | imazaquin |
| B.33 | imazethapyr |
| B.34 | imazosulfuron |
| B.35 | iodosulfuron-methyl-sodium |
| B.36 | mesosulfuron |
| B.37 | metazosulfuron |
| B.38 | metsulfuron |
| B.39 | metosulam |
| B.40 | nicosulfuron |
| B.41 | penoxsulam |
| B.42 | propoxycarbazon-sodium |
| B.43 | pyrazosulfuron-ethyl |
| B.44 | pyribenzoxim |
| B.45 | pyriftalid |
| B.46 | pyroxsulam |
| B.47 | rimsulfuron |
| B.48 | sulfosulfuron |
| B.49 | thiencarbazone-methyl |
| B.50 | thifensulfuron |
| B.51 | tribenuron |
| B.52 | tritosulfuron |

| | Herbicide B |
|---|---|
| B.53 | ametryne |
| B.54 | atrazine |
| B.55 | bentazon |
| B.56 | bromoxynil |
| B.57 | diuron |
| B.58 | fluometuron |
| B.59 | hexazinone |
| B.60 | isoproturon |
| B.61 | linuron |
| B.62 | metamitron |
| B.63 | metribuzin |
| B.64 | propanil |
| B.65 | simazin |
| B.66 | terbuthylazine |
| B.67 | terbutryn |
| B.68 | paraquat-dichloride |
| B.69 | acifluorfen |
| B.70 | butafenacil |
| B.71 | carfentrazone-ethyl |
| B.72 | flumioxazin |
| B.73 | fomesafen |
| B.74 | oxadiargyl |
| B.75 | oxyfluorfen |
| B.76 | saflufenacil |
| B.77 | sulfentrazone |
| B.78 | ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6) |
| B.79 | 3-[7-fluoro-3-oxo-4-(prop-2-ynyl)-3,4-dihydro-2H-benzo[1,4]-oxazin-6-yl]-1,5-dimethyl-6-thioxo-[1,3,5]triazinan-2,4-dione |
| B.80 | benzobicyclon |
| B.81 | clomazone |
| B.82 | diflufenican |
| B.83 | flurochloridone |
| B.84 | isoxaflutole |
| B.85 | mesotrione |

| | Herbicide B |
|---|---|
| B.86 | norflurazone |
| B.87 | picolinafen |
| B.88 | sulcotrione |
| B.89 | tefuryltrione |
| B.90 | tembotrione |
| B.91 | topramezone |
| B.92 | bicyclopyrone |
| B.93 | amitrole |
| B.94 | fluometuron |
| B.95 | glyphosate |
| B.96 | glyphosate-isopropylammonium |
| B.97 | glyphosate-trimesium (sulfosa-te) |
| B.98 | glufosinate |
| B.99 | glufosinate-P |
| B.100 | glufosinate-ammonium |
| B.101 | pendimethalin |
| B.102 | trifluralin |
| B.103 | acetochlor |
| B.104 | butachlor |
| B.105 | cafenstrole |
| B.106 | dimethenamid-P |
| B.107 | fentrazamide |
| B.108 | flufenacet |
| B.109 | mefenacet |
| B.110 | metazachlor |
| B.111 | metolachlor |
| B.112 | S-metolachlor |
| B.113 | pretilachlor |

| | Herbicide B |
|---|---|
| B.114 | fenoxasulfone |
| B.115 | isoxaben |
| B.116 | pyroxasulfone |
| B.117 | 2,4-D |
| B.118 | aminopyralid |
| B.119 | clopyralid |
| B.120 | dicamba |
| B.121 | fluroxypyr-meptyl |
| B.122 | MCPA |
| B.123 | quinclorac |
| B.124 | quinmerac |
| B.125 | aminocyclopyrachlor |
| B.126 | diflufenzopyr |
| B.127 | diflufenzopyr-sodium |
| B.128 | dymron |
| B.129 | indanofan |
| B.130 | indaziflam |
| B.131 | oxaziclomefone |
| B.132 | triaziflam |
| B.133 | II.1 |
| B.134 | II.2 |
| B.135 | II.3 |
| B.136 | II.4 |
| B.137 | II.5 |
| B.138 | II.6 |
| B.139 | II.7 |
| B.140 | II.8 |
| B.141 | II.9 |

Particularly preferred safeners C according to the invention are the safeners C as defined above; in particular the safeners C.1 - C.12, especially C.1 - C.11 listed below in table C:

Table C

| | Safener C |
|---|---|
| C.1 | benoxacor |
| C.2 | cloquintocet |
| C.3 | cyprosulfamide |

(continued)

| | Safener C |
|---|---|
| C.4 | dichlormid |
| C.5 | fenclorim |
| C.6 | fenchlorazole |
| C.7 | furilazole |
| C.8 | isoxadifen |
| C.9 | mefenpyr |
| C.10 | 4-(dichloroacetyl)-1-oxa-4-azaspiro[4.5]decane (MON4660, CAS 71526-07-3) |
| C.11 | 2,2,5-trimethyl-3-(dichloroacetyl)-1,3-oxazolidine (R-29148, CAS 52836-31-4) |
| C.12 | naphtalic acid anhydride |

The weight ratios of the individual components in the preferred mixtures mentioned below are within the limits given above, in particular within the preferred limits.

Particularly preferred are the compositions mentioned below,
comprising the crystalline form B of benzoxazinone (I)as defined and and the substance(s) as defined in the respective row of table 1;
especially preferred comprising as only herbicidal active compounds the crystalline form B of benzoxazinone (I) as defined and and the substance(s) as defined in the respective row of table 1;
most preferably comprising as only active compounds the crystalline form B of benzoxazinone (I)as defined and the substance(s) as defined in the respective row of table 1.

Particularly preferred are compositions 1.1 to 1.1833, especially 1.1 to 1.1692, comprising the crystalline form B of benzoxazinone (I) and the substance(s) as defined in the respective row of table 1:

Table 1(compositions 1.1 to 1.1833):

| comp. no. | herbicide B | safener C | comp. no. | herbicide B | safener C | comp. no. | herbicide B | safener C |
|---|---|---|---|---|---|---|---|---|
| 1.1 | B.1 | -- | 1.12 | B.12 | -- | 1.23 | B.23 | -- |
| 1.2 | B.2 | -- | 1.13 | B.13 | -- | 1.24 | B.24 | -- |
| 1.3 | B.3 | -- | 1.14 | B.14 | -- | 1.25 | B.25 | -- |
| 1.4 | B.4 | -- | 1.15 | B.15 | -- | 1.26 | B.26 | -- |
| 1.5 | B.5 | -- | 1.16 | B.16 | -- | 1.27 | B.27 | -- |
| 1.6 | B.6 | -- | 1.17 | B.17 | -- | 1.28 | B.28 | -- |
| 1.7 | B.7 | -- | 1.18 | B.18 | -- | 1.29 | B.29 | -- |
| 1.8 | B.8 | -- | 1.19 | B.19 | -- | 1.30 | B.30 | -- |
| 1.9 | B.9 | -- | 1.20 | B.20 | -- | 1.31 | B.31 | -- |
| 1.10 | B.10 | -- | 1.21 | B.21 | -- | 1.32 | B.32 | -- |
| 1.11 | B.11 | -- | 1.22 | B.22 | -- | 1.33 | B.33 | -- |

| comp. no. | herbi-cide B | safe-ner C |
|---|---|---|
| 1.34 | B.34 | -- |
| 1.35 | B.35 | -- |
| 1.36 | B.36 | -- |
| 1.37 | B.37 | -- |
| 1.38 | B.38 | -- |
| 1.39 | B.39 | -- |
| 1.40 | B.40 | -- |
| 1.41 | B.41 | -- |
| 1.42 | B.42 | -- |
| 1.43 | B.43 | -- |
| 1.44 | B.44 | -- |
| 1.45 | B.45 | -- |
| 1.46 | B.46 | -- |
| 1.47 | B.47 | -- |
| 1.48 | B.48 | -- |
| 1.49 | B.49 | -- |
| 1.50 | B.50 | -- |
| 1.51 | B.51 | -- |
| 1.52 | B.52 | -- |
| 1.53 | B.53 | -- |
| 1.54 | B.54 | -- |
| 1.55 | B.55 | -- |
| 1.56 | B.56 | -- |
| 1.57 | B.57 | -- |
| 1.58 | B.58. | -- |
| 1.59 | B.59 | -- |
| 1.60 | B.60 | -- |
| 1.61 | B.61 | -- |
| 1.62 | B.62 | -- |
| 1.63 | B.63 | -- |
| 1.64 | B.64 | -- |
| 1.65 | B.65 | -- |
| 1.66 | B.66 | -- |
| 1.67 | B.67 | -- |
| 1.68 | B.68 | -- |
| 1.69 | B.69 | -- |
| 1.70 | B.70 | -- |
| 1.71 | B.71 | -- |
| 1.72 | B.72 | -- |
| 1.73 | B.73 | -- |
| 1.74 | B.74 | -- |
| 1.75 | B.75 | -- |
| 1.76 | B.76 | -- |
| 1.77 | B.77 | -- |
| 1.78 | B.78 | -- |
| 1.79 | B.79 | -- |
| 1.80 | B.80 | -- |
| 1.81 | B.81 | -- |
| 1.82 | B.82 | -- |
| 1.83 | B.83 | -- |
| 1.84 | B.84 | -- |
| 1.85 | B.85 | -- |
| 1.86 | B.86 | -- |
| 1.87 | B.87 | -- |
| 1.88 | B.88 | -- |
| 1.89 | B.89 | -- |
| 1.90 | B.90 | -- |
| 1.91 | B.91 | -- |
| 1.92 | B.92 | -- |
| 1.93 | B.93 | -- |
| 1.94 | B.94 | -- |
| 1.95 | B.95 | -- |
| 1.96 | B.96 | -- |
| 1.97 | B.97 | -- |
| 1.98 | B.98 | -- |
| 1.99 | B.99 | -- |
| 1.100 | B.100 | -- |
| 1.101 | B.101 | -- |
| 1.102 | B.102 | -- |
| 1.103 | B.103 | -- |
| 1.104 | B.104 | -- |
| 1.105 | B.105 | -- |
| 1.106 | B.106 | -- |
| 1.107 | B.107 | -- |
| 1.108 | B.108 | -- |
| 1.109 | B.109 | -- |
| 1.110 | B.110 | -- |
| 1.111 | B.111 | -- |
| 1.112 | B.112 | -- |
| 1.113 | B.113 | -- |
| 1.114 | B.114 | -- |
| 1.115 | B.115 | -- |
| 1.116 | B.116 | -- |
| 1.117 | B.117 | -- |
| 1.118 | B.118 | -- |
| 1.119 | B.119 | -- |
| 1.120 | B.120 | -- |
| 1.121 | B.121 | -- |
| 1.122 | B.122 | -- |
| 1.123 | B.123 | -- |
| 1.124 | B.124 | -- |
| 1.125 | B.125 | -- |
| 1.126 | B.126 | -- |
| 1.127 | B.127 | -- |
| 1.128 | B.128 | -- |
| 1.129 | B.129 | -- |
| 1.130 | B.130 | -- |
| 1.131 | B.131 | -- |
| 1.132 | B.132 | -- |
| 1.133 | B.133 | -- |
| 1.134 | B.134 | -- |
| 1.135 | B.135 | -- |
| 1.136 | B.136 | -- |
| 1.137 | B.137 | -- |
| 1.138 | B.138 | -- |
| 1.139 | B.139 | |
| 1.140 | B.140 | -- |
| 1.141 | B.141 | -- |
| 1.142 | B.1 | C.1 |
| 1.143 | B.2 | C.1 |
| 1.144 | B.3 | C.1 |
| 1.145 | B.4 | C.1 |
| 1.146 | B.5 | C.1 |
| 1.147 | B.6 | C.1 |

| comp. no. | herbicide B | safener C |
|---|---|---|
| 1.148 | B.7 | C.1 |
| 1.149 | B.8 | C.1 |
| 1.150 | B.9 | C.1 |
| 1.151 | B.10 | C.1 |
| 1.152 | B.11 | C.1 |
| 1.153 | B.12 | C.1 |
| 1.154 | B.13 | C.1 |
| 1.155 | B.14 | C.1 |
| 1.156 | B.15 | C.1 |
| 1.157 | B.16 | C.1 |
| 1.158 | B.17 | C.1 |
| 1.159 | B.18 | C.1 |
| 1.160 | B.19 | C.1 |
| 1.161 | B.20 | C.1 |
| 1.162 | B.21 | C.1 |
| 1.163 | B.22 | C.1 |
| 1.164 | B.23 | C.1 |
| 1.165 | B.24 | C.1 |
| 1.166 | B.25 | C.1 |
| 1.167 | B.26 | C.1 |
| 1.168 | B.27 | C.1 |
| 1.169 | B.28 | C.1 |
| 1.170 | B.29 | C.1 |
| 1.171 | B.30 | C.1 |
| 1.172 | B.31 | C.1 |
| 1.173 | B.32 | C.1 |
| 1.174 | B.33 | C.1 |
| 1.175 | B.34 | C.1 |
| 1.176 | B.35 | C.1 |
| 1.177 | B.36 | C.1 |
| 1.178 | B.37 | C.1 |
| 1.179 | B.38 | C.1 |
| 1.180 | B.39 | C.1 |
| 1.181 | B.40 | C.1 |
| 1.182 | B.41 | C.1 |
| 1.183 | B.42 | C.1 |
| 1.184 | B.43 | C.1 |
| 1.185 | B.44 | C.1 |

| comp. no. | herbicide B | safener C |
|---|---|---|
| 1.186 | B.45 | C.1 |
| 1.187 | B.46 | C.1 |
| 1.188 | B.47 | C.1 |
| 1.189 | B.48 | C.1 |
| 1.190 | B.49 | C.1 |
| 1.191 | B.50 | C.1 |
| 1.192 | B.51 | C.1 |
| 1.193 | B.52 | C.1 |
| 1.194 | B.53 | C.1 |
| 1.195 | B.54 | C.1 |
| 1.196 | B.55 | C.1 |
| 1.197 | B.56 | C.1 |
| 1.198 | B.57 | C.1 |
| 1.199 | B.58. | C.1 |
| 1.200 | B.59 | C.1 |
| 1.201 | B.60 | C.1 |
| 1.202 | B.61 | C.1 |
| 1.203 | B.62 | C.1 |
| 1.204 | B.63 | C.1 |
| 1.205 | B.64 | C.1 |
| 1.206 | B.65 | C.1 |
| 1.207 | B.66 | C.1 |
| 1.208 | B.67 | C.1 |
| 1.209 | B.68 | C.1 |
| 1.210 | B.69 | C.1 |
| 1.211 | B.70 | C.1 |
| 1.212 | B.71 | C.1 |
| 1.213 | B.72 | C.1 |
| 1.214 | B.73 | C.1 |
| 1.215 | B.74 | C.1 |
| 1.216 | B.75 | C.1 |
| 1.217 | B.76 | C.1 |
| 1.218 | B.77 | C.1 |
| 1.219 | B.78 | C.1 |
| 1.220 | B.79 | C.1 |
| 1.221 | B.80 | C.1 |
| 1.222 | B.81 | C.1 |
| 1.223 | B.82 | C.1 |

| comp. no. | herbicide B | safener C |
|---|---|---|
| 1.224 | B.83 | C.1 |
| 1.225 | B.84 | C.1 |
| 1.226 | B.85 | C.1 |
| 1.227 | B.86 | C.1 |
| 1.228 | B.87 | C.1 |
| 1.229 | B.88 | C.1 |
| 1.230 | B.89 | C.1 |
| 1.231 | B.90 | C.1 |
| 1.232 | B.91 | C.1 |
| 1.233 | B.92 | C.1 |
| 1.234 | B.93 | C.1 |
| 1.235 | B.94 | C.1 |
| 1.236 | B.95 | C.1 |
| 1.237 | B.96 | C.1 |
| 1.238 | B.97 | C.1 |
| 1.239 | B.98 | C.1 |
| 1.240 | B.99 | C.1 |
| 1.241 | B.100 | C.1 |
| 1.242 | B.101 | C.1 |
| 1.243 | B.102 | C.1 |
| 1.244 | B.103 | C.1 |
| 1.245 | B.104 | C.1 |
| 1.246 | B.105 | C.1 |
| 1.247 | B.106 | C.1 |
| 1.248 | B.107 | C.1 |
| 1.249 | B.108 | C.1 |
| 1.250 | B.109 | C.1 |
| 1.251 | B.110 | C.1 |
| 1.252 | B.111 | C.1 |
| 1.253 | B.112 | C.1 |
| 1.254 | B.113 | C.1 |
| 1.255 | B.114 | C.1 |
| 1.256 | B.115 | C.1 |
| 1.257 | B.116 | C.1 |
| 1.258 | B.117 | C.1 |
| 1.259 | B.118 | C.1 |
| 1.260 | B.119 | C.1 |
| 1.261 | B.120 | C.1 |

| comp. no. | herbicide B | safener C |
|---|---|---|
| 1.262 | B.121 | C.1 |
| 1.263 | B.122 | C.1 |
| 1.264 | B.123 | C.1 |
| 1.265 | B.124 | C.1 |
| 1.266 | B.125 | C.1 |
| 1.267 | B.126 | C.1 |
| 1.268 | B.127 | C.1 |
| 1.269 | B.128 | C.1 |
| 1.270 | B.129 | C.1 |
| 1.271 | B.130 | C.1 |
| 1.272 | B.131 | C.1 |
| 1.273 | B.132 | C.1 |
| 1.274 | B.133 | C.1 |
| 1.275 | B.134 | C.1 |
| 1.276 | B.135 | C.1 |
| 1.277 | B.136 | C.1 |
| 1.278 | B.137 | C.1 |
| 1.279 | B.138 | C.1 |
| 1.280 | B.139 | C.1 |
| 1.281 | B.140 | C.1 |
| 1.282 | B.141 | C.1 |
| 1.283 | B.1 | C.2 |
| 1.284 | B.2 | C.2 |
| 1.285 | B.3 | C.2 |
| 1.286 | B.4 | C.2 |
| 1.287 | B.5 | C.2 |
| 1.288 | B.6 | C.2 |
| 1.289 | B.7 | C.2 |
| 1.290 | B.8 | C.2 |
| 1.291 | B.9 | C.2 |
| 1.292 | B.10 | C.2 |
| 1.293 | B.11 | C.2 |
| 1.294 | B.12 | C.2 |
| 1.295 | B.13 | C.2 |
| 1.296 | B.14 | C.2 |
| 1.297 | B.15 | C.2 |
| 1.298 | B.16 | C.2 |
| 1.299 | B.17 | C.2 |

| comp. no. | herbicide B | safener C |
|---|---|---|
| 1.300 | B.18 | C.2 |
| 1.301 | B.19 | C.2 |
| 1.302 | B.20 | C.2 |
| 1.303 | B.21 | C.2 |
| 1.304 | B.22 | C.2 |
| 1.305 | B.23 | C.2 |
| 1.306 | B.24 | C.2 |
| 1.307 | B.25 | C.2 |
| 1.308 | B.26 | C.2 |
| 1.309 | B.27 | C.2 |
| 1.310 | B.28 | C.2 |
| 1.311 | B.29 | C.2 |
| 1.312 | B.30 | C.2 |
| 1.313 | B.31 | C.2 |
| 1.314 | B.32 | C.2 |
| 1.315 | B.33 | C.2 |
| 1.316 | B.34 | C.2 |
| 1.317 | B.35 | C.2 |
| 1.318 | B.36 | C.2 |
| 1.319 | B.37 | C.2 |
| 1.320 | B.38 | C.2 |
| 1.321 | B.39 | C.2 |
| 1.322 | B.40 | C.2 |
| 1.323 | B.41 | C.2 |
| 1.324 | B.42 | C.2 |
| 1.325 | B.43 | C.2 |
| 1.326 | B.44 | C.2 |
| 1.327 | B.45 | C.2 |
| 1.328 | B.46 | C.2 |
| 1.329 | B.47 | C.2 |
| 1.330 | B.48 | C.2 |
| 1.331 | B.49 | C.2 |
| 1.332 | B.50 | C.2 |
| 1.333 | B.51 | C.2 |
| 1.334 | B.52 | C.2 |
| 1.335 | B.53 | C.2 |
| 1.336 | B.54 | C.2 |
| 1.337 | B.55 | C.2 |

| comp. no. | herbicide B | safener C |
|---|---|---|
| 1.338 | B.56 | C.2 |
| 1.339 | B.57 | C.2 |
| 1.340 | B.58. | C.2 |
| 1.341 | B.59 | C.2 |
| 1.342 | B.60 | C.2 |
| 1.343 | B.61 | C.2 |
| 1.344 | B.62 | C.2 |
| 1.345 | B.63 | C.2 |
| 1.346 | B.64 | C.2 |
| 1.347 | B.65 | C.2 |
| 1.348 | B.66 | C.2 |
| 1.349 | B.67 | C.2 |
| 1.350 | B.68 | C.2 |
| 1.351 | B.69 | C.2 |
| 1.352 | B.70 | C.2 |
| 1.353 | B.71 | C.2 |
| 1.354 | B.72 | C.2 |
| 1.355 | B.73 | C.2 |
| 1.356 | B.74 | C.2 |
| 1.357 | B.75 | C.2 |
| 1.358 | B.76 | C.2 |
| 1.359 | B.77 | C.2 |
| 1.360 | B.78 | C.2 |
| 1.361 | B.79 | C.2 |
| 1.362 | B.80 | C.2 |
| 1.363 | B.81 | C.2 |
| 1.364 | B.82 | C.2 |
| 1.365 | B.83 | C.2 |
| 1.366 | B.84 | C.2 |
| 1.367 | B.85 | C.2 |
| 1.368 | B.86 | C.2 |
| 1.369 | B.87 | C.2 |
| 1.370 | B.88 | C.2 |
| 1.371 | B.89 | C.2 |
| 1.372 | B.90 | C.2 |
| 1.373 | B.91 | C.2 |
| 1.374 | B.92 | C.2 |
| 1.375 | B.93 | C.2 |

| comp. no. | herbicide B | safener C |
|---|---|---|
| 1.376 | B.94 | C.2 |
| 1.377 | B.95 | C.2 |
| 1.378 | B.96 | C.2 |
| 1.379 | B.97 | C.2 |
| 1.380 | B.98 | C.2 |
| 1.381 | B.99 | C.2 |
| 1.382 | B.100 | C.2 |
| 1.383 | B.101 | C.2 |
| 1.384 | B.102 | C.2 |
| 1.385 | B.103 | C.2 |
| 1.386 | B.104 | C.2 |
| 1.387 | B.105 | C.2 |
| 1.388 | B.106 | C.2 |
| 1.389 | B.107 | C.2 |
| 1.390 | B.108 | C.2 |
| 1.391 | B.109 | C.2 |
| 1.392 | B.110 | C.2 |
| 1.393 | B.111 | C.2 |
| 1.394 | B.112 | C.2 |
| 1.395 | B.113 | C.2 |
| 1.396 | B.114 | C.2 |
| 1.397 | B.115 | C.2 |
| 1.398 | B.116 | C.2 |
| 1.399 | B.117 | C.2 |
| 1.400 | B.118 | C.2 |
| 1.401 | B.119 | C.2 |
| 1.402 | B.120 | C.2 |
| 1.403 | B.121 | C.2 |
| 1.404 | B.122 | C.2 |
| 1.405 | B.123 | C.2 |
| 1.406 | B.124 | C.2 |
| 1.407 | B.125 | C.2 |
| 1.408 | B.126 | C.2 |
| 1.409 | B.127 | C.2 |
| 1.410 | B.128 | C.2 |
| 1.411 | B.129 | C.2 |
| 1.412 | B.130 | C.2 |
| 1.413 | B.131 | C.2 |

| comp. no. | herbicide B | safener C |
|---|---|---|
| 1.414 | B.132 | C.2 |
| 1.415 | B.133 | C.2 |
| 1.416 | B.134 | C.2 |
| 1.417 | B.135 | C.2 |
| 1.418 | B.136 | C.2 |
| 1.419 | B.137 | C.2 |
| 1.420 | B.138 | C.2 |
| 1.421 | B.139 | C.2 |
| 1.422 | B.140 | C.2 |
| 1.423 | B.141 | C.2 |
| 1.424 | B.1 | C.3 |
| 1.425 | B.2 | C.3 |
| 1.426 | B.3 | C.3 |
| 1.427 | B.4 | C.3 |
| 1.428 | B.5 | C.3 |
| 1.429 | B.6 | C.3 |
| 1.430 | B.7 | C.3 |
| 1.431 | B.8 | C.3 |
| 1.432 | B.9 | C.3 |
| 1.433 | B.10 | C.3 |
| 1.434 | B.11 | C.3 |
| 1.435 | B.12 | C.3 |
| 1.436 | B.13 | C.3 |
| 1.437 | B.14 | C.3 |
| 1.438 | B.15 | C.3 |
| 1.439 | B.16 | C.3 |
| 1.440 | B.17 | C.3 |
| 1.441 | B.18 | C.3 |
| 1.442 | B.19 | C.3 |
| 1.443 | B.20 | C.3 |
| 1.444 | B.21 | C.3 |
| 1.445 | B.22 | C.3 |
| 1.446 | B.23 | C.3 |
| 1.447 | B.24 | C.3 |
| 1.448 | B.25 | C.3 |
| 1.449 | B.26 | C.3 |
| 1.450 | B.27 | C.3 |
| 1.451 | B.28 | C.3 |

| comp. no. | herbicide B | safener C |
|---|---|---|
| 1.452 | B.29 | C.3 |
| 1.453 | B.30 | C.3 |
| 1.454 | B.31 | C.3 |
| 1.455 | B.32 | C.3 |
| 1.456 | B.33 | C.3 |
| 1.457 | B.34 | C.3 |
| 1.458 | B.35 | C.3 |
| 1.459 | B.36 | C.3 |
| 1.460 | B.37 | C.3 |
| 1.461 | B.38 | C.3 |
| 1.462 | B.39 | C.3 |
| 1.463 | B.40 | C.3 |
| 1.464 | B.41 | C.3 |
| 1.465 | B.42 | C.3 |
| 1.466 | B.43 | C.3 |
| 1.467 | B.44 | C.3 |
| 1.468 | B.45 | C.3 |
| 1.469 | B.46 | C.3 |
| 1.470 | B.47 | C.3 |
| 1.471 | B.48 | C.3 |
| 1.472 | B.49 | C.3 |
| 1.473 | B.50 | C.3 |
| 1.474 | B.51 | C.3 |
| 1.475 | B.52 | C.3 |
| 1.476 | B.53 | C.3 |
| 1.477 | B.54 | C.3 |
| 1.478 | B.55 | C.3 |
| 1.479 | B.56 | C.3 |
| 1.480 | B.57 | C.3 |
| 1.481 | B.58. | C.3 |
| 1.482 | B.59 | C.3 |
| 1.483 | B.60 | C.3 |
| 1.484 | B.61 | C.3 |
| 1.485 | B.62 | C.3 |
| 1.486 | B.63 | C.3 |
| 1.487 | B.64 | C.3 |
| 1.488 | B.65 | C.3 |
| 1.489 | B.66 | C.3 |

| comp. no. | herbicide B | safener C |
|---|---|---|
| 1.490 | B.67 | C.3 |
| 1.491 | B.68 | C.3 |
| 1.492 | B.69 | C.3 |
| 1.493 | B.70 | C.3 |
| 1.494 | B.71 | C.3 |
| 1.495 | B.72 | C.3 |
| 1.496 | B.73 | C.3 |
| 1.497 | B.74 | C.3 |
| 1.498 | B.75 | C.3 |
| 1.499 | B.76 | C.3 |
| 1.500 | B.77 | C.3 |
| 1.501 | B.78 | C.3 |
| 1.502 | B.79 | C.3 |
| 1.503 | B.80 | C.3 |
| 1.504 | B.81 | C.3 |
| 1.505 | B.82 | C.3 |
| 1.506 | B.83 | C.3 |
| 1.507 | B.84 | C.3 |
| 1.508 | B.85 | C.3 |
| 1.509 | B.86 | C.3 |
| 1.510 | B.87 | C.3 |
| 1.511 | B.88 | C.3 |
| 1.512 | B.89 | C.3 |
| 1.513 | B.90 | C.3 |
| 1.514 | B.91 | C.3 |
| 1.515 | B.92 | C.3 |
| 1.516 | B.93 | C.3 |
| 1.517 | B.94 | C.3 |
| 1.518 | B.95 | C.3 |
| 1.519 | B.96 | C.3 |
| 1.520 | B.97 | C.3 |
| 1.521 | B.98 | C.3 |
| 1.522 | B.99 | C.3 |
| 1.523 | B.100 | C.3 |
| 1.524 | B.101 | C.3 |
| 1.525 | B.102 | C.3 |
| 1.526 | B.103 | C.3 |
| 1.527 | B.104 | C.3 |

| comp. no. | herbicide B | safener C |
|---|---|---|
| 1.528 | B.105 | C.3 |
| 1.529 | B.106 | C.3 |
| 1.530 | B.107 | C.3 |
| 1.531 | B.108 | C.3 |
| 1.532 | B.109 | C.3 |
| 1.533 | B.110 | C.3 |
| 1.534 | B.111 | C.3 |
| 1.535 | B.112 | C.3 |
| 1.536 | B.113 | C.3 |
| 1.537 | B.114 | C.3 |
| 1.538 | B.115 | C.3 |
| 1.539 | B.116 | C.3 |
| 1.540 | B.117 | C.3 |
| 1.541 | B.118 | C.3 |
| 1.542 | B.119 | C.3 |
| 1.543 | B.120 | C.3 |
| 1.544 | B.121 | C.3 |
| 1.545 | B.122 | C.3 |
| 1.546 | B.123 | C.3 |
| 1.547 | B.124 | C.3 |
| 1.548 | B.125 | C.3 |
| 1.549 | B.126 | C.3 |
| 1.550 | B.127 | C.3 |
| 1.551 | B.128 | C.3 |
| 1.552 | B.129 | C.3 |
| 1.553 | B.130 | C.3 |
| 1.554 | B.131 | C.3 |
| 1.555 | B.132 | C.3 |
| 1.556 | B.133 | C.3 |
| 1.557 | B.134 | C.3 |
| 1.558 | B.135 | C.3 |
| 1.559 | B.136 | C.3 |
| 1.560 | B.137 | C.3 |
| 1.561 | B.138 | C.3 |
| 1.562 | B.139 | C.3 |
| 1.563 | B.140 | C.3 |
| 1.564 | B.141 | C.3 |
| 1.565 | B.1 | C.4 |

| comp. no. | herbicide B | safener C |
|---|---|---|
| 1.566 | B.2 | C.4 |
| 1.567 | B.3 | C.4 |
| 1.568 | B.4 | C.4 |
| 1.569 | B.5 | C.4 |
| 1.570 | B.6 | C.4 |
| 1.571 | B.7 | C.4 |
| 1.572 | B.8 | C.4 |
| 1.573 | B.9 | C.4 |
| 1.574 | B.10 | C.4 |
| 1.575 | B.11 | C.4 |
| 1.576 | B.12 | C.4 |
| 1.577 | B.13 | C.4 |
| 1.578 | B.14 | C.4 |
| 1.579 | B.15 | C.4 |
| 1.580 | B.16 | C.4 |
| 1.581 | B.17 | C.4 |
| 1.582 | B.18 | C.4 |
| 1.583 | B.19 | C.4 |
| 1.584 | B.20 | C.4 |
| 1.585 | B.21 | C.4 |
| 1.586 | B.22 | C.4 |
| 1.587 | B.23 | C.4 |
| 1.588 | B.24 | C.4 |
| 1.589 | B.25 | C.4 |
| 1.590 | B.26 | C.4 |
| 1.591 | B.27 | C.4 |
| 1.592 | B.28 | C.4 |
| 1.593 | B.29 | C.4 |
| 1.594 | B.30 | C.4 |
| 1.595 | B.31 | C.4 |
| 1.596 | B.32 | C.4 |
| 1.597 | B.33 | C.4 |
| 1.598 | B.34 | C.4 |
| 1.599 | B.35 | C.4 |
| 1.600 | B.36 | C.4 |
| 1.601 | B.37 | C.4 |
| 1.602 | B.38 | C.4 |
| 1.603 | B.39 | C.4 |

| comp. no. | herbicide B | safener C |
|---|---|---|
| 1.604 | B.40 | C.4 |
| 1.605 | B.41 | C.4 |
| 1.606 | B.42 | C.4 |
| 1.607 | B.43 | C.4 |
| 1.608 | B.44 | C.4 |
| 1.609 | B.45 | C.4 |
| 1.610 | B.46 | C.4 |
| 1.611 | B.47 | C.4 |
| 1.612 | B.48 | C.4 |
| 1.613 | B.49 | C.4 |
| 1.614 | B.50 | C.4 |
| 1.615 | B.51 | C.4 |
| 1.616 | B.52 | C.4 |
| 1.617 | B.53 | C.4 |
| 1.618 | B.54 | C.4 |
| 1.619 | B.55 | C.4 |
| 1.620 | B.56 | C.4 |
| 1.621 | B.57 | C.4 |
| 1.622 | B.58. | C.4 |
| 1.623 | B.59 | C.4 |
| 1.624 | B.60 | C.4 |
| 1.625 | B.61 | C.4 |
| 1.626 | B.62 | C.4 |
| 1.627 | B.63 | C.4 |
| 1.628 | B.64 | C.4 |
| 1.629 | B.65 | C.4 |
| 1.630 | B.66 | C.4 |
| 1.631 | B.67 | C.4 |
| 1.632 | B.68 | C.4 |
| 1.633 | B.69 | C.4 |
| 1.634 | B.70 | C.4 |
| 1.635 | B.71 | C.4 |
| 1.636 | B.72 | C.4 |
| 1.637 | B.73 | C.4 |
| 1.638 | B.74 | C.4 |
| 1.639 | B.75 | C.4 |
| 1.640 | B.76 | C.4 |
| 1.641 | B.77 | C.4 |

| comp. no. | herbicide B | safener C |
|---|---|---|
| 1.642 | B.78 | C.4 |
| 1.643 | B.79 | C.4 |
| 1.644 | B.80 | C.4 |
| 1.645 | B.81 | C.4 |
| 1.646 | B.82 | C.4 |
| 1.647 | B.83 | C.4 |
| 1.648 | B.84 | C.4 |
| 1.649 | B.85 | C.4 |
| 1.650 | B.86 | C.4 |
| 1.651 | B.87 | C.4 |
| 1.652 | B.88 | C.4 |
| 1.653 | B.89 | C.4 |
| 1.654 | B.90 | C.4 |
| 1.655 | B.91 | C.4 |
| 1.656 | B.92 | C.4 |
| 1.657 | B.93 | C.4 |
| 1.658 | B.94 | C.4 |
| 1.659 | B.95 | C.4 |
| 1.660 | B.96 | C.4 |
| 1.661 | B.97 | C.4 |
| 1.662 | B.98 | C.4 |
| 1.663 | B.99 | C.4 |
| 1.664 | B.100 | C.4 |
| 1.665 | B.101 | C.4 |
| 1.666 | B.102 | C.4 |
| 1.667 | B.103 | C.4 |
| 1.668 | B.104 | C.4 |
| 1.669 | B.105 | C.4 |
| 1.670 | B.106 | C.4 |
| 1.671 | B.107 | C.4 |
| 1.672 | B.108 | C.4 |
| 1.673 | B.109 | C.4 |
| 1.674 | B.110 | C.4 |
| 1.675 | B.111 | C.4 |
| 1.676 | B.112 | C.4 |
| 1.677 | B.113 | C.4 |
| 1.678 | B.114 | C.4 |
| 1.679 | B.115 | C.4 |

| comp. no. | herbicide B | safener C |
|---|---|---|
| 1.680 | B.116 | C.4 |
| 1.681 | B.117 | C.4 |
| 1.682 | B.118 | C.4 |
| 1.683 | B.119 | C.4 |
| 1.684 | B.120 | C.4 |
| 1.685 | B.121 | C.4 |
| 1.686 | B.122 | C.4 |
| 1.687 | B.123 | C.4 |
| 1.688 | B.124 | C.4 |
| 1.689 | B.125 | C.4 |
| 1.690 | B.126 | C.4 |
| 1.691 | B.127 | C.4 |
| 1.692 | B.128 | C.4 |
| 1.693 | B.129 | C.4 |
| 1.694 | B.130 | C.4 |
| 1.695 | B.131 | C.4 |
| 1.696 | B.132 | C.4 |
| 1.697 | B.133 | C.4 |
| 1.698 | B.134 | C.4 |
| 1.699 | B.135 | C.4 |
| 1.700 | B.136 | C.4 |
| 1.701 | B.137 | C.4 |
| 1.702 | B.138 | C.4 |
| 1.703 | B.139 | C.4 |
| 1.704 | B.140 | C.4 |
| 1.705 | B.141 | C.4 |
| 1.706 | B.1 | C.5 |
| 1.707 | B.2 | C.5 |
| 1.708 | B.3 | C.5 |
| 1.709 | B.4 | C.5 |
| 1.710 | B.5 | C.5 |
| 1.711 | B.6 | C.5 |
| 1.712 | B.7 | C.5 |
| 1.713 | B.8 | C.5 |
| 1.714 | B.9 | C.5 |
| 1.715 | B.10 | C.5 |
| 1.716 | B.11 | C.5 |
| 1.717 | B.12 | C.5 |

| comp. no. | herbicide B | safener C |
|---|---|---|
| 1.718 | B.13 | C.5 |
| 1.719 | B.14 | C.5 |
| 1.720 | B.15 | C.5 |
| 1.721 | B.16 | C.5 |
| 1.722 | B.17 | C.5 |
| 1.723 | B.18 | C.5 |
| 1.724 | B.19 | C.5 |
| 1.725 | B.20 | C.5 |
| 1.726 | B.21 | C.5 |
| 1.727 | B.22 | C.5 |
| 1.728 | B.23 | C.5 |
| 1.729 | B.24 | C.5 |
| 1.730 | B.25 | C.5 |
| 1.731 | B.26 | C.5 |
| 1.732 | B.27 | C.5 |
| 1.733 | B.28 | C.5 |
| 1.734 | B.29 | C.5 |
| 1.735 | B.30 | C.5 |
| 1.736 | B.31 | C.5 |
| 1.737 | B.32 | C.5 |
| 1.738 | B.33 | C.5 |
| 1.739 | B.34 | C.5 |
| 1.740 | B.35 | C.5 |
| 1.741 | B.36 | C.5 |
| 1.742 | B.37 | C.5 |
| 1.743 | B.38 | C.5 |
| 1.744 | B.39 | C.5 |
| 1.745 | B.40 | C.5 |
| 1.746 | B.41 | C.5 |
| 1.747 | B.42 | C.5 |
| 1.748 | B.43 | C.5 |
| 1.749 | B.44 | C.5 |
| 1.750 | B.45 | C.5 |
| 1.751 | B.46 | C.5 |
| 1.752 | B.47 | C.5 |
| 1.753 | B.48 | C.5 |
| 1.754 | B.49 | C.5 |
| 1.755 | B.50 | C.5 |

| comp. no. | herbicide B | safener C |
|---|---|---|
| 1.756 | B.51 | C.5 |
| 1.757 | B.52 | C.5 |
| 1.758 | B.53 | C.5 |
| 1.759 | B.54 | C.5 |
| 1.760 | B.55 | C.5 |
| 1.761 | B.56 | C.5 |
| 1.762 | B.57 | C.5 |
| 1.763 | B.58. | C.5 |
| 1.764 | B.59 | C.5 |
| 1.765 | B.60 | C.5 |
| 1.766 | B.61 | C.5 |
| 1.767 | B.62 | C.5 |
| 1.768 | B.63 | C.5 |
| 1.769 | B.64 | C.5 |
| 1.770 | B.65 | C.5 |
| 1.771 | B.66 | C.5 |
| 1.772 | B.67 | C.5 |
| 1.773 | B.68 | C.5 |
| 1.774 | B.69 | C.5 |
| 1.775 | B.70 | C.5 |
| 1.776 | B.71 | C.5 |
| 1.777 | B.72 | C.5 |
| 1.778 | B.73 | C.5 |
| 1.779 | B.74 | C.5 |
| 1.780 | B.75 | C.5 |
| 1.781 | B.76 | C.5 |
| 1.782 | B.77 | C.5 |
| 1.783 | B.78 | C.5 |
| 1.784 | B.79 | C.5 |
| 1.785 | B.80 | C.5 |
| 1.786 | B.81 | C.5 |
| 1.787 | B.82 | C.5 |
| 1.788 | B.83 | C.5 |
| 1.789 | B.84 | C.5 |
| 1.790 | B.85 | C.5 |
| 1.791 | B.86 | C.5 |
| 1.792 | B.87 | C.5 |
| 1.793 | B.88 | C.5 |

| comp. no. | herbicide B | safener C |
|---|---|---|
| 1.794 | B.89 | C.5 |
| 1.795 | B.90 | C.5 |
| 1.796 | B.91 | C.5 |
| 1.797 | B.92 | C.5 |
| 1.798 | B.93 | C.5 |
| 1.799 | B.94 | C.5 |
| 1.800 | B.95 | C.5 |
| 1.801 | B.96 | C.5 |
| 1.802 | B.97 | C.5 |
| 1.803 | B.98 | C.5 |
| 1.804 | B.99 | C.5 |
| 1.805 | B.100 | C.5 |
| 1.806 | B.101 | C.5 |
| 1.807 | B.102 | C.5 |
| 1.808 | B.103 | C.5 |
| 1.809 | B.104 | C.5 |
| 1.810 | B.105 | C.5 |
| 1.811 | B.106 | C.5 |
| 1.812 | B.107 | C.5 |
| 1.813 | B.108 | C.5 |
| 1.814 | B.109 | C.5 |
| 1.815 | B.110 | C.5 |
| 1.816 | B.111 | C.5 |
| 1.817 | B.112 | C.5 |
| 1.818 | B.113 | C.5 |
| 1.819 | B.114 | C.5 |
| 1.820 | B.115 | C.5 |
| 1.821 | B.116 | C.5 |
| 1.822 | B.117 | C.5 |
| 1.823 | B.118 | C.5 |
| 1.824 | B.119 | C.5 |
| 1.825 | B.120 | C.5 |
| 1.826 | B.121 | C.5 |
| 1.827 | B.122 | C.5 |
| 1.828 | B.123 | C.5 |
| 1.829 | B.124 | C.5 |
| 1.830 | B.125 | C.5 |
| 1.831 | B.126 | C.5 |

| comp. no. | herbicide B | safener C |
|---|---|---|
| 1.832 | B.127 | C.5 |
| 1.833 | B.128 | C.5 |
| 1.834 | B.129 | C.5 |
| 1.835 | B.130 | C.5 |
| 1.836 | B.131 | C.5 |
| 1.837 | B.132 | C.5 |
| 1.838 | B.133 | C.5 |
| 1.839 | B.134 | C.5 |
| 1.840 | B.135 | C.5 |
| 1.841 | B.136 | C.5 |
| 1.842 | B.137 | C.5 |
| 1.843 | B.138 | C.5 |
| 1.844 | B.139 | C.5 |
| 1.845 | B.140 | C.5 |
| 1.846 | B.141 | C.5 |
| 1.847 | B.1 | C.6 |
| 1.848 | B.2 | C.6 |
| 1.849 | B.3 | C.6 |
| 1.850 | B.4 | C.6 |
| 1.851 | B.5 | C.6 |
| 1.852 | B.6 | C.6 |
| 1.853 | B.7 | C.6 |
| 1.854 | B.8 | C.6 |
| 1.855 | B.9 | C.6 |
| 1.856 | B.10 | C.6 |
| 1.857 | B.11 | C.6 |
| 1.858 | B.12 | C.6 |
| 1.859 | B.13 | C.6 |
| 1.860 | B.14 | C.6 |
| 1.861 | B.15 | C.6 |
| 1.862 | B.16 | C.6 |
| 1.863 | B.17 | C.6 |
| 1.864 | B.18 | C.6 |
| 1.865 | B.19 | C.6 |
| 1.866 | B.20 | C.6 |
| 1.867 | B.21 | C.6 |
| 1.868 | B.22 | C.6 |
| 1.869 | B.23 | C.6 |

| comp. no. | herbicide B | safener C |
|---|---|---|
| 1.870 | B.24 | C.6 |
| 1.871 | B.25 | C.6 |
| 1.872 | B.26 | C.6 |
| 1.873 | B.27 | C.6 |
| 1.874 | B.28 | C.6 |
| 1.875 | B.29 | C.6 |
| 1.876 | B.30 | C.6 |
| 1.877 | B.31 | C.6 |
| 1.878 | B.32 | C.6 |
| 1.879 | B.33 | C.6 |
| 1.880 | B.34 | C.6 |
| 1.881 | B.35 | C.6 |
| 1.882 | B.36 | C.6 |
| 1.883 | B.37 | C.6 |
| 1.884 | B.38 | C.6 |
| 1.885 | B.39 | C.6 |
| 1.886 | B.40 | C.6 |
| 1.887 | B.41 | C.6 |
| 1.888 | B.42 | C.6 |
| 1.889 | B.43 | C.6 |
| 1.890 | B.44 | C.6 |
| 1.891 | B.45 | C.6 |
| 1.892 | B.46 | C.6 |
| 1.893 | B.47 | C.6 |
| 1.894 | B.48 | C.6 |
| 1.895 | B.49 | C.6 |
| 1.896 | B.50 | C.6 |
| 1.897 | B.51 | C.6 |
| 1.898 | B.52 | C.6 |
| 1.899 | B.53 | C.6 |
| 1.900 | B.54 | C.6 |
| 1.901 | B.55 | C.6 |
| 1.902 | B.56 | C.6 |
| 1.903 | B.57 | C.6 |
| 1.904 | B.58. | C.6 |
| 1.905 | B.59 | C.6 |
| 1.906 | B.60 | C.6 |
| 1.907 | B.61 | C.6 |

| comp. no. | herbicide B | safener C |
|---|---|---|
| 1.908 | B.62 | C.6 |
| 1.909 | B.63 | C.6 |
| 1.910 | B.64 | C.6 |
| 1.911 | B.65 | C.6 |
| 1.912 | B.66 | C.6 |
| 1.913 | B.67 | C.6 |
| 1.914 | B.68 | C.6 |
| 1.915 | B.69 | C.6 |
| 1.916 | B.70 | C.6 |
| 1.917 | B.71 | C.6 |
| 1.918 | B.72 | C.6 |
| 1.919 | B.73 | C.6 |
| 1.920 | B.74 | C.6 |
| 1.921 | B.75 | C.6 |
| 1.922 | B.76 | C.6 |
| 1.923 | B.77 | C.6 |
| 1.924 | B.78 | C.6 |
| 1.925 | B.79 | C.6 |
| 1.926 | B.80 | C.6 |
| 1.927 | B.81 | C.6 |
| 1.928 | B.82 | C.6 |
| 1.929 | B.83 | C.6 |
| 1.930 | B.84 | C.6 |
| 1.931 | B.85 | C.6 |
| 1.932 | B.86 | C.6 |
| 1.933 | B.87 | C.6 |
| 1.934 | B.88 | C.6 |
| 1.935 | B.89 | C.6 |
| 1.936 | B.90 | C.6 |
| 1.937 | B.91 | C.6 |
| 1.938 | B.92 | C.6 |
| 1.939 | B.93 | C.6 |
| 1.940 | B.94 | C.6 |
| 1.941 | B.95 | C.6 |
| 1.942 | B.96 | C.6 |
| 1.943 | B.97 | C.6 |
| 1.944 | B.98 | C.6 |
| 1.945 | B.99 | C.6 |

| comp. no. | herbicide B | safener C |
|---|---|---|
| 1.946 | B.100 | C.6 |
| 1.947 | B.101 | C.6 |
| 1.948 | B.102 | C.6 |
| 1.949 | B.103 | C.6 |
| 1.950 | B.104 | C.6 |
| 1.951 | B.105 | C.6 |
| 1.952 | B.106 | C.6 |
| 1.953 | B.107 | C.6 |
| 1.954 | B.108 | C.6 |
| 1.955 | B.109 | C.6 |
| 1.956 | B.110 | C.6 |
| 1.957 | B.111 | C.6 |
| 1.958 | B.112 | C.6 |
| 1.959 | B.113 | C.6 |
| 1.960 | B.114 | C.6 |
| 1.961 | B.115 | C.6 |
| 1.962 | B.116 | C.6 |
| 1.963 | B.117 | C.6 |
| 1.964 | B.118 | C.6 |
| 1.965 | B.119 | C.6 |
| 1.966 | B.120 | C.6 |
| 1.967 | B.121 | C.6 |
| 1.968 | B.122 | C.6 |
| 1.969 | B.123 | C.6 |
| 1.970 | B.124 | C.6 |
| 1.971 | B.125 | C.6 |
| 1.972 | B.126 | C.6 |
| 1.973 | B.127 | C.6 |
| 1.974 | B.128 | C.6 |
| 1.975 | B.129 | C.6 |
| 1.976 | B.130 | C.6 |
| 1.977 | B.131 | C.6 |
| 1.978 | B.132 | C.6 |
| 1.979 | B.133 | C.6 |
| 1.980 | B.134 | C.6 |
| 1.981 | B.135 | C.6 |
| 1.982 | B.136 | C.6 |
| 1.983 | B.137 | C.6 |

| comp. no. | herbicide B | safener C |
|---|---|---|
| 1.984 | B.138 | C.6 |
| 1.985 | B.139 | C.6 |
| 1.986 | B.140 | C.6 |
| 1.987 | B.141 | C.6 |
| 1.988 | B.1 | C.7 |
| 1.989 | B.2 | C.7 |
| 1.990 | B.3 | C.7 |
| 1.991 | B.4 | C.7 |
| 1.992 | B.5 | C.7 |
| 1.993 | B.6 | C.7 |
| 1.994 | B.7 | C.7 |
| 1.995 | B.8 | C.7 |
| 1.996 | B.9 | C.7 |
| 1.997 | B.10 | C.7 |
| 1.998 | B.11 | C.7 |
| 1.999 | B.12 | C.7 |
| 1.1000 | B.13 | C.7 |
| 1.1001 | B.14 | C.7 |
| 1.1002 | B.15 | C.7 |
| 1.1003 | B.16 | C.7 |
| 1.1004 | B.17 | C.7 |
| 1.1005 | B.18 | C.7 |
| 1.1006 | B.19 | C.7 |
| 1.1007 | B.20 | C.7 |
| 1.1008 | B.21 | C.7 |
| 1.1009 | B.22 | C.7 |
| 1.1010 | B.23 | C.7 |
| 1.1011 | B.24 | C.7 |
| 1.1012 | B.25 | C.7 |
| 1.1013 | B.26 | C.7 |
| 1.1014 | B.27 | C.7 |
| 1.1015 | B.28 | C.7 |
| 1.1016 | B.29 | C.7 |
| 1.1017 | B.30 | C.7 |
| 1.1018 | B.31 | C.7 |
| 1.1019 | B.32 | C.7 |
| 1.1020 | B.33 | C.7 |
| 1.1021 | B.34 | C.7 |

| comp. no. | herbicide B | safener C |
|---|---|---|
| 1.1022 | B.35 | C.7 |
| 1.1023 | B.36 | C.7 |
| 1.1024 | B.37 | C.7 |
| 1.1025 | B.38 | C.7 |
| 1.1026 | B.39 | C.7 |
| 1.1027 | B.40 | C.7 |
| 1.1028 | B.41 | C.7 |
| 1.1029 | B.42 | C.7 |
| 1.1030 | B.43 | C.7 |
| 1.1031 | B.44 | C.7 |
| 1.1032 | B.45 | C.7 |
| 1.1033 | B.46 | C.7 |
| 1.1034 | B.47 | C.7 |
| 1.1035 | B.48 | C.7 |
| 1.1036 | B.49 | C.7 |
| 1.1037 | B.50 | C.7 |
| 1.1038 | B.51 | C.7 |
| 1.1039 | B.52 | C.7 |
| 1.1040 | B.53 | C.7 |
| 1.1041 | B.54 | C.7 |
| 1.1042 | B.55 | C.7 |
| 1.1043 | B.56 | C.7 |
| 1.1044 | B.57 | C.7 |
| 1.1045 | B.58. | C.7 |
| 1.1046 | B.59 | C.7 |
| 1.1047 | B.60 | C.7 |
| 1.1048 | B.61 | C.7 |
| 1.1049 | B.62 | C.7 |
| 1.1050 | B.63 | C.7 |
| 1.1051 | B.64 | C.7 |
| 1.1052 | B.65 | C.7 |
| 1.1053 | B.66 | C.7 |
| 1.1054 | B.67 | C.7 |
| 1.1055 | B.68 | C.7 |
| 1.1056 | B.69 | C.7 |
| 1.1057 | B.70 | C.7 |
| 1.1058 | B.71 | C.7 |
| 1.1059 | B.72 | C.7 |

| comp. no. | herbicide B | safener C |
|---|---|---|
| 1.1060 | B.73 | C.7 |
| 1.1061 | B.74 | C.7 |
| 1.1062 | B.75 | C.7 |
| 1.1063 | B.76 | C.7 |
| 1.1064 | B.77 | C.7 |
| 1.1065 | B.78 | C.7 |
| 1.1066 | B.79 | C.7 |
| 1.1067 | B.80 | C.7 |
| 1.1068 | B.81 | C.7 |
| 1.1069 | B.82 | C.7 |
| 1.1070 | B.83 | C.7 |
| 1.1071 | B.84 | C.7 |
| 1.1072 | B.85 | C.7 |
| 1.1073 | B.86 | C.7 |
| 1.1074 | B.87 | C.7 |
| 1.1075 | B.88 | C.7 |
| 1.1076 | B.89 | C.7 |
| 1.1077 | B.90 | C.7 |
| 1.1078 | B.91 | C.7 |
| 1.1079 | B.92 | C.7 |
| 1.1080 | B.93 | C.7 |
| 1.1081 | B.94 | C.7 |
| 1.1082 | B.95 | C.7 |
| 1.1083 | B.96 | C.7 |
| 1.1084 | B.97 | C.7 |
| 1.1085 | B.98 | C.7 |
| 1.1086 | B.99 | C.7 |
| 1.1087 | B.100 | C.7 |
| 1.1088 | B.101 | C.7 |
| 1.1089 | B.102 | C.7 |
| 1.1090 | B.103 | C.7 |
| 1.1091 | B.104 | C.7 |
| 1.1092 | B.105 | C.7 |
| 1.1093 | B.106 | C.7 |
| 1.1094 | B.107 | C.7 |
| 1.1095 | B.108 | C.7 |
| 1.1096 | B.109 | C.7 |
| 1.1097 | B.110 | C.7 |

| comp. no. | herbicide B | safener C |
|---|---|---|
| 1.1098 | B.111 | C.7 |
| 1.1099 | B.112 | C.7 |
| 1.1100 | B.113 | C.7 |
| 1.1101 | B.114 | C.7 |
| 1.1102 | B.115 | C.7 |
| 1.1103 | B.116 | C.7 |
| 1.1104 | B.117 | C.7 |
| 1.1105 | B.118 | C.7 |
| 1.1106 | B.119 | C.7 |
| 1.1107 | B.120 | C.7 |
| 1.1108 | B.121 | C.7 |
| 1.1109 | B.122 | C.7 |
| 1.1110 | B.123 | C.7 |
| 1.1111 | B.124 | C.7 |
| 1.1112 | B.125 | C.7 |
| 1.1113 | B.126 | C.7 |
| 1.1114 | B.127 | C.7 |
| 1.1115 | B.128 | C.7 |
| 1.1116 | B.129 | C.7 |
| 1.1117 | B.130 | C.7 |
| 1.1118 | B.131 | C.7 |
| 1.1119 | B.132 | C.7 |
| 1.1120 | B.133 | C.7 |
| 1.1121 | B.134 | C.7 |
| 1.1122 | B.135 | C.7 |
| 1.1123 | B.136 | C.7 |
| 1.1124 | B.137 | C.7 |
| 1.1125 | B.138 | C.7 |
| 1.1126 | B.139 | C.7 |
| 1.1127 | B.140 | C.7 |
| 1.1128 | B.141 | C.7 |
| 1.1129 | B.1 | C.8 |
| 1.1130 | B.2 | C.8 |
| 1.1131 | B.3 | C.8 |
| 1.1132 | B.4 | C.8 |
| 1.1133 | B.5 | C.8 |
| 1.1134 | B.6 | C.8 |
| 1.1135 | B.7 | C.8 |

| comp. no. | herbicide B | safener C |
|---|---|---|
| 1.1136 | B.8 | C.8 |
| 1.1137 | B.9 | C.8 |
| 1.1138 | B.10 | C.8 |
| 1.1139 | B.11 | C.8 |
| 1.1140 | B.12 | C.8 |
| 1.1141 | B.13 | C.8 |
| 1.1142 | B.14 | C.8 |
| 1.1143 | B.15 | C.8 |
| 1.1144 | B.16 | C.8 |
| 1.1145 | B.17 | C.8 |
| 1.1146 | B.18 | C.8 |
| 1.1147 | B.19 | C.8 |
| 1.1148 | B.20 | C.8 |
| 1.1149 | B.21 | C.8 |
| 1.1150 | B.22 | C.8 |
| 1.1151 | B.23 | C.8 |
| 1.1152 | B.24 | C.8 |
| 1.1153 | B.25 | C.8 |
| 1.1154 | B.26 | C.8 |
| 1.1155 | B.27 | C.8 |
| 1.1156 | B.28 | C.8 |
| 1.1157 | B.29 | C.8 |
| 1.1158 | B.30 | C.8 |
| 1.1159 | B.31 | C.8 |
| 1.1160 | B.32 | C.8 |
| 1.1161 | B.33 | C.8 |
| 1.1162 | B.34 | C.8 |
| 1.1163 | B.35 | C.8 |
| 1.1164 | B.36 | C.8 |
| 1.1165 | B.37 | C.8 |
| 1.1166 | B.38 | C.8 |
| 1.1167 | B.39 | C.8 |
| 1.1168 | B.40 | C.8 |
| 1.1169 | B.41 | C.8 |
| 1.1170 | B.42 | C.8 |
| 1.1171 | B.43 | C.8 |
| 1.1172 | B.44 | C.8 |
| 1.1173 | B.45 | C.8 |

| comp. no. | herbicide B | safener C |
|---|---|---|
| 1.1174 | B.46 | C.8 |
| 1.1175 | B.47 | C.8 |
| 1.1176 | B.48 | C.8 |
| 1.1177 | B.49 | C.8 |
| 1.1178 | B.50 | C.8 |
| 1.1179 | B.51 | C.8 |
| 1.1180 | B.52 | C.8 |
| 1.1181 | B.53 | C.8 |
| 1.1182 | B.54 | C.8 |
| 1.1183 | B.55 | C.8 |
| 1.1184 | B.56 | C.8 |
| 1.1185 | B.57 | C.8 |
| 1.1186 | B.58. | C.8 |
| 1.1187 | B.59 | C.8 |
| 1.1188 | B.60 | C.8 |
| 1.1189 | B.61 | C.8 |
| 1.1190 | B.62 | C.8 |
| 1.1191 | B.63 | C.8 |
| 1.1192 | B.64 | C.8 |
| 1.1193 | B.65 | C.8 |
| 1.1194 | B.66 | C.8 |
| 1.1195 | B.67 | C.8 |
| 1.1196 | B.68 | C.8 |
| 1.1197 | B.69 | C.8 |
| 1.1198 | B.70 | C.8 |
| 1.1199 | B.71 | C.8 |
| 1.1200 | B.72 | C.8 |
| 1.1201 | B.73 | C.8 |
| 1.1202 | B.74 | C.8 |
| 1.1203 | B.75 | C.8 |
| 1.1204 | B.76 | C.8 |
| 1.1205 | B.77 | C.8 |
| 1.1206 | B.78 | C.8 |
| 1.1207 | B.79 | C.8 |
| 1.1208 | B.80 | C.8 |
| 1.1209 | B.81 | C.8 |
| 1.1210 | B.82 | C.8 |
| 1.1211 | B.83 | C.8 |

| comp. no. | herbicide B | safener C |
|---|---|---|
| 1.1212 | B.84 | C.8 |
| 1.1213 | B.85 | C.8 |
| 1.1214 | B.86 | C.8 |
| 1.1215 | B.87 | C.8 |
| 1.1216 | B.88 | C.8 |
| 1.1217 | B.89 | C.8 |
| 1.1218 | B.90 | C.8 |
| 1.1219 | B.91 | C.8 |
| 1.1220 | B.92 | C.8 |
| 1.1221 | B.93 | C.8 |
| 1.1222 | B.94 | C.8 |
| 1.1223 | B.95 | C.8 |
| 1.1224 | B.96 | C.8 |
| 1.1225 | B.97 | C.8 |
| 1.1226 | B.98 | C.8 |
| 1.1227 | B.99 | C.8 |
| 1.1228 | B.100 | C.8 |
| 1.1229 | B.101 | C.8 |
| 1.1230 | B.102 | C.8 |
| 1.1231 | B.103 | C.8 |
| 1.1232 | B.104 | C.8 |
| 1.1233 | B.105 | C.8 |
| 1.1234 | B.106 | C.8 |
| 1.1235 | B.107 | C.8 |
| 1.1236 | B.108 | C.8 |
| 1.1237 | B.109 | C.8 |
| 1.1238 | B.110 | C.8 |
| 1.1239 | B.111 | C.8 |
| 1.1240 | B.112 | C.8 |
| 1.1241 | B.113 | C.8 |
| 1.1242 | B.114 | C.8 |
| 1.1243 | B.115 | C.8 |
| 1.1244 | B.116 | C.8 |
| 1.1245 | B.117 | C.8 |
| 1.1246 | B.118 | C.8 |
| 1.1247 | B.119 | C.8 |
| 1.1248 | B.120 | C.8 |
| 1.1249 | B.121 | C.8 |

| comp. no. | herbicide B | safener C |
|---|---|---|
| 1.1250 | B.122 | C.8 |
| 1.1251 | B.123 | C.8 |
| 1.1252 | B.124 | C.8 |
| 1.1253 | B.125 | C.8 |
| 1.1254 | B.126 | C.8 |
| 1.1255 | B.127 | C.8 |
| 1.1256 | B.128 | C.8 |
| 1.1257 | B.129 | C.8 |
| 1.1258 | B.130 | C.8 |
| 1.1259 | B.131 | C.8 |
| 1.1260 | B.132 | C.8 |
| 1.1261 | B.133 | C.8 |
| 1.1262 | B.134 | C.8 |
| 1.1263 | B.135 | C.8 |
| 1.1264 | B.136 | C.8 |
| 1.1265 | B.137 | C.8 |
| 1.1266 | B.138 | C.8 |
| 1.1267 | B.139 | C.8 |
| 1.1268 | B.140 | C.8 |
| 1.1269 | B.141 | C.8 |
| 1.1270 | B.1 | C.9 |
| 1.1271 | B.2 | C.9 |
| 1.1272 | B.3 | C.9 |
| 1.1273 | B.4 | C.9 |
| 1.1274 | B.5 | C.9 |
| 1.1275 | B.6 | C.9 |
| 1.1276 | B.7 | C.9 |
| 1.1277 | B.8 | C.9 |
| 1.1278 | B.9 | C.9 |
| 1.1279 | B.10 | C.9 |
| 1.1280 | B.11 | C.9 |
| 1.1281 | B.12 | C.9 |
| 1.1282 | B.13 | C.9 |
| 1.1283 | B.14 | C.9 |
| 1.1284 | B.15 | C.9 |
| 1.1285 | B.16 | C.9 |
| 1.1286 | B.17 | C.9 |
| 1.1287 | B.18 | C.9 |

| comp. no. | herbicide B | safener C |
|---|---|---|
| 1.1288 | B.19 | C.9 |
| 1.1289 | B.20 | C.9 |
| 1.1290 | B.21 | C.9 |
| 1.1291 | B.22 | C.9 |
| 1.1292 | B.23 | C.9 |
| 1.1293 | B.24 | C.9 |
| 1.1294 | B.25 | C.9 |
| 1.1295 | B.26 | C.9 |
| 1.1296 | B.27 | C.9 |
| 1.1297 | B.28 | C.9 |
| 1.1298 | B.29 | C.9 |
| 1.1299 | B.30 | C.9 |
| 1.1300 | B.31 | C.9 |
| 1.1301 | B.32 | C.9 |
| 1.1302 | B.33 | C.9 |
| 1.1303 | B.34 | C.9 |
| 1.1304 | B.35 | C.9 |
| 1.1305 | B.36 | C.9 |
| 1.1306 | B.37 | C.9 |
| 1.1307 | B.38 | C.9 |
| 1.1308 | B.39 | C.9 |
| 1.1309 | B.40 | C.9 |
| 1.1310 | B.41 | C.9 |
| 1.1311 | B.42 | C.9 |
| 1.1312 | B.43 | C.9 |
| 1.1313 | B.44 | C.9 |
| 1.1314 | B.45 | C.9 |
| 1.1315 | B.46 | C.9 |
| 1.1316 | B.47 | C.9 |
| 1.1317 | B.48 | C.9 |
| 1.1318 | B.49 | C.9 |
| 1.1319 | B.50 | C.9 |
| 1.1320 | B.51 | C.9 |
| 1.1321 | B.52 | C.9 |
| 1.1322 | B.53 | C.9 |
| 1.1323 | B.54 | C.9 |
| 1.1324 | B.55 | C.9 |
| 1.1325 | B.56 | C.9 |

| comp. no. | herbicide B | safener C |
|---|---|---|
| 1.1326 | B.57 | C.9 |
| 1.1327 | B.58. | C.9 |
| 1.1328 | B.59 | C.9 |
| 1.1329 | B.60 | C.9 |
| 1.1330 | B.61 | C.9 |
| 1.1331 | B.62 | C.9 |
| 1.1332 | B.63 | C.9 |
| 1.1333 | B.64 | C.9 |
| 1.1334 | B.65 | C.9 |
| 1.1335 | B.66 | C.9 |
| 1.1336 | B.67 | C.9 |
| 1.1337 | B.68 | C.9 |
| 1.1338 | B.69 | C.9 |
| 1.1339 | B.70 | C.9 |
| 1.1340 | B.71 | C.9 |
| 1.1341 | B.72 | C.9 |
| 1.1342 | B.73 | C.9 |
| 1.1343 | B.74 | C.9 |
| 1.1344 | B.75 | C.9 |
| 1.1345 | B.76 | C.9 |
| 1.1346 | B.77 | C.9 |
| 1.1347 | B.78 | C.9 |
| 1.1348 | B.79 | C.9 |
| 1.1349 | B.80 | C.9 |
| 1.1350 | B.81 | C.9 |
| 1.1351 | B.82 | C.9 |
| 1.1352 | B.83 | C.9 |
| 1.1353 | B.84 | C.9 |
| 1.1354 | B.85 | C.9 |
| 1.1355 | B.86 | C.9 |
| 1.1356 | B.87 | C.9 |
| 1.1357 | B.88 | C.9 |
| 1.1358 | B.89 | C.9 |
| 1.1359 | B.90 | C.9 |
| 1.1360 | B.91 | C.9 |
| 1.1361 | B.92 | C.9 |
| 1.1362 | B.93 | C.9 |
| 1.1363 | B.94 | C.9 |

| comp. no. | herbicide B | safener C |
|---|---|---|
| 1.1364 | B.95 | C.9 |
| 1.1365 | B.96 | C.9 |
| 1.1366 | B.97 | C.9 |
| 1.1367 | B.98 | C.9 |
| 1.1368 | B.99 | C.9 |
| 1.1369 | B.100 | C.9 |
| 1.1370 | B.101 | C.9 |
| 1.1371 | B.102 | C.9 |
| 1.1372 | B.103 | C.9 |
| 1.1373 | B.104 | C.9 |
| 1.1374 | B.105 | C.9 |
| 1.1375 | B.106 | C.9 |
| 1.1376 | B.107 | C.9 |
| 1.1377 | B.108 | C.9 |
| 1.1378 | B.109 | C.9 |
| 1.1379 | B.110 | C.9 |
| 1.1380 | B.111 | C.9 |
| 1.1381 | B.112 | C.9 |
| 1.1382 | B.113 | C.9 |
| 1.1383 | B.114 | C.9 |
| 1.1384 | B.115 | C.9 |
| 1.1385 | B.116 | C.9 |
| 1.1386 | B.117 | C.9 |
| 1.1387 | B.118 | C.9 |
| 1.1388 | B.119 | C.9 |
| 1.1389 | B.120 | C.9 |
| 1.1390 | B.121 | C.9 |
| 1.1391 | B.122 | C.9 |
| 1.1392 | B.123 | C.9 |
| 1.1393 | B.124 | C.9 |
| 1.1394 | B.125 | C.9 |
| 1.1395 | B.126 | C.9 |
| 1.1396 | B.127 | C.9 |
| 1.1397 | B.128 | C.9 |
| 1.1398 | B.129 | C.9 |
| 1.1399 | B.130 | C.9 |
| 1.1400 | B.131 | C.9 |
| 1.1401 | B.132 | C.9 |

| comp. no. | herbicide B | safener C |
|---|---|---|
| 1.1402 | B.133 | C.9 |
| 1.1403 | B.134 | C.9 |
| 1.1404 | B.135 | C.9 |
| 1.1405 | B.136 | C.9 |
| 1.1406 | B.137 | C.9 |
| 1.1407 | B.138 | C.9 |
| 1.1408 | B.139 | C.9 |
| 1.1409 | B.140 | C.9 |
| 1.1410 | B.141 | C.9 |
| 1.1411 | B.1 | C.10 |
| 1.1412 | B.2 | C.10 |
| 1.1413 | B.3 | C.10 |
| 1.1414 | B.4 | C.10 |
| 1.1415 | B.5 | C.10 |
| 1.1416 | B.6 | C.10 |
| 1.1417 | B.7 | C.10 |
| 1.1418 | B.8 | C.10 |
| 1.1419 | B.9 | C.10 |
| 1.1420 | B.10 | C.10 |
| 1.1421 | B.11 | C.10 |
| 1.1422 | B.12 | C.10 |
| 1.1423 | B.13 | C.10 |
| 1.1424 | B.14 | C.10 |
| 1.1425 | B.15 | C.10 |
| 1.1426 | B.16 | C.10 |
| 1.1427 | B.17 | C.10 |
| 1.1428 | B.18 | C.10 |
| 1.1429 | B.19 | C.10 |
| 1.1430 | B.20 | C.10 |
| 1.1431 | B.21 | C.10 |
| 1.1432 | B.22 | C.10 |
| 1.1433 | B.23 | C.10 |
| 1.1434 | B.24 | C.10 |
| 1.1435 | B.25 | C.10 |
| 1.1436 | B.26 | C.10 |
| 1.1437 | B.27 | C.10 |
| 1.1438 | B.28 | C.10 |
| 1.1439 | B.29 | C.10 |

| comp. no. | herbicide B | safener C |
|---|---|---|
| 1.1440 | B.30 | C.10 |
| 1.1441 | B.31 | C.10 |
| 1.1442 | B.32 | C.10 |
| 1.1443 | B.33 | C.10 |
| 1.1444 | B.34 | C.10 |
| 1.1445 | B.35 | C.10 |
| 1.1446 | B.36 | C.10 |
| 1.1447 | B.37 | C.10 |
| 1.1448 | B.38 | C.10 |
| 1.1449 | B.39 | C.10 |
| 1.1450 | B.40 | C.10 |
| 1.1451 | B.41 | C.10 |
| 1.1452 | B.42 | C.10 |
| 1.1453 | B.43 | C.10 |
| 1.1454 | B.44 | C.10 |
| 1.1455 | B.45 | C.10 |
| 1.1456 | B.46 | C.10 |
| 1.1457 | B.47 | C.10 |
| 1.1458 | B.48 | C.10 |
| 1.1459 | B.49 | C.10 |
| 1.1460 | B.50 | C.10 |
| 1.1461 | B.51 | C.10 |
| 1.1462 | B.52 | C.10 |
| 1.1463 | B.53 | C.10 |
| 1.1464 | B.54 | C.10 |
| 1.1465 | B.55 | C.10 |
| 1.1466 | B.56 | C.10 |
| 1.1467 | B.57 | C.10 |
| 1.1468 | B.58. | C.10 |
| 1.1469 | B.59 | C.10 |
| 1.1470 | B.60 | C.10 |
| 1.1471 | B.61 | C.10 |
| 1.1472 | B.62 | C.10 |
| 1.1473 | B.63 | C.10 |
| 1.1474 | B.64 | C.10 |
| 1.1475 | B.65 | C.10 |
| 1.1476 | B.66 | C.10 |
| 1.1477 | B.67 | C.10 |

| comp. no. | herbicide B | safener C |
|---|---|---|
| 1.1478 | B.68 | C.10 |
| 1.1479 | B.69 | C.10 |
| 1.1480 | B.70 | C.10 |
| 1.1481 | B.71 | C.10 |
| 1.1482 | B.72 | C.10 |
| 1.1483 | B.73 | C.10 |
| 1.1484 | B.74 | C.10 |
| 1.1485 | B.75 | C.10 |
| 1.1486 | B.76 | C.10 |
| 1.1487 | B.77 | C.10 |
| 1.1488 | B.78 | C.10 |
| 1.1489 | B.79 | C.10 |
| 1.1490 | B.80 | C.10 |
| 1.1491 | B.81 | C.10 |
| 1.1492 | B.82 | C.10 |
| 1.1493 | B.83 | C.10 |
| 1.1494 | B.84 | C.10 |
| 1.1495 | B.85 | C.10 |
| 1.1496 | B.86 | C.10 |
| 1.1497 | B.87 | C.10 |
| 1.1498 | B.88 | C.10 |
| 1.1499 | B.89 | C.10 |
| 1.1500 | B.90 | C.10 |
| 1.1501 | B.91 | C.10 |
| 1.1502 | B.92 | C.10 |
| 1.1503 | B.93 | C.10 |
| 1.1504 | B.94 | C.10 |
| 1.1505 | B.95 | C.10 |
| 1.1506 | B.96 | C.10 |
| 1.1507 | B.97 | C.10 |
| 1.1508 | B.98 | C.10 |
| 1.1509 | B.99 | C.10 |
| 1.1510 | B.100 | C.10 |
| 1.1511 | B.101 | C.10 |
| 1.1512 | B.102 | C.10 |
| 1.1513 | B.103 | C.10 |
| 1.1514 | B.104 | C.10 |
| 1.1515 | B.105 | C.10 |

| comp. no. | herbicide B | safener C |
|---|---|---|
| 1.1516 | B.106 | C.10 |
| 1.1517 | B.107 | C.10 |
| 1.1518 | B.108 | C.10 |
| 1.1519 | B.109 | C.10 |
| 1.1520 | B.110 | C.10 |
| 1.1521 | B.111 | C.10 |
| 1.1522 | B.112 | C.10 |
| 1.1523 | B.113 | C.10 |
| 1.1524 | B.114 | C.10 |
| 1.1525 | B.115 | C.10 |
| 1.1526 | B.116 | C.10 |
| 1.1527 | B.117 | C.10 |
| 1.1528 | B.118 | C.10 |
| 1.1529 | B.119 | C.10 |
| 1.1530 | B.120 | C.10 |
| 1.1531 | B.121 | C.10 |
| 1.1532 | B.122 | C.10 |
| 1.1533 | B.123 | C.10 |
| 1.1534 | B.124 | C.10 |
| 1.1535 | B.125 | C.10 |
| 1.1536 | B.126 | C.10 |
| 1.1537 | B.127 | C.10 |
| 1.1538 | B.128 | C.10 |
| 1.1539 | B.129 | C.10 |
| 1.1540 | B.130 | C.10 |
| 1.1541 | B.131 | C.10 |
| 1.1542 | B.132 | C.10 |
| 1.1543 | B.133 | C.10 |
| 1.1544 | B.134 | C.10 |
| 1.1545 | B.135 | C.10 |
| 1.1546 | B.136 | C.10 |
| 1.1547 | B.137 | C.10 |
| 1.1548 | B.138 | C.10 |
| 1.1549 | B.139 | C.10 |
| 1.1550 | B.140 | C.10 |
| 1.1551 | B.141 | C.10 |
| 1.1552 | B.1 | C.11 |
| 1.1553 | B.2 | C.11 |
| 1.1554 | B.3 | C.11 |
| 1.1555 | B.4 | C.11 |
| 1.1556 | B.5 | C.11 |
| 1.1557 | B.6 | C.11 |
| 1.1558 | B.7 | C.11 |
| 1.1559 | B.8 | C.11 |
| 1.1560 | B.9 | C.11 |
| 1.1561 | B.10 | C.11 |
| 1.1562 | B.11 | C.11 |
| 1.1563 | B.12 | C.11 |
| 1.1564 | B.13 | C.11 |
| 1.1565 | B.14 | C.11 |
| 1.1566 | B.15 | C.11 |
| 1.1567 | B.16 | C.11 |
| 1.1568 | B.17 | C.11 |
| 1.1569 | B.18 | C.11 |
| 1.1570 | B.19 | C.11 |
| 1.1571 | B.20 | C.11 |
| 1.1572 | B.21 | C.11 |
| 1.1573 | B.22 | C.11 |
| 1.1574 | B.23 | C.11 |
| 1.1575 | B.24 | C.11 |
| 1.1576 | B.25 | C.11 |
| 1.1577 | B.26 | C.11 |
| 1.1578 | B.27 | C.11 |
| 1.1579 | B.28 | C.11 |
| 1.1580 | B.29 | C.11 |
| 1.1581 | B.30 | C.11 |
| 1.1582 | B.31 | C.11 |
| 1.1583 | B.32 | C.11 |
| 1.1584 | B.33 | C.11 |
| 1.1585 | B.34 | C.11 |
| 1.1586 | B.35 | C.11 |
| 1.1587 | B.36 | C.11 |
| 1.1588 | B.37 | C.11 |
| 1.1589 | B.38 | C.11 |
| 1.1590 | B.39 | C.11 |
| 1.1591 | B.40 | C.11 |
| 1.1592 | B.41 | C.11 |
| 1.1593 | B.42 | C.11 |
| 1.1594 | B.43 | C.11 |
| 1.1595 | B.44 | C.11 |
| 1.1596 | B.45 | C.11 |
| 1.1597 | B.46 | C.11 |
| 1.1598 | B.47 | C.11 |
| 1.1599 | B.48 | C.11 |
| 1.1600 | B.49 | C.11 |
| 1.1601 | B.50 | C.11 |
| 1.1602 | B.51 | C.11 |
| 1.1603 | B.52 | C.11 |
| 1.1604 | B.53 | C.11 |
| 1.1605 | B.54 | C.11 |
| 1.1606 | B.55 | C.11 |
| 1.1607 | B.56 | C.11 |
| 1.1608 | B.57 | C.11 |
| 1.1609 | B.58. | C.11 |
| 1.1610 | B.59 | C.11 |
| 1.1611 | B.60 | C.11 |
| 1.1612 | B.61 | C.11 |
| 1.1613 | B.62 | C.11 |
| 1.1614 | B.63 | C.11 |
| 1.1615 | B.64 | C.11 |
| 1.1616 | B.65 | C.11 |
| 1.1617 | B.66 | C.11 |
| 1.1618 | B.67 | C.11 |
| 1.1619 | B.68 | C.11 |
| 1.1620 | B.69 | C.11 |
| 1.1621 | B.70 | C.11 |
| 1.1622 | B.71 | C.11 |
| 1.1623 | B.72 | C.11 |
| 1.1624 | B.73 | C.11 |
| 1.1625 | B.74 | C.11 |
| 1.1626 | B.75 | C.11 |
| 1.1627 | B.76 | C.11 |
| 1.1628 | B.77 | C.11 |
| 1.1629 | B.78 | C.11 |

| comp. no. | herbicide B | safener C |
|---|---|---|
| 1.1630 | B.79 | C.11 |
| 1.1631 | B.80 | C.11 |
| 1.1632 | B.81 | C.11 |
| 1.1633 | B.82 | C.11 |
| 1.1634 | B.83 | C.11 |
| 1.1635 | B.84 | C.11 |
| 1.1636 | B.85 | C.11 |
| 1.1637 | B.86 | C.11 |
| 1.1638 | B.87 | C.11 |
| 1.1639 | B.88 | C.11 |
| 1.1640 | B.89 | C.11 |
| 1.1641 | B.90 | C.11 |
| 1.1642 | B.91 | C.11 |
| 1.1643 | B.92 | C.11 |
| 1.1644 | B.93 | C.11 |
| 1.1645 | B.94 | C.11 |
| 1.1646 | B.95 | C.11 |
| 1.1647 | B.96 | C.11 |
| 1.1648 | B.97 | C.11 |
| 1.1649 | B.98 | C.11 |
| 1.1650 | B.99 | C.11 |
| 1.1651 | B.100 | C.11 |
| 1.1652 | B.101 | C.11 |
| 1.1653 | B.102 | C.11 |
| 1.1654 | B.103 | C.11 |
| 1.1655 | B.104 | C.11 |
| 1.1656 | B.105 | C.11 |
| 1.1657 | B.106 | C.11 |
| 1.1658 | B.107 | C.11 |
| 1.1659 | B.108 | C.11 |
| 1.1660 | B.109 | C.11 |
| 1.1661 | B.110 | C.11 |
| 1.1662 | B.111 | C.11 |
| 1.1663 | B.112 | C.11 |
| 1.1664 | B.113 | C.11 |
| 1.1665 | B.114 | C.11 |
| 1.1666 | B.115 | C.11 |
| 1.1667 | B.116 | C.11 |

| comp. no. | herbicide B | safener C |
|---|---|---|
| 1.1668 | B.117 | C.11 |
| 1.1669 | B.118 | C.11 |
| 1.1670 | B.119 | C.11 |
| 1.1671 | B.120 | C.11 |
| 1.1672 | B.121 | C.11 |
| 1.1673 | B.122 | C.11 |
| 1.1674 | B.123 | C.11 |
| 1.1675 | B.124 | C.11 |
| 1.1676 | B.125 | C.11 |
| 1.1677 | B.126 | C.11 |
| 1.1678 | B.127 | C.11 |
| 1.1679 | B.128 | C.11 |
| 1.1680 | B.129 | C.11 |
| 1.1681 | B.130 | C.11 |
| 1.1682 | B.131 | C.11 |
| 1.1683 | B.132 | C.11 |
| 1.1684 | B.133 | C.11 |
| 1.1685 | B.134 | C.11 |
| 1.1686 | B.135 | C.11 |
| 1.1687 | B.136 | C.11 |
| 1.1688 | B.137 | C.11 |
| 1.1689 | B.138 | C.11 |
| 1.1690 | B.139 | C.11 |
| 1.1691 | B.140 | C.11 |
| 1.1692 | B.141 | C.11 |
| 1.1693 | B.1 | C.12 |
| 1.1694 | B.2 | C.12 |
| 1.1695 | B.3 | C.12 |
| 1.1696 | B.4 | C.12 |
| 1.1697 | B.5 | C.12 |
| 1.1698 | B.6 | C.12 |
| 1.1699 | B.7 | C.12 |
| 1.1700 | B.8 | C.12 |
| 1.1701 | B.9 | C.12 |
| 1.1702 | B.10 | C.12 |
| 1.1703 | B.11 | C.12 |
| 1.1704 | B.12 | C.12 |
| 1.1705 | B.13 | C.12 |

| comp. no. | herbicide B | safener C |
|---|---|---|
| 1.1706 | B.14 | C.12 |
| 1.1707 | B.15 | C.12 |
| 1.1708 | B.16 | C.12 |
| 1.1709 | B.17 | C.12 |
| 1.1710 | B.18 | C.12 |
| 1.1711 | B.19 | C.12 |
| 1.1712 | B.20 | C.12 |
| 1.1713 | B.21 | C.12 |
| 1.1714 | B.22 | C.12 |
| 1.1715 | B.23 | C.12 |
| 1.1716 | B.24 | C.12 |
| 1.1717 | B.25 | C.12 |
| 1.1718 | B.26 | C.12 |
| 1.1719 | B.27 | C.12 |
| 1.1720 | B.28 | C.12 |
| 1.1721 | B.29 | C.12 |
| 1.1722 | B.30 | C.12 |
| 1.1723 | B.31 | C.12 |
| 1.1724 | B.32 | C.12 |
| 1.1725 | B.33 | C.12 |
| 1.1726 | B.34 | C.12 |
| 1.1727 | B.35 | C.12 |
| 1.1728 | B.36 | C.12 |
| 1.1729 | B.37 | C.12 |
| 1.1730 | B.38 | C.12 |
| 1.1731 | B.39 | C.12 |
| 1.1732 | B.40 | C.12 |
| 1.1733 | B.41 | C.12 |
| 1.1734 | B.42 | C.12 |
| 1.1735 | B.43 | C.12 |
| 1.1736 | B.44 | C.12 |
| 1.1737 | B.45 | C.12 |
| 1.1738 | B.46 | C.12 |
| 1.1739 | B.47 | C.12 |
| 1.1740 | B.48 | C.12 |
| 1.1741 | B.49 | C.12 |
| 1.1742 | B.50 | C.12 |
| 1.1743 | B.51 | C.12 |

| comp. no. | herbicide B | safener C |
|---|---|---|
| 1.1744 | B.52 | C.12 |
| 1.1745 | B.53 | C.12 |
| 1.1746 | B.54 | C.12 |
| 1.1747 | B.55 | C.12 |
| 1.1748 | B.56 | C.12 |
| 1.1749 | B.57 | C.12 |
| 1.1750 | B.58. | C.12 |
| 1.1751 | B.59 | C.12 |
| 1.1752 | B.60 | C.12 |
| 1.1753 | B.61 | C.12 |
| 1.1754 | B.62 | C.12 |
| 1.1755 | B.63 | C.12 |
| 1.1756 | B.64 | C.12 |
| 1.1757 | B.65 | C.12 |
| 1.1758 | B.66 | C.12 |
| 1.1759 | B.67 | C.12 |
| 1.1760 | B.68 | C.12 |
| 1.1761 | B.69 | C.12 |
| 1.1762 | B.70 | C.12 |
| 1.1763 | B.71 | C.12 |
| 1.1764 | B.72 | C.12 |
| 1.1765 | B.73 | C.12 |
| 1.1766 | B.74 | C.12 |
| 1.1767 | B.75 | C.12 |
| 1.1768 | B.76 | C.12 |
| 1.1769 | B.77 | C.12 |
| 1.1770 | B.78 | C.12 |
| 1.1771 | B.79 | C.12 |
| 1.1772 | B.80 | C.12 |
| 1.1773 | B.81 | C.12 |

| comp. no. | herbicide B | safener C |
|---|---|---|
| 1.1774 | B.82 | C.12 |
| 1.1775 | B.83 | C.12 |
| 1.1776 | B.84 | C.12 |
| 1.1777 | B.85 | C.12 |
| 1.1778 | B.86 | C.12 |
| 1.1779 | B.87 | C.12 |
| 1.1780 | B.88 | C.12 |
| 1.1781 | B.89 | C.12 |
| 1.1782 | B.90 | C.12 |
| 1.1783 | B.91 | C.12 |
| 1.1784 | B.92 | C.12 |
| 1.1785 | B.93 | C.12 |
| 1.1786 | B.94 | C.12 |
| 1.1787 | B.95 | C.12 |
| 1.1788 | B.96 | C.12 |
| 1.1789 | B.97 | C.12 |
| 1.1790 | B.98 | C.12 |
| 1.1791 | B.99 | C.12 |
| 1.1792 | B.100 | C.12 |
| 1.1793 | B.101 | C.12 |
| 1.1794 | B.102 | C.12 |
| 1.1795 | B.103 | C.12 |
| 1.1796 | B.104 | C.12 |
| 1.1797 | B.105 | C.12 |
| 1.1798 | B.106 | C.12 |
| 1.1799 | B.107 | C.12 |
| 1.1800 | B.108 | C.12 |
| 1.1801 | B.109 | C.12 |
| 1.1802 | B.110 | C.12 |
| 1.1803 | B.111 | C.12 |

| comp. no. | herbicide B | safener C |
|---|---|---|
| 1.1804 | B.112 | C.12 |
| 1.1805 | B.113 | C.12 |
| 1.1806 | B.114 | C.12 |
| 1.1807 | B.115 | C.12 |
| 1.1808 | B.116 | C.12 |
| 1.1809 | B.117 | C.12 |
| 1.1810 | B.118 | C.12 |
| 1.1811 | B.119 | C.12 |
| 1.1812 | B.120 | C.12 |
| 1.1813 | B.121 | C.12 |
| 1.1814 | B.122 | C.12 |
| 1.1815 | B.123 | C.12 |
| 1.1816 | B.124 | C.12 |
| 1.1817 | B.125 | C.12 |
| 1.1818 | B.126 | C.12 |
| 1.1819 | B.127 | C.12 |
| 1.1820 | B.128 | C.12 |
| 1.1821 | B.129 | C.12 |
| 1.1822 | B.130 | C.12 |
| 1.1823 | B.131 | C.12 |
| 1.1824 | B.132 | C.12 |
| 1.1825 | B.133 | C.12 |
| 1.1826 | B.134 | C.12 |
| 1.1827 | B.135 | C.12 |
| 1.1828 | B.136 | C.12 |
| 1.1829 | B.137 | C.12 |
| 1.1830 | B.138 | C.12 |
| 1.1831 | B.139 | C.12 |
| 1.1832 | B.140 | C.12 |
| 1.1833 | B.141 | C.12 |

Also especially preferred are compositions 69.1 to 69.12, preferably compositions 69.1 to 69.11, comprising the crystalline form B of benzoxazinone (I) and as further compound the substance as defined in the respective row of table C:

Table C

| comp. no. | safener C | | comp. no. | safener C |
|---|---|---|---|---|
| 69.1 | C.1 | | 69.7 | C.7 |
| 69.2 | C.2 | | 69.8 | C.8 |
| 69.3 | C.3 | | 69.9 | C.9 |
| 69.4 | C.4 | | 69.10 | C.10 |
| 69.5 | C.5 | | 69.11 | C.11 |
| 69.6 | C.6 | | 69.12 | C.12 |

The specific number for each single composition is deductible as follows:

Composition 1.777 for example comprises the crystalline form B of benzoxazinone (I), flumioxazin (B.72) and fenclorim (C.5) (see table 1, entry 1.777; as well as table B, entry B.72 and table C, entry C.5).
Composition 7.777 for example comprises imazaquin (B.32) (see the definition for compositions 7.1 to 7.1692 below), and the crystalline form B of benzoxazinone (I), flumioxazin (B.72) and fenclorim (C.5) (see table 1, entry 1.777; as well as table B, entry B.77 and table C, entry C.5).

Also especially preferred are compositions 2.1 to 2.1833, preferably compositions 2.1 to 2.1692, which differ from the corresponding compositions 1.1 to 1.1833, preferably compositions 1.1 to 1.1692, only in that they additionally comprise B.2 as further herbicide B.
Also especially preferred are compositions 3.1 to 3.1833, preferably compositions 3.1 to 3.1692, which differ from the corresponding compositions 1.1 to 1.1833, preferably compositions 1.1 to 1.1692, only in that they additionally comprise B.7 as further herbicide B.
Also especially preferred are compositions 4.1 to 4.1833, preferably compositions 4.1 to 4.1692, which differ from the corresponding compositions 1.1 to 1.1833, preferably compositions 1.1 to 1.1692, only in that they additionally comprise B.29 as further herbicide B.
Also especially preferred are compositions 5.1 to 5.1833, preferably compositions 5.1 to 5.1692, which differ from the corresponding compositions 1.1 to 1.1833, preferably compositions 1.1 to 1.1692, only in that they additionally comprise B.30 as further herbicide B.
Also especially preferred are compositions 6.1 to 6.1833, preferably compositions 6.1 to 6.1692, which differ from the corresponding compositions 1.1 to 1.1833, preferably compositions 1.1 to 1.1692, only in that they additionally comprise B.31 as further herbicide B.
Also especially preferred are compositions 7.1 to 7.1833, preferably compositions 7.17 to 7.1692, which differ from the corresponding compositions 1.1 to 1.1833, preferably compositions 1.1 to 1.1692, only in that they additionally comprise B.32 as further herbicide B.
Also especially preferred are compositions 8.1 to 8.1833, preferably compositions 8.1 to 8.1692, which differ from the corresponding compositions 1.1 to 1.1833, preferably compositions 1.1 to 1.1692, only in that they additionally comprise B.33 as further herbicide B.
Also especially preferred are compositions 9.1 to 9.1833, preferably compositions 9.1 to 9.1692, which differ from the corresponding compositions 1.1 to 1.1833, preferably compositions 1.1 to 1.1692, only in that they additionally comprise B.40 as further herbicide B.
Also especially preferred are compositions 10.1 to 10.1833, preferably compositions 10.1. to 10.1692, which differ from the corresponding compositions 1.1 to 1.1833, preferably compositions 1.1 to 1.1692, only in that they additionally comprise B.44 as further herbicide B.
Also especially preferred are compositions 11.1 to 11.1833, preferably compositions 11.1 to 11.1692, which differ from the corresponding compositions 1.1 to 1.1833, preferably compositions 1.1 to 1.1692, only in that they additionally comprise B.45 as further herbicide B.
Also especially preferred are compositions 12.1 to 12.1833, preferably compositions 12.1 to 12.1692, which differ from the corresponding compositions 1.1 to 1.1833, preferably compositions 1.1 to 1.1692, only in that they additionally comprise B.52 as further herbicide B.
Also especially preferred are compositions 13.1 to 13.1833, preferably compositions 13.1 to 13.1692, which differ from the corresponding compositions 1.1 to 1.1833, preferably compositions 1.1 to 1.1692, only in that they additionally comprise B.53 as further herbicide B.
Also especially preferred are compositions 14.1 to 14.1833, preferably compositions 14.1 to 14.1692, which

differ from the corresponding compositions 1.1 to 1.1833, preferably compositions 1.1 to 1.1692, only in that they additionally comprise B.54 as further herbicide B.

Also especially preferred are compositions 15.1 to 15.1833, preferably compositions 15.1 to 15.1692, which differ from the corresponding compositions 1.1 to 1.1833, preferably compositions 1.1 to 1.1692, only in that they additionally comprise B.55 as further herbicide B.

Also especially preferred are compositions 16.1 to 16.1833, preferably compositions 16.1 to 16.1692, which differ from the corresponding compositions 1.1 to 1.1833, preferably compositions 1.1 to 1.1692, only in that they additionally comprise B.56 as further herbicide B.

Also especially preferred are compositions 17.1 to 17.1833, preferably compositions 17.1 to 17.1692, which differ from the corresponding compositions 1.1 to 1.1833, preferably compositions 1.1 to 1.1692, only in that they additionally comprise B.57 as further herbicide B.

Also especially preferred are compositions 18.1 to 18.1833, preferably compositions 18.1 to 18.1692, which differ from the corresponding compositions 1.1 to 1.1833, preferably compositions 1.1 to 1.1692, only in that they additionally comprise B.60 as further herbicide B.

Also especially preferred are compositions 19.1 to 19.1833, preferably compositions 19.1 to 19.1692, which differ from the corresponding compositions 1.1 to 1.1833, preferably compositions 1.1 to 1.1692, only in that they additionally comprise B.65 as further herbicide B.

Also especially preferred are compositions 20.1 to 20.1833, preferably compositions 20.1 to 20.1692, which differ from the corresponding compositions 1.1 to 1.1833, preferably compositions 1.1 to 1.1692, only in that they additionally comprise B.66 as further herbicide B.

Also especially preferred are compositions 21.1 to 21.1833, preferably compositions 21.1 to 21.1692, which differ from the corresponding compositions 1.1 to 1.1833, preferably compositions 1.1 to 1.1692, only in that they additionally comprise B.69 as further herbicide B.

Also especially preferred are compositions 22.1 to 22.1833, preferably compositions 22.1 to 22.1692, which differ from the corresponding compositions 1.1 to 1.1833, preferably compositions 1.1 to 1.1692, only in that they additionally comprise B.72 as further herbicide B.

Also especially preferred are compositions 23.1 to 23.1833, preferably compositions 23.1 to 23.1692, which differ from the corresponding compositions 1.1 to 1.1833, preferably compositions 1.1 to 1.1692, only in that they additionally comprise B.73 as further herbicide B.

Also especially preferred are compositions 24.1 to 24.1833, preferably compositions 24.1 to 24.1692, which differ from the corresponding compositions 1.1 to 1.1833, preferably compositions 1.1 to 1.1692, only in that they additionally comprise B.76 as further herbicide B.

Also especially preferred are compositions 25.1 to 25.1833, preferably compositions 25.1 to 25.1692, which differ from the corresponding compositions 1.1 to 1.1833, preferably compositions 1.1 to 1.1692, only in that they additionally comprise B.77 as further herbicide B.

Also especially preferred are compositions 26.1 to 26.1833, preferably compositions 26.1 to 26.1692, which differ from the corresponding compositions 1.1 to 1.1833, preferably compositions 1.1 to 1.1692, only in that they additionally comprise B.80 as further herbicide B.

Also especially preferred are compositions 27.1 to 27.1833, preferably compositions 27.1 to 27.1692, which differ from the corresponding compositions 1.1 to 1.1833, preferably compositions 1.1 to 1.1692, only in that they additionally comprise B.81 as further herbicide B.

Also especially preferred are compositions 28.1 to 28.1833, preferably compositions 28.1 to 28.1692, which differ from the corresponding compositions 1.1 to 1.1833, preferably compositions 1.1 to 1.1692, only in that they additionally comprise B.84 as further herbicide B.

Also especially preferred are compositions 29.1 to 29.1833, preferably compositions 29.1 to 29.1692, which differ from the corresponding compositions 1.1 to 1.1833, preferably compositions 1.1 to 1.1692, only in that they additionally comprise B.84 and B.54 as further herbicides B.

Also especially preferred are compositions 30.1 to 30.1833, preferably compositions 30.1 to 30.1692, which differ from the corresponding compositions 1.1 to 1.1833, preferably compositions 1.1 to 1.1692, only in that they additionally comprise B.84 and B.60 as further herbicides B.

Also especially preferred are compositions 31.1 to 31.1833, preferably compositions 31.1 to 31.1692, which differ from the corresponding compositions 1.1 to 1.1833, preferably compositions 1.1 to 1.1692, only in that they additionally comprise B.84 and B.66 as further herbicides B.

Also especially preferred are compositions 32.1 to 32.1833, preferably compositions 32.1 to 32.1692, which differ from the corresponding compositions 1.1 to 1.1833, preferably compositions 1.1 to 1.1692, only in that they additionally comprise B. 85 as further herbicide B.

Also especially preferred are compositions 33.1 to 33.1833, preferably compositions 33.1 to 33.1692, which differ from the corresponding compositions 1.1 to 1.1833, preferably compositions 1.1 to 1.1692, only in that they additionally comprise B. 85 and B.54 as further herbicides B.

Also especially preferred are compositions 34.1 to 34.1833, preferably compositions 34.1 to 34.1692, which differ from the corresponding compositions 1.1 to 1.1833, preferably compositions 1.1 to 1.1692, only in that they additionally comprise B. 85 and B.60 as further herbicides B.

Also especially preferred are compositions 35.1 to 35.1833, preferably compositions 35.1 to 35.1692, which differ from the corresponding compositions 1.1 to 1.1833, preferably compositions 1.1 to 1.1692, only in that they additionally comprise B. 85 and B.66 as further herbicides B.

Also especially preferred are compositions 36.1 to 36.1833, preferably compositions 36.1 to 36.1692, which differ from the corresponding compositions 1.1 to 1.1833, preferably compositions 1.1 to 1.1692, only in that they additionally comprise B.87 as further herbicide B.

Also especially preferred are compositions 37.1 to 37.1833, preferably compositions 37.1 to 37.1692, which differ from the corresponding compositions 1.1 to 1.1833, preferably compositions 1.1 to 1.1692, only in that they additionally comprise B. 88 as further herbicide B.

Also especially preferred are compositions 38.1 to 38.1833, preferably compositions 38.1 to 38.1692, which differ from the corresponding compositions 1.1 to 1.1833, preferably compositions 1.1 to 1.1692, only in that they additionally comprise B. 88 and B.54 as further herbicides B.

Also especially preferred are compositions 39.1 to 39.1833, preferably compositions 39.1 to 39.1692, which differ from the corresponding compositions 1.1 to 1.1833, preferably compositions 1.1 to 1.1692, only in that they additionally comprise B. 88 and B.60 as further herbicides B.

Also especially preferred are compositions 40.1 to 40.1833, preferably compositions 40.1 to 40.1692, which differ from the corresponding compositions 1.1 to 1.1833, preferably compositions 1.1 to 1.1692, only in that they additionally comprise B. 88 and B.66 as further herbicides B.

Also especially preferred are compositions 41.1 to 41.1833, preferably compositions 41.1 to 41.1692, which differ from the corresponding compositions 1.1 to 1.1833, preferably compositions 1.1 to 1.1692, only in that they additionally comprise B.90 as further herbicide B.

Also especially preferred are compositions 42.1 to 42.1833, preferably compositions 42.1 to 42.1692, which differ from the corresponding compositions 1.1 to 1.1833, preferably compositions 1.1 to 1.1692, only in that they additionally comprise B.91 as further herbicide B.

Also especially preferred are compositions 43.1 to 43.1833, preferably compositions 43.1 to 43.1692, which differ from the corresponding compositions 1.1 to 1.1833, preferably compositions 1.1 to 1.1692, only in that they additionally comprise B.91 and B.54 as further herbicides B.

Also especially preferred are compositions 44.1 to 44.1833, preferably compositions 44.1 to 44.1692, which differ from the corresponding compositions 1.1 to 1.1833, preferably compositions 1.1 to 1.1692, only in that they additionally comprise B.91 and B.60 as further herbicides B.

Also especially preferred are compositions 45.1 to 45.1833, preferably compositions 45.1 to 45.1692, which differ from the corresponding compositions 1.1 to 1.1833, preferably compositions 1.1 to 1.1692, only in that they additionally comprise B.91 and B.66 as further herbicides B.

Also especially preferred are compositions 46.1 to 46.1833, preferably compositions 46.1 to 46.1692, which differ from the corresponding compositions 1.1 to 1.1833, preferably compositions 1.1 to 1.1692, only in that they additionally comprise B.95 as further herbicide B.

Also especially preferred are compositions 47.1 to 47.1833, preferably compositions 47.1 to 47.1692, which differ from the corresponding compositions 1.1 to 1.1833, preferably compositions 1.1 to 1.1692, only in that they additionally comprise B.95 and B.54 as further herbicides B.

Also especially preferred are compositions 48.1 to 48.1833, preferably compositions 48.1 to 48.1692, which differ from the corresponding compositions 1.1 to 1.1833, preferably compositions 1.1 to 1.1692, only in that they additionally comprise B.95 and B.76 as further herbicides B.

Also especially preferred are compositions 49.1 to 49.1833, preferably compositions 49.1 to 49.1692, which differ from the corresponding compositions 1.1 to 1.1833, preferably compositions 1.1 to 1.1692, only in that they additionally comprise B.95 and B.84 as further herbicides B.

Also especially preferred are compositions 50.1 to 50.1833, preferably compositions 50.1 to 50.1692, which differ from the corresponding compositions 1.1 to 1.1833, preferably compositions 1.1 to 1.1692, only in that they additionally comprise B.95 and B.103 as further herbicides B.

Also especially preferred are compositions 51.1 to 51.1833, preferably compositions 51.1 to 51.1692, which differ from the corresponding compositions 1.1 to 1.1833, preferably compositions 1.1 to 1.1692, only in that they additionally comprise B.95 and B.85 as further herbicides B.

Also especially preferred are compositions 52.1 to 52.1833, preferably compositions 52.1 to 52.1692, which differ from the corresponding compositions 1.1 to 1.1833, preferably compositions 1.1 to 1.1692, only in that they additionally comprise B.95 and B.88 as further herbicides B.

Also especially preferred are compositions 53.1 to 53.1833, preferably compositions 53.1 to 53.1692, which differ from the corresponding compositions 1.1 to 1.1833, preferably compositions 1.1 to 1.1692, only in that they additionally comprise B.95 and B.91 as further herbicides B.
Also especially preferred are compositions 54.1 to 54.1833, preferably compositions 54.1 to 54.1692, which differ from the corresponding compositions 1.1 to 1.1833, preferably compositions 1.1 to 1.1692, only in that they additionally comprise B.98 as further herbicide B.
Also especially preferred are compositions 55.1 to 55.1833, preferably compositions 55.1 to 55.1692, which differ from the corresponding compositions 1.1 to 1.1833, preferably compositions 1.1 to 1.1692, only in that they additionally comprise B.101 as further herbicide B.
Also especially preferred are compositions 56.1 to 56.1833, preferably compositions 56.1 to 56.1692, which differ from the corresponding compositions 1.1 to 1.1833, preferably compositions 1.1 to 1.1692, only in that they additionally comprise B.103 as further herbicide B.
Also especially preferred are compositions 57.1 to 57.1833, preferably compositions 57.1 to 57.1692, which differ from the corresponding compositions 1.1 to 1.1833, preferably compositions 1.1 to 1.1692, only in that they additionally comprise B.106 as further herbicide B.
Also especially preferred are compositions 58.1 to 58.1833, preferably compositions 58.1 to 58.1692, which differ from the corresponding compositions 1.1 to 1.1833, preferably compositions 1.1 to 1.1692, only in that they additionally comprise B.107 as further herbicide B.
Also especially preferred are compositions 59.1 to 59.1833, preferably compositions 59.1 to 59.1692, which differ from the corresponding compositions 1.1 to 1.1833, preferably compositions 1.1 to 1.1692, only in that they additionally comprise B.108 as further herbicide B.
Also especially preferred are compositions 60.1 to 60.1833, preferably compositions 60.1 to 60.1692, which differ from the corresponding compositions 1.1 to 1.1833, preferably compositions 1.1 to 1.1692, only in that they additionally comprise B.110 as further herbicide B.
Also especially preferred are compositions 61.1 to 61.1833, preferably compositions 61.1 to 61.1692, which differ from the corresponding compositions 1.1 to 1.1833, preferably compositions 1.1 to 1.1692, only in that they additionally comprise B.112 as further herbicide B.
Also especially preferred are compositions 62.1 to 62.1833, preferably compositions 62.1 to 62.1692, which differ from the corresponding compositions 1.1 to 1.1833, preferably compositions 11.1 to 1.1692, only in that they additionally comprise B.113 as further herbicide B.
Also especially preferred are compositions 63.1 to 63.1833, preferably compositions 63.1 to 63.1692, which differ from the corresponding compositions 1.1 to 1.1833, preferably compositions 1.1 to 1.1692, only in that they additionally comprise B.117 as further herbicide B.
Also especially preferred are compositions 64.1 to 64.1833, preferably compositions 64.1 to 64.1692, which differ from the corresponding compositions 1.1 to 1.1833, preferably compositions 1.1 to 1.1692, only in that they additionally comprise B.119 as further herbicide B.
Also especially preferred are compositions 65.1 to 65.1833, preferably compositions 65.1 to 65.1692, which differ from the corresponding compositions 1.1 to 1.1833, preferably compositions 1.1 to 1.1692, only in that they additionally comprise B.120 as further herbicide B.
Also especially preferred are compositions 66.1 to 66.1833, preferably compositions 66.1 to 66.1692, which differ from the corresponding compositions 1.1 to 1.1833, preferably compositions 1.1 to 1.1692, only in that they additionally comprise B.122 as further herbicide B.
Also especially preferred are compositions 67.1 to 67.1833, preferably compositions 67.1 to 67.1692, which differ from the corresponding compositions 1.1 to 1.1833, preferably compositions 1.1 to 1.1692, only in that they additionally comprise B.123 as further herbicide B.
Also especially preferred are compositions 68.1 to 68.1833, preferably compositions 68.1 to 68.1692, which differ from the corresponding compositions 1.1 to 1.1833, preferably compositions 1.1 to 1.1692, only in that they additionally comprise B.130 as further herbicide B.
The invention also relates to agrochemical compositions comprising an auxiliary and at least one composition according to the invention.
The crystalline form B of benzoxazinone (I), and the herbicidal compositions comprising the crystalline form B of benzoxazinone (I), can be used, for example, in the form of ready-to-spray aqueous solutions, powders, suspensions, also highly concentrated aqueous, oily or other suspensions or dispersions, emulsions, oil dispersions, pastes, dusts, materials for broadcasting, or granules, by means of spraying, atomizing, dusting, spreading, watering or treatment of the seed or mixing with the seed. The use forms depend on the intended purpose; in any case, they should ensure the finest possible distribution of the active ingredients according to the invention.
The agrochemical compositions comprise an herbicidal effective amount of the crystalline form B of benzoxazinone (I)and at least one further active compound selected from herbicides B and safeners C, and auxiliaries which

are customary for the formulation of crop protection agents.

Examples of auxiliaries customary for the formulation of crop protection agents are inert auxiliaries, solid carriers, surfactants (such as dispersants, protective colloids, emulsifiers, wetting agents and tackifiers), organic and inorganic thickeners, bactericides, antifreeze agents, antifoams, optionally colorants and, for seed formulations, adhesives.

The person skilled in the art is sufficiently familiar with the recipes for such formulations.

Examples of thickeners (i.e. compounds which impart to the formulation modified flow properties, i.e. high viscosity in the state of rest and low viscosity in motion) are polysaccharides, such as xanthan gum (Kelzan® from Kelco), Rhodopol® 23 (Rhone Poulenc) or Veegum® (from R.T. Vanderbilt), and also organic and inorganic sheet minerals, such as Attaclay® (from Engelhard).

Examples of antifoams are silicone emulsions (such as, for example, Silikon® SRE, Wacker or Rhodorsil® from Rhodia), long-chain alcohols, fatty acids, salts of fatty acids, organofluorine compounds and mixtures thereof.

Bactericides can be added for stabilizing the aqueous herbicidal formulations. Examples of bactericides are bactericides based on diclorophen and benzyl alcohol hemiformal (Proxel® from ICI or Acticide® RS from Thor Chemie and Kathon® MK from Rohm & Haas), and also isothiazolinone derivates, such as alkylisothiazolinones and benzisothiazolinones (Acticide MBS from Thor Chemie).

Examples of antifreeze agents are ethylene glycol, propylene glycol, urea or glycerol.

Examples of colorants are both sparingly water-soluble pigments and water-soluble dyes. Examples which may be mentioned are the dyes known under the names Rhodamin B, C.I. Pigment Red 112 and C.I. Solvent Red 1, and also pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

Examples of adhesives are polyvinylpyrrolidone, polyvinyl acetate, polyvinyl alcohol and tylose.

Suitable inert auxiliaries are, for example, the following:

mineral oil fractions of medium to high boiling point, such as kerosene and diesel oil,
furthermore coal tar oils and oils of vegetable or animal origin, aliphatic, cyclic and aromatic hydrocarbons, for example paraffin, tetrahydronaphthalene, alkylated naphthalenes and their derivatives, alkylated benzenes and their derivatives, alcohols such as methanol, ethanol, propanol, butanol and cyclohexanol, ketones such as cyclohexanone or strongly polar solvents,
for example amines such as N-methylpyrrolidone, and water.

Suitable carriers include liquid and solid carriers.
Liquid carriers include e.g. non-aqueous solvents such as cyclic and aromatic hydrocarbons, e.g. paraffins, tetrahydronaphthalene, alkylated naphthalenes and their derivatives, alkylated benzenes and their derivatives, alcohols such as methanol, ethanol, propanol, butanol and cyclohexanol, ketones such as cyclohexanone, strongly polar solvents, e.g. amines such as N-methylpyrrolidone, and water as well as mixtures thereof.

Solid carriers include e.g. mineral earths such as silicas, silica gels, silicates, talc, kaolin, limestone, lime, chalk, bole, loess, clay, dolomite, diatomaceous earth, calcium sulfate, magnesium sulfate and magnesium oxide, ground synthetic materials, fertilizers such as ammonium sulfate, ammonium phosphate, ammonium nitrate and ureas, and products of vegetable origin, such as cereal meal, tree bark meal, wood meal and nutshell meal, cellulose powders, or other solid carriers.

Suitable surfactants (adjuvants, wetting agents, tackifiers, dispersants and also emulsifiers) are the alkali metal salts, alkaline earth metal salts and ammonium salts of aromatic sulfonic acids, for example lignosulfonic acids (e.g. Borrespers-types, Borregaard), phenolsulfonic acids, naphthalenesulfonic acids (Morwet types, Akzo Nobel) and dibutylnaphthalenesulfonic acid (Nekal types, BASF SE), and of fatty acids, alkyl- and alkylarylsulfonates, alkyl sulfates, lauryl ether sulfates and fatty alcohol sulfates, and salts of sulfated hexa-, hepta- and octadecanols, and also of fatty alcohol glycol ethers, condensates of sulfonated naphthalene and its derivatives with formaldehyde, condensates of naphthalene or of the naphthalenesulfonic acids with phenol and formaldehyde, polyoxyethylene octylphenol ether, ethoxylated isooctyl-, octyl- or nonylphenol, alkylphenyl or tributylphenyl polyglycol ether, alkylaryl polyether alcohols, isotridecyl alcohol, fatty alcohol/ethylene oxide condensates, ethoxylated castor oil, polyoxyethylene alkyl ethers or polyoxypropylene alkyl ethers, lauryl alcohol polyglycol ether acetate, sorbitol esters, lignosulfite waste liquors and proteins, denaturated proteins, polysaccharides (e.g. methylcellulose), hydrophobically modified starches, polyvinyl alcohol (Mowiol types Clariant), polycarboxylates (BASF SE, Sokalan types), polyalkoxylates, polyvinylamine (BASF SE, Lupamine types), polyethyleneimine (BASF SE, Lupasol types), polyvinylpyrrolidone and copolymers thereof.

Powders, materials for broadcasting and dusts can be prepared by mixing or concomitant grinding the active ingredients together with a solid carrier.

Granules, for example coated granules, impregnated granules and homogeneous granules, can be prepared by binding the active ingredients to solid carriers.

Aqueous use forms can be prepared from emulsion concentrates, suspensions, pastes, wettable powders or water-dispersible granules by adding water.

To prepare emulsions, pastes or oil dispersions, the crystalline form B of benzoxazinone (I), either as such or dissolved in an oil or solvent, can be homogenized in water by means of a wetting agent, tackifier, dispersant or emulsifier. Alternatively, it is also possible to prepare concentrates comprising active compound, wetting agent, tackifier, dispersant or emulsifier and, if desired, solvent or oil, which are suitable for dilution with water.

The crystalline form B of benzoxazinone (I) and the compostions according to the present invention can, for example, be formulated as follows:

1. Products for dilution with water

A Water-soluble concentrates

10 parts by weight of active compound are dissolved in 90 parts by weight of water or a water-soluble solvent. As an alternative, wetters or other adjuvants are added. The active compound dissolves upon dilution with water. This gives a formulation with an active compound content of 10% by weight.

B Dispersible concentrates

20 parts by weight of active compound are dissolved in 70 parts by weight of cyclohexanone with addition of 10 parts by weight of a dispersant, for example polyvinylpyrrolidone. Dilution with water gives a dispersion. The active compound content is 20% by weight.

C Emulsifiable concentrates

15 parts by weight of active compound are dissolved in 75 parts by weight of an organic solvent (e.g. alkylaromatics) with addition of calcium dodecylbenzenesulfonate and castor oil ethoxylate (in each case 5 parts by weight). Dilution with water gives an emulsion. The formulation has an active compound content of 15% by weight.

D Emulsions

25 parts by weight of active compound are dissolved in 35 parts by weight of an organic solvent (eg. alkylaromatics) with addition of calcium dodecylbenzenesulfonate and castor oil ethoxylate (in each case 5 parts by weight). This mixture is introduced into 30 parts by weight of water by means of an emulsifier (Ultraturrax) and made into a homogeneous emulsion. Dilution with water gives an emulsion. The formulation has an active compound content of 25% by weight.

E Suspensions

In an agitated ball mill, 20 parts by weight of active compound are comminuted with addition of 10 parts by weight of dispersants and wetters and 70 parts by weight of water or an organic solvent to give a fine active compound suspension. Dilution with water gives a stable suspension of the active compound. The active compound content in the formulation is 20% by weight.

F Water-dispersible granules and water-soluble granules

50 parts by weight of active compound are ground finely with addition of 50 parts by weight of dispersants and wetters and made into water-dispersible or water-soluble granules by means of technical appliances (for example extrusion, spray tower, fluidized bed). Dilution with water gives a stable dispersion or solution of the active compound. The formulation has an active compound content of 50% by weight.

G Water-dispersible powders and water-soluble powders

75 parts by weight of active compound are ground in a rotor-stator mill with addition of 25 parts by weight of dispersants, wetters and silica gel. Dilution with water gives a stable dispersion or solution of the active compound. The active compound content of the formulation is 75% by weight.

H Gel formulations

In a ball mill, 20 parts by weight of active compound, 10 parts by weight of dispersant, 1 part by weight of gelling agent and 70 parts by weight of water or of an organic solvent are mixed to give a fine suspension. Dilution with water gives a stable suspension with active compound content of 20% by weight.

2. Products to be applied undiluted

I Dusts

5 parts by weight of active compound are ground finely and mixed intimately with 95 parts by weight of finely divided kaolin. This gives a dusting powder with an active compound content of 5% by weight.

J Granules (GR, FG, GG, MG)

0.5 parts by weight of active compound are ground finely and associated with 99.5 parts by weight of carriers. Current methods here are extrusion, spray-drying or the fluidized bed. This gives granules to be applied undiluted with an active compound content of 0.5% by weight.

K ULV solutions (UL)

10 parts by weight of active compound are dissolved in 90 parts by weight of an organic solvent, for example xylene. This gives a product to be applied undiluted with an active compound content of 10% by weight.

Aqueous use forms can be prepared from emulsion concentrates, suspensions, pastes, wettable powders or water-dispersible granules by adding water.

The concentrations of the active compounds, especially of the crystalline form B of benzoxazinone (I) in the ready-to-use preparations (formulations) can be varied within wide ranges. In general, the formulations comprise approximately from 0.001 to 98% by weight, preferably 0.01 to 95% by weight of at least one active ingredient. The active ingredients are employed in a purity of from 90% to 100%, preferably 95% to 100% (according to NMR spectrum).

In the formulation of the crystalline form B of benzoxazinone (I) according to the present invention the active ingredients, e.g. the the crystalline form B of benzoxazinone (I), are present in suspended, emulsified or dissolved form. The formulation according to the invention can be in the form of aqueous solutions, powders, suspensions, also highly-concentrated aqueous, oily or other suspensions or dispersions, aqueous emulsions, aqueous microemulsions, aqueous suspo-emulsions, oil dispersions, pastes, dusts, materials for spreading or granules.

According to one embodiment of the invention, in the ready-to-use preparations of herbicidal compositions, i.e. in the compositions according to the invention in the form of crop protection compositions, the crystalline form B of benzoxazinone (I) and herbicides B and/or safeners C can be present formulated jointly or separately in suspended, emulsified or dissolved form. The use forms depend entirely on the intended applications.

Accordingly, a first embodiment of the invention relates to compositions in the form of a crop protection composition formulated as a 1-component composition comprising the crystalline form B of benzoxazinone (I) and at least one further active compound selected from the herbicides B and the safeners C and also a solid or liquid carrier and, if appropriate, one or more surfactants.

Accordingly, a second embodiment of the invention relates to compositions in the form of a crop protection composition formulated as a 2-component composition comprising a first formulation (component) comprising the crystalline form B of benzoxazinone (I), a solid or liquid carrier and, if appropriate, one or more surfactants, and a second component comprising at least one further active compound selected from the herbicides B and safeners C, a solid or liquid carrier and, if appropriate, one or more surfactants.

The crystalline form B of benzoxazinone (I) and the at least one further herbicide B and/or safener C can be applied jointly or separately, simultaneously or in succession, before, during or after the emergence of the plants. The order of the application of the crystalline form B of benzoxazinone (I), herbicide B and/or safener C is of minor importance. The only thing that is important is that the crystalline form B of benzoxazinone (I) and the at least one further herbicide B and/or safener C are present simultaneously at the site of action, i.e. are at the same time in contact with or taken up by the plant to be controlled.

The crystalline form B of benzoxazinone (I) and the compositions according to the invention are suitable as herbicides. They are suitable as such or as an appropriately formulated composition (agrochemical composition) comprising as herbicide the crystalline form B of benzoxazinone (I), and herbicides B and/or safeners C.
The compositions according to the invention control vegetation on non-crop areas very efficiently, especially at high rates of application. They act against broad-leaved weeds and grass weeds in crops such as wheat, rice, maize, soya

and cotton without causing any significant damage to the crop plants. This effect is mainly observed at low rates of application.

The crystalline form B of benzoxazinone (I) and the compositions according to the invention comprising them are applied to the plants mainly by spraying the leaves. Here, the application can be carried out using, for example, water as carrier by customary spraying techniques using spray liquor amounts of from about 100 to 1000 l/ha (for example from 300 to 400 l/ha). The herbicidal compositions may also be applied by the low-volume or the ultra-low-volume method, or in the form of microgranules.

The crystalline form B of benzoxazinone (I) and the compositions according to the present invention can be applied pre-, post-emergence or pre-plant, or together with the seed of a crop plant.

It is also possible to apply the compounds and compositions according to the invention by applying seed, pretreated with the herbicidal compositions or active compounds, of a crop plant. If the crystalline form B of benzoxazinone (I), herbicides B, and/or safeners C are less well tolerated by certain crop plants, application techniques may be used in which the herbicidal compositions are sprayed, with the aid of the spraying equipment, in such a way that as far as possible they do not come into contact with the leaves of the sensitive crop plants, while the active ingredients reach the leaves of undesirable plants growing underneath, or the bare soil surface (post-directed, lay-by).

In a further embodiment, the crystalline form B of benzoxazinone (I) or the herbicidal compositions comprising them can be applied by treating seed. The treatment of seeds comprises essentially all procedures familiar to the person skilled in the art (seed dressing, seed coating, seed dusting, seed soaking, seed film coating, seed multilayer coating, seed encrusting, seed dripping and seed pelleting) based on the crystalline form B of benzoxazinone (I) according to the invention or the compositions prepared therefrom. Here, the herbicidal compositions can be applied diluted or undiluted.

The term "seed" comprises seed of all types, such as, for example, corns, seeds, fruits, tubers, seedlings and similar forms. Here, preferably, the term seed describes corns and seeds. The seed used can be seed of the useful plants mentioned above, but also the seed of transgenic plants or plants obtained by customary breeding methods.

The required application rate of pure active compound composition, i.e. crystalline form B of benzoxazinone (I) and herbicide B, and, if appropriate, safener C without formulation auxiliaries depends on the composition of the plant stand, on the development stage of the plants, on the climatic conditions at the site of use and on the application technique.

The rates of application of the crystalline form B of benzoxazinone (I) according to the present invention are from 0.1 g/ha to 3000 g/ha, preferably 10 g/ha to 1000 g/ha of active substance (a.s.), depending on the control target, the season, the target plants and the growth stage.

In another embodiment of the invention, the application rates of the crystalline form B of benzoxazinone (I) are in the range from 0.1 g/ha to 5000 g/ha and preferably in the range from 1 g/ha to 2500 g/ha or from 5 g/ha to 2000 g/ha of active substance (a.s.).

In another embodiment of the invention, the application rate of the crystalline form B of benzoxazinone (I) is 0.1 to 1000 g/ha, preferably 1 to 750 g/ha, more preferably 5 to 500 g/ha, of active substance.

In another embodiment of the invention the required application rate of the crystalline form B of benzoxazinone (I), is generally in the range of from 0.0005 kg/ha to 2.5 kg/ha and preferably in the range of from 0.005 kg/ha to 2 kg/ha or 0.01 kg/ha to 1.5 kg/h of a.s.

To treat the seed, the crystalline form B of benzoxazinone (I) is generally employed in amounts of from 0.001 to 10 kg per 100 kg of seed.

In general, the application rate of the crystalline form B of benzoxazinone (I) and herbicides B and, if appropriate, safeners C, is from 0.001 to 3 kg/ha, preferably from 0.005 to 2.5 kg/ha and in particular from 0.01 to 2 kg/ha of active substance (a.s.).

The required application rates of herbicides B are generally in the range of from 0.0005 kg/ha to 2.5 kg/ha and preferably in the range of from 0.005 kg/ha to 2 kg/ha or 0.01 kg/ha to 1.5 kg/h of a.s.

The required application rates of safeners C are generally in the range of from 0.0005 kg/ha to 2.5 kg/ha and preferably in the range of from 0.005 kg/ha to 2 kg/ha or 0.01 kg/ha to 1.5 kg/h of a.s.

Depending on the application method in question, the compositions according to the invention can additionally be employed in a further number of crop plants for eliminating undesirable plants. Examples of suitable crops are the following:

Allium cepa, Ananas comosus, Arachis hypogaea, Asparagus officinalis, Avena sativa, Beta vulgaris spec. altissima, Beta vulgaris spec. rapa, Brassica napus var. napus, Brassica napus var. napobrassica, Brassica rapa var. silvestris, Brassica oleracea, Brassica nigra, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot

esculenta, Medicago sativa, Musa spec., Nicotiana tabacum (N. rustica), Olea europaea, Oryza sativa, Phaseolus lunatus, Phaseolus vulgaris, Picea abies, Pinus spec., Pistacia vera, Pisum sativum, Prunus avium, Prunus persica, Pyrus communis, Prunus armeniaca, Prunus cerasus, Prunus dulcis and Prunus domestica, Ribes sylvestre, Ricinus communis, Saccharum officinarum, Secale cereale, Sinapis alba, Solanum tuberosum, Sorghum bicolor (s. vulgare), Theobroma cacao, Trifolium pratense, Triticum aestivum, Triticale, Triticum durum, Vicia faba, Vitis vinifera and Zea mays.

Preferred crops are the following: Arachis hypogaea, Beta vulgaris spec. altissima, Brassica napus var. napus, Brassica oleracea, Citrus limon, Citrus sinensis, Coffea arabica (Coffea canephora, Coffea liberica), Cynodon dactylon, Glycine max, Gossypium hirsutum, (Gossypium arboreum, Gossypium herbaceum, Gossypium vitifolium), Helianthus annuus, Hordeum vulgare, Juglans regia, Lens culinaris, Linum usitatissimum, Lycopersicon lycopersicum, Malus spec., Medicago sativa, Nicotiana tabacum (N.rustica), Olea europaea, Oryza sativa , Phaseolus lunatus, Phaseolus vulgaris, Pistacia vera, Pisum sativum, Prunus dulcis, Saccharum officinarum, Secale cereale, Solanum tuberosum, Sorghum bicolor (s. vulgare), Triticale, Triticum aestivum, Triticum durum, Vicia faba, Vitis vinifera and Zea mays.

Especially preferred crops of cereals, corn, soybeans, rice, oilseed rape, cotton, potatoes, peanuts or permanent crops, and also in crops which are resistant to one or more herbicides or to attack by insects owing to genetic engineering or breeding.

The compositions according to the invention can also be used in genetically modified plants. The term "genetically modified plants" is to be understood as plants, which genetic material has been modified by the use of recombinant DNA techniques in a way that under natural circumstances it cannot readily be obtained by cross breeding, mutations or natural recombination. Typically, one or more genes have been integrated into the genetic material of a genetically modified plant in order to improve certain properties of the plant. Such genetic modifications also include but are not limited to targeted post-transitional modification of protein(s), oligo- or polypeptides e. g. by glycosylation or polymer additions such as prenylated, acetylated or farnesylated moieties or PEG moieties. Plants that have been modified by breeding, mutagenesis or genetic engineering, e.g. have been rendered tolerant to applications of specific classes of herbicides, such as hydroxyphenylpyruvate dioxygenase (HPPD) inhibitors; acetolactate synthase (ALS) inhibitors, such as sulfonyl ureas (see e.g. US 6,222,100, WO 01/82685, WO 00/26390, WO 97/41218, WO 98/02526, WO 98/02527, WO 04/106529, WO 05/20673, WO 03/14357, WO 03/13225, WO 03/14356, WO 04/16073) or imidazolinones (see e. g. US 6,222,100, WO 01/82685, WO 00/026390, WO 97/41218, WO 98/002526, WO 98/02527, WO 04/106529, WO 05/20673, WO 03/014357, WO 03/13225, WO 03/14356, WO 04/16073); enolpyruvylshikimate-3-phosphate synthase (EPSPS) inhibitors, such as glyphosate (see e.g. WO 92/00377); glutamine synthetase (GS) inhibitors, such as glufosinate (see e.g. EP-A 242 236, EP-A 242 246) or oxynil herbicides (see e. g. US 5,559,024) as a result of conventional methods of breeding or genetic engineering. Several cultivated plants have been rendered tolerant to herbicides by conventional methods of breeding (mutagenesis), e. g. Clearfield® summer rape (Canola, BASF SE, Germany) being tolerant to imidazolinones, e. g. imazamox. Genetic engineering methods have been used to render cultivated plants such as soybean, cotton, corn, beets and rape, tolerant to herbicides such as glypho-sate and glufosinate, some of which are commercially available under the trade names RoundupReady® (glyphosate-tolerant, Monsanto, U.S.A.) and LibertyLink® (glufosinate-tolerant, Bayer CropScience, Germany).

Furthermore, plants are also covered that are by the use of recombinant DNA techniques capable to synthesize one or more insecticidal proteins, especially those known from the bacterial genus Bacillus, particularly from Bacillus thuringiensis, such as ä-endotoxins, e. g. Cry-IA(b), CryIA(c), CryIF, CryIF(a2), CryIIA(b), CryIIIA, CryIIIB(b1) or Cry9c; vegetative insecticidal proteins (VIP), e. g. VIP1, VIP2, VIP3 or VIP3A; insecticidal proteins of bacteria colonizing nematodes, e. g. Photorhabdus spp. or Xenorhabdus spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; aggluti-nins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxy-steroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilbene synthase, bibenzyl synthase, chitinases or glucanases. In the context of the present invention these insecticidal proteins or toxins are to be under-stood expressly also as pre-toxins, hybrid proteins, truncated or otherwise modified proteins. Hybrid proteins are characterized by a new combination of protein domains, (see, e. g. WO 02/015701). Further examples of such toxins or genetically modified plants capable of synthesizing such toxins are dis-closed, e. g., in EP-A 374 753, WO 93/007278, WO 95/34656, EP-A 427 529, EP-A 451 878, WO 03/18810 und WO 03/52073. The methods for producing such genetically modified plants are generally known to the person skilled in the art and are described, e. g. in the publications mentioned above. These insecticidal proteins contained in the genetically modified plants impart to the plants producing these proteins tolerance to harmful pests from all taxonomic groups of arthropods, especially to beetles (Coeloptera), two-winged insects (Diptera), and moths (Lepidoptera) and to nematodes (Nematoda). Genetically

modified plants capable to synthesize one or more insecticidal pro-teins are, e. g., described in the publications mentioned above, and some of which are commercially available such as YieldGard® (corn cultivars producing the Cry1Ab toxin), YieldGard® Plus (corn cultivars producing Cry1Ab and Cry3Bb1 toxins), Starlink® (corn cultivars producing the Cry9c toxin), Herculex® RW (corn cultivars producing Cry34Ab1, Cry35Ab1 and the enzyme Phosphinothricin-N-Acetyltransferase [PAT]); NuCOTN® 33B (cotton cultivars producing the Cry1Ac toxin), Bollgard® I (cotton culti-vars producing the Cry1Ac toxin), Bollgard® II (cotton cultivars producing Cry1Ac and Cry2Ab2 toxins); VIPCOT® (cotton cultivars producing a VIP-toxin); NewLeaf® (potato cultivars producing the Cry3A toxin); Bt-Xtra®, NatureGard®, KnockOut®, BiteGard®, Protecta®, Bt11 (e. g. Agrisure® CB) and Bt176 from Syngenta Seeds SAS, France, (corn cultivars producing the Cry1Ab toxin and PAT enzyme), MIR604 from Syngenta Seeds SAS, France (corn cultivars producing a modified version of the Cry3A toxin, c.f. WO 03/018810), MON 863 from Monsanto Europe S.A., Belgium (corn cultivars produ-cing the Cry3Bb1 toxin), IPC 531 from Monsanto Europe S.A., Belgium (cotton cultivars producing a modified version of the Cry1Ac toxin) and 1507 from Pioneer Overseas Corporation, Belgium (corn cultivars producing the Cry1 F toxin and PAT enzyme).

Furthermore, plants are also covered that are by the use of recombinant DNA techniques capable to synthesize one or more proteins to in-crease the resistance or tolerance of those plants to bacterial, viral or fungal pathogens. Examples of such proteins are the so-called "pathogenesis-related proteins" (PR proteins, see, e.g. EP-A 392 225), plant disease resistance genes (e. g. potato culti-vars, which express resistance genes acting against Phytophthora infestans derived from the Mexican wild potato Solanum bulbocastanum) or T4-lyso-zym (e.g. potato cultivars capable of synthesizing these proteins with increased resistance against bacteria such as Erwinia amylvora). The methods for producing such genetically modi-fied plants are generally known to the person skilled in the art and are described, e.g. in the publications mentioned above.

Furthermore, plants are also covered that are by the use of recombinant DNA techniques capable to synthesize one or more proteins to increase the productivity (e.g. bio mass production, grain yield, starch content, oil content or protein content), tolerance to drought, salinity or other growth-limiting environ-mental factors or tolerance to pests and fungal, bacterial or viral pathogens of those plants.

Furthermore, plants are also covered that contain by the use of recombinant DNA techniques a modified amount of substances of content or new substances of content, specifically to improve human or animal nutrition, e. g. oil crops that produce health-promoting long-chain omega-3 fatty acids or unsaturated omega-9 fatty acids (e. g. Nexera® rape, DOW Agro Sciences, Canada).

Furthermore, plants are also covered that contain by the use of recombinant DNA techniques a modified amount of substances of content or new substances of content, specifically to improve raw material production, e.g. potatoes that produce increased amounts of amylopectin (e.g. Amflora® potato, BASF SE, Germany).

The compositions according to the invention can also be used in crop plants which are resistant to one or more herbicides owing to genetic engineering or breeding, which are resistant to one or more pathogens such as plant pathogenous fungi owing to genetic engineering or breeding, or which are resistant to attack by insects owing to genetic engineering or breeding.

Suitable are for example crop plants, preferably corn, wheat, sunflower, sugarcane, cotton, rice, canola, oilseed rape or soybeans, which crops are resistant to herbicidal PPO inhibitors, or crop plants which, owing to introduction of the gene for Bt toxin by genetic modification, are resistant to attack by certain insects.

Furthermore, it has been found that the compositions according to the invention are also suitable for the defoliation and/or desiccation of plant parts, for which crop plants such as cotton, potato, oilseed rape, sunflower, soybean or field beans, in particular cotton, are suitable. In this regard, there have been found compositions for the desiccation and/or defoliation of plants, processes for preparing these compositions and methods for desiccating and/or defoliating plants using the crystalline form B of benzoxazinone (I).

As desiccants, the crystalline form B of benzoxazinone (I) are particularly suitable for desiccating the above-ground parts of crop plants such as potato, oilseed rape, sunflower and soybean, but also cereals. This makes possible the fully mechanical harvesting of these important crop plants.

Also of economic interest is to facilitate harvesting, which is made possible by concentrating within a certain period of time the dehiscence, or reduction of adhesion to the tree, in citrus fruit, olives and other species and varieties of pernicious fruit, stone fruit and nuts. The same mechanism, i.e. the promotion of the development of abscission tissue between fruit part or leaf part and shoot part of the plants is also essential for the controlled defoliation of useful plants, in particular cotton.

Moreover, a shortening of the time interval in which the individual cotton plants mature leads to an increased fiber quality after harvesting.

For the defoliation and/or desiccation of plant parts, it is of no importance whether the crystalline form B of benzoxazinone (I) and herbicides B and, if appropriate, safeners C are formulated and applied jointly or separately, and in which order application is carried out in case of separate application.

Examples

The following illustrations and examples serve to illustrate the invention and should not be regarded as limiting.

Figure 1 shows an X-ray powder diffraction diagram of form B. The X-ray diffraction diagram of form B was recorded by using Panalytical X'Pert Pro diffractometer (manufacturer: Panalytical) in reflection geometry in the range from 2θ =3°-35° with increments of 0,0167°C using Cu-Kα radiation ( at 25°C). The recorded 2θ values were used to calculate the stated interplanar spacings d. The intensity of the peaks (y-axis: linear intensity counts) is plotted versus the 2θ angle (x-axis in degrees 2θ).

Figure 2 shows an X-ray powder diffraction diagram of form A. The X-ray diffraction diagram was recorded under the conditions stated for Figure 1.

Figure 3 shows an X-ray powder diffraction diagram of a mixture of forms A + B + C. The X-ray diffraction diagram was recorded under the conditions stated for Figure 1.

The single crystal X-ray diffraction data of Form A was collected on a Bruker AXS CCD Detector using graphite Cu-Kα radiation (at -173°C). The structure was solved using direct methods, refined and expanded by using Fourier techniques with SHELX software package (G. M. Shel-drick, SHELX-97, University of Göttingen, 1997). Absorption correction was performed with SA-DABS software.

DSC was performed on a Mettler Toledo DSC 822e module. Tha samples were placed in crimped but vented aluminium pans. The samples size in each case was 5 to 10 mg. The thermal behaviour was analized in the range 30 - 250 °C. The heating rate was 5°C/min. The samples were purged with a stream of nitrogen flowing at 150 ml/ during the experiment. Melting points values were confirmed by a Mettler Hot Stage in combination with a light microscope.

<u>Preparation of form B of benzoxazinone (I) by crystallization from a slurry in a mixture of water and organic solvent</u>

Example 1:

Form A of benzoxazinone (I), obtained by example 16 (500 mg) were suspended in 3 ml of a mixture of water and ethanol (1:1 v/v) and the slurry was stirred for 48 h at 90°C. A slurry of crystalline material was obtained, which was filtered and analysed by XRPD and DSC. The obtained material was pure form B of benzoxazinone (I).

Example 2:

A mixture forms A and B of benzoxazinone (I), obtained by comparative example 1 (500 mg) were suspended in 3 ml of a mixture of water and 1,3-propanediol (1:1 v/v) and the slurry was stirred for 48 h at 90°C. A slurry of crystalline material was obtained, which was filtered and analysed by PXRD and DSC. The obtained material was pure form B of benzoxazinone (I).

<u>Preparation of form B of benzoxazinone (I) by crystallization from a solution in an organic solvent with evaporation</u>

Example 3:

50 mg of benzoxazinone (I) were dissolved in 2-3 ml of toluene in a test vessel. The test vessel placed in a greenhouse and heated to 95°C and a nitrogen flow (5 l/min) was passed over the surface of the solvent. In this manner, benzoxazinone (I) was obtained in the form of small crystalline plates, which were isolated and analyzed by X-ray powder diffraction (XRPD). On the basis of the characteristic reflections, form B was identified.

<u>Preparation of form B of benzoxazinone (I) by heating form A</u>

Example 4:

500 mg of form A of benzoxazinone (I), obtained by example 16 were placed into an open vessel. The vessel was purged with nitrogen and sealed and than heated to 180°C for 2 h. The obtained material was isolated and analyzed by X-ray powder diffraction (XRPD). On the basis of the characteristic reflections, form B was identified.

Preparation of form A of benzoxazinone (I) by crystallization from a solution in an organic solvent with evaporation

Examples 5 to 14 (not according to the invention):

50 mg of benzoxazinone (I) were dissolved in 2-3 ml of the respective solvent in a test vessel. The test vessel was placed in a greenhouse and a nitrogen flow (5 l/min) was passed over the surface of the solvent. In this manner, benzoxazinone (I) was obtained in the form of small crystalline rods, which were isolated and analyzed by X-ray powder diffraction (XRPD). On the basis of the characteristic reflections, form A was identified.

Table 1:

| Example | Solvent | Form | Crystal form |
|---|---|---|---|
| 5 | ethylbenzene | A | small rods |
| 6 | dichlorobenzene | A | small rods |
| 7 | chlorobenze | A | small rods |
| 8 | p-xylene | A | small rods |
| 9 | acetone | A | small rods |
| 10 | methylethylketone | A | small rods |
| 11 | methylbutylketone | A | small rods |
| 12 | methanol | A | small rods |
| 13 | ethanol | A | small rods |
| 14 | ispropanol | A | small rods |

Preparation of form A of benzoxazinone (I) by crystallization from a slurry in a mixture of water and organic solvent

Example 15 (not according to the invention):

A mixture of forms A and B of benzoxazinone (I), obtained by comparative example 1 (500 mg) were suspended in 3 ml of a mixture of water and Ethanol (1:1 v/v) and the slurry was stirred for 48 h at 23°C. A slurry of crystalline material was obtained, which was filtered and analysed by XRPD and DSC. The obtained material was pure form A of benzoxazinone (I).

Example 16 (not according to the invention):

Form B of benzoxazinone (I), obtained by example 16 (500 mg) were suspended in 3 ml of a mixture of water and tetrahydrofurane (1:1 v/v) and the slurry was stirred for 48 h at 23°C. A slurry of crystalline material was obtained, which was filtered and analysed by XRPD and DSC. The obtained material was pure form A of benzoxazinone (I).

Example 17 (not according to the invention):

A mixture forms A and B of benzoxazinone (I), obtained by comparative example 1 (500 mg) were suspended in 3 ml of a mixture of toluene and the slurry was stirred for 48 h at 23°C. A slurry of crystalline material was obtained, which was filtered and analysed by XRPD and DSC. The obtained material was pure form A of benzoxazinone (I).

Example 18 (not according to the invention):

A mixture forms A and B of benzoxazinone (I), obtained by comparative example 1 (500 mg) were suspended in 3 ml of a mixture of water and 1,3-propanediol (1:1 v/v) and the slurry was stirred for 48 h at 23°C. A slurry of crystalline material was obtained, which was filtered and analysed by XRPD and DSC. The obtained material was pure form A of benzoxazinone (I).

Preparation of a mixture of forms A and B of benzoxazinone (I)

Comparative Example 1

50 mg of benzoxazinone (I) were dissolved in 2-3 ml of the respective solvent (e.g. 1-butanol, isobutanol) in a test vessel. The test vessel was placed in a greenhouse and heated to 90 °C. A nitrogen flow (5 l/min) was passed over the surface of the solvent. In this manner, benzoxazinone (I) was obtained in the form of small crystalline rods, which were isolated and analyzed by X-ray powder diffraction (XRPD). On the basis of the characteristic reflections, a mixture of forms A and B was identified.

Use examples

The herbicidal activity of the crystalline form B of benzoxazinone (I) and the compositions according to the invention was demonstrated by the following greenhouse experiments:

The culture containers used were plastic flowerpots containing loamy sand with approximately 3.0% of humus as the substrate. The seeds of the test plants were sown separately for each species.

For the pre-emergence treatment, the active ingredients, which had been suspended or emulsified in water, were applied directly after sowing by means of finely distributing nozzles. The containers were irrigated gently to promote germination and growth and subsequently covered with transparent plastic hoods until the plants had rooted. This cover caused uniform germination of the test plants, unless this has been impaired by the active ingredients.

For the post-emergence treatment, the test plants were first grown to a height of 3 to 15 cm, depending on the plant habit, and only then treated with the active ingredients which had been suspended or emulsified in water. For this purpose, the test plants were either sown directly and grown in the same containers, or they were first grown separately as seedlings and transplanted into the test containers a few days prior to treatment.

Depending on the species, the plants were kept at 10 - 25°C or 20 - 35°C. The test period extended over 2 to 4 weeks. During this time, the plants were tended, and their response to the individual treatments was evaluated.

Evaluation was carried out using a scale from 0 to 100. 100 means no emergence of the plants, or complete destruction of at least the aerial moieties, and 0 means no damage, or normal course of growth. A good herbicidal activity is given at values of at least 70 and a very good herbicidal activity is given at values of at least 85.

In the examples, using the method of S. R. Colby (1967) "Calculating synergistic and antagonistic responses of herbicide combinations", Weeds 15, p. 22ff., the value E, which is expected if the activity of the individual active compounds is only additive, was calculated.

$$E = X + Y - (X \cdot Y/100)$$

where
X = percent activity using active compound A at an application rate a;
Y = percent activity using active compound B at an application rate b;
E = expected activity (in %) by A + B at application rates a + b.

If the value found experimentally is higher than the value E calculated according to Colby, a synergistic effect is present.

The results of these tests are given in the use examples and demonstrate the synergistic effect of the mixtures comprising the crystalline form B of benzoxazinone (I) and at least one herbicide B. In this context, a.s. means active substance, based on 100 % active ingredient.

## Claims

**1.** Herbicidal compositions comprising

A) the crystalline form B of 1,5-dimethyl-6-thioxo-3-(2,2,7-trifluoro-3-oxo-4-(prop-2-ynyl)-3,4-dihydro-2H-benzo[b][1,4]oxazin-6-yl)-1,3,5-triazinane-2,4-dione, which in an X-ray powder diffraction diagram at 25°C and Cu-K$\alpha$ radiation displays at least 3 of the following reflections, quoted as 2$\theta$ values: 9.0 $\pm$ 0.2°, 10.9 $\pm$ 0.2°, 11.5 $\pm$ 0.2°, 12.9 $\pm$ 0.2°, 13.5 $\pm$ 0.2°, 14.9 $\pm$ 0.2°, 16.4 $\pm$ 0.2°, 16.5 $\pm$ 0.2°, 17.5 $\pm$ 0.2° and 20.3 $\pm$ 0.2°.;

and at least one further active compound selected from
B) herbicides of class b1) to b15):

b1) lipid biosynthesis inhibitors;
b2) acetolactate synthase inhibitors (ALS inhibitors);
b3) photosynthesis inhibitors;
b4) protoporphyrinogen-IX oxidase inhibitors,
b5) bleacher herbicides;
b6) enolpyruvyl shikimate 3-phosphate synthase inhibitors (EPSP inhibitors);
b7) glutamine synthetase inhibitors;
b8) 7,8-dihydropteroate synthase inhibitors (DHP inhibitors);
b9) mitose inhibitors;
b10) inhibitors of the synthesis of very long chain fatty acids (VLCFA inhibitors);
b11) cellulose biosynthesis inhibitors;
b12) decoupler herbicides;
b13) auxin herbicides;
b14) auxin transport inhibitors; and
b15) other herbicides selected from the group consisting of bromobutide, chlorflurenol, chlorflurenol-methyl, cinmethylin, cumyluron, dalapon, dazomet, difenzoquat, difenzoquat-metilsulfate, dimethipin, DSMA, dymron, endothal and its salts, etobenzanid, flamprop, flamprop-isopropyl, flamprop-methyl, flamprop-M-isopropyl, flamprop-M-methyl, flurenol, flurenol-butyl, flurprimidol, fosamine, fosamine-ammonium, indanofan, indaziflam, maleic hydrazide, mefluidide, metam, methyl azide, methyl bromide, methyl-dymron, methyl iodide, MSMA, oleic acid, oxaziclomefone, pelargonic acid, pyributicarb, quinoclamine, triaziflam, tridiphane and 6-chloro-3-(2-cyclopropyl-6-methylphenoxy)-4-pyridazinol (CAS 499223-49-3) and its salts and esters;

and

C) safeners;

including the agriculturally acceptable salts or derivatives of herbicides B and safeners C having a carboxyl group.

**2.** A composition as claimed in claim 1, wherein the 1,5-dimethyl-6-thioxo-3-(2,2,7-trifluoro-3-oxo-4-(prop-2-ynyl)-3,4-dihydro-2H-benzo[b][1,4]oxazin-6-yl)-1,3,5-triazinane-2,4-dione has a content of at least 94 wt.% of the crystalline form B.

**3.** A composition according to claims 1 or 2, wherein at last one herbicide B is selected from the group consisting of:

b1) from the group of the lipid biosynthesis inhibitors:

clethodim, clodinafop-propargyl, cycloxydim, cyhalofop-butyl, diclofop-methyl, fenoxaprop-P-ethyl, fluazifop-P-butyl, haloxyfop-P-methyl, metamifop, pinoxaden, profoxydim, propaquizafop, quizalofop-P-ethyl, quizalofop-P-tefuryl, sethoxydim, tepraloxydim, tralkoxydim, benfuresate, dimepiperate, EPTC, esprocarb, ethofumesate, molinate, orbencarb, prosulfocarb, thiobencarb and triallate;

b2) from the group of the ALS inhibitors:

amidosulfuron, azimsulfuron, bensulfuron-methyl, bispyribac-sodium, chlorimuron-ethyl, chlorsulfuron, cloransulam-methyl, cyclosulfamuron, diclosulam, ethametsulfuron-methyl, ethoxysulfuron, flazasulfuron, florasulam, flucarbazone-sodium, flucetosulfuron, flumetsulam, flupyrsulfuron-methyl-sodium, foramsulfuron, halosulfuron-methyl, imazamethabenz-methyl, imazamox, imazapic, imazapyr, imazaquin, imazethapyr, imazosulfuron, iodosulfuron, iodosulfuron-methyl-sodium, mesosulfuron, metazosulfuron, metosulam, metsulfuron-methyl, nicosulfuron, orthosulfamuron, oxasulfuron, penoxsulam, primisulfuron-methyl, propoxycarbazon-sodium, propyrisulfuron, prosulfuron, pyrazosulfuron-ethyl, pyribenzoxim, pyrimisulfan, pyriftalid, pyriminobac-methyl, pyrithiobac-sodium, pyroxsulam, rimsulfuron, sulfometuron-methyl, sulfosulfuron, thiencarbazone-methyl, thifensulfuron-methyl, triasulfuron, tribenuron-methyl, trifloxysulfuron, triflusulfuron-methyl and tritosulfuron;

b3) from the group of the photosynthesis inhibitors:

ametryn, amicarbazone, atrazine, bentazone, bentazone-sodium, bromoxynil and its salts and esters, chloridazone, chlorotoluron, cyanazine, desmedipham, diquat-dibromide, diuron, fluometuron, hexazinone, ioxynil and its salts and esters, isoproturon, lenacil, linuron, metamitron, methabenzthiazuron, metribuzin, paraquat, paraquat-dichloride, phenmedipham, propanil, pyridate, simazine, terbutryn, terbuthylazine and thidiazuron;

b4) from the group of the protoporphyrinogen-IX oxidase inhibitors:

acifluorfen-sodium, bencarbazone, benzfendizone, butafenacil, carfentrazone-ethyl, cinidon-ethyl, flufenpyr-ethyl, flumiclorac-pentyl, flumioxazin, fluoroglycofen-ethyl, fomesafen, lactofen, oxadiargyl, oxadiazon, oxyfluorfen, pentoxazone, pyraflufen-ethyl, saflufenacil, sulfentrazone, ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100), N-ethyl-3-(2,6-dichloro-4-trifluoromethylphenoxy)-5-methyl-1*H*-pyrazole-1-carboxamide (CAS 452098-92-9), N-tetrahydrofurfuryl-3-(2,6-dichloro-4-trifluoromethylphenoxy)-5-methyl-1*H*-pyrazole-1-carboxamide (CAS 915396-43-9), N-ethyl-3-(2-chloro-6-fluoro-4-trifluoromethylphenoxy)-5-methyl-1*H*-pyrazole-1-carboxamide (CAS 452099-05-7), N-tetrahydrofurfuryl-3-(2-chloro-6-fluoro-4-trifluoromethylphenoxy)-5-methyl-1*H*-pyrazole-1-carboxamide (CAS 45100-03-7) and 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione;

b5) from the group of the bleacher herbicides:

aclonifen, beflubutamid, benzobicyclon, clomazone, diflufenican, flurochloridone, flurtamone, isoxaflutole, mesotrione, norflurazon, picolinafen, pyrasulfotole, pyrazolynate, sulcotrione, tefuryltrione, tembotrione, topramezone, bicyclopyrone, 4-(3-trifluoromethyl-phenoxy)-2-(4-trifluoromethylphenyl)pyrimidine (CAS 180608-33-7), amitrole and flumeturon;

b6) from the group of the EPSP synthase inhibitors:

glyphosate, glyphosate-isopropylammonium and glyphosate-trimesium (sulfosate);

b7) from the group of the glutamine synthase inhibitors:

glufosinate, glufosinate-P, glufosinate-ammonium;

b8) from the group of the DHP synthase inhibitors: asulam;

b9) from the group of the mitose inhibitors:

benfluralin, dithiopyr, ethalfluralin, oryzalin, pendimethalin, thiazopyr and trifluralin;

b10) from the group of the VLCFA inhibitors:

acetochlor, alachlor, anilofos, butachlor, cafenstrole, dimethenamid, dimethenamid-P, fentrazamide, flufenacet, mefenacet, metazachlor, metolachlor, S-metolachlor, naproanilide, napropamide, pretilachlor, fenoxasulfone, ipfencarbazone, pyroxasulfone thenylchlor and isoxazoline-compounds of the formulae II.1, II.2, II.3, II.4, II.5, II.6, II.7, II.8 and II.9 as mentioned above;

b11) from the group of the cellulose biosynthesis inhibitors: dichlobenil, flupoxam, isoxaben, 1-Cyclohexyl-5-pentafluorphenyloxy-$1^{4}$-[1,2,4,6]thiatriazin-3-ylamine and the piperazine compounds of formula III as mentioned above;

b13) from the group of the auxin herbicides:

2,4-D and its salts and esters, aminopyralid and its salts such as aminopyralid-tris(2-hydroxypropyl)ammonium and its esters, clopyralid and its salts and esters, dicamba and its salts and esters, dichlorprop-P and its salts and esters, fluroxypyr-meptyl, MCPA and its salts and esters, MCPB and its salts and esters, mecoprop-P and its salts and esters, picloram and its salts and esters, quinclorac, quinmerac, triclopyr and its salts and esters, and aminocyclopyrachlor and its salts and esters;

b14) from the group of the auxin transport inhibitors: diflufenzopyr and diflufenzopyrsodium;

b15) from the group of the other herbicides: bromobutide, cinmethylin, cumyluron, dalapon, difenzoquat, difenzoquat-metilsulfate, DSMA, dymron (= daimuron), flamprop, flamprop-isopropyl, flamprop-methyl, flamprop-M-isopropyl, flamprop-M-methyl, indanofan, indaziflam, metam, methylbromide, MSMA, oxaziclomefone, pyributicarb, triaziflam, tridiphane and 6-chloro-3-(2-cyclopropyl-6-methylphenoxy)-4-pyridazinol (CAS 499223-49-3) and its salts and esters.

**4.** A composition according to any of claims 1 to 3, wherein the herbicide B is selected from the group consisting of b2, b3, b4, b5, b6, b9 and b10.

**5.** A composition according to any of claims 1 to 4, wherein the safener C is selected from the group consisting of benoxacor, cloquintocet, cyometrinil, cyprosulfamide, dichlormid, dicyclonon, dietholate, fenchlorazole, fenclorim, flurazole, fluxofenim, furilazole, isoxadifen, mefenpyr, mephenate, naphthalic anhydride, oxabetrinil, 4-(dichloroacetyl)-1-oxa-4-azaspiro[4.5]decane (MON4660, CAS 71526-07-3) and 2,2,5-trimethyl-3-(dichloroacetyl)-1,3-oxazolidine (R-29148, CAS 52836-31-4).

**6.** A composition as claimed in any of claims 1 to 5, wherein the weight ratio of the crystalline form B to herbicide B is in the range of from 1:500 to 500:1.

**7.** A composition as claimed in any of claims 1 to 6, wherein the weight ratio of the crystalline form B to safener C is in the range of 1:500 to 500:1.

**8.** An agrochemical composition comprising a composition as claimed in any of claims 1 to 7 and auxiliaries customary for formulating crop protection agents.

**9.** A process for the preparation of agrochemical compositions, which comprises mixing a herbicidal active amount of at least one composition as claimed in any of claims 1 to 8 and auxiliaries customary for formulating crop protection agents.

**10.** A method for controlling unwanted vegetation, wherein an herbicidal effective amount of a composition according to any of claims 1 to 8 is allowed to act on plants, their habitat or seed.

**11.** The use of the compositions as claimed in any of claims 1 to 8 as herbicides or for the desiccation/defoliation of plants.

**12.** The use as claimed in claim 11 for controlling unwanted vegetation in crops.

Figure 1

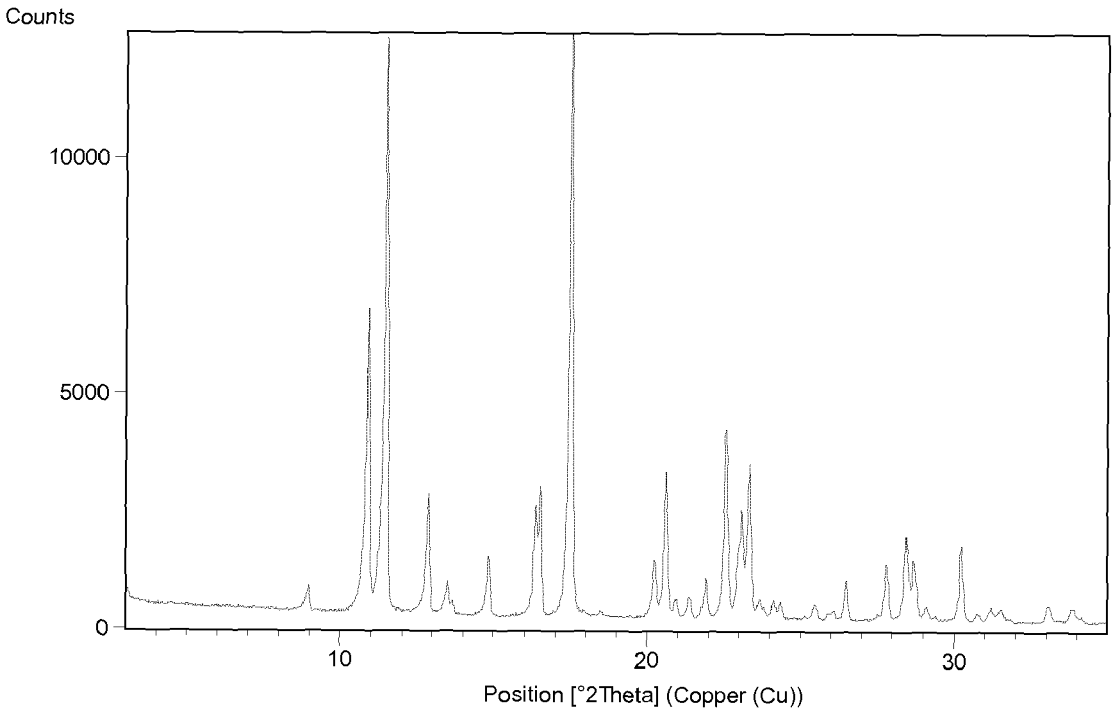


Figure 2

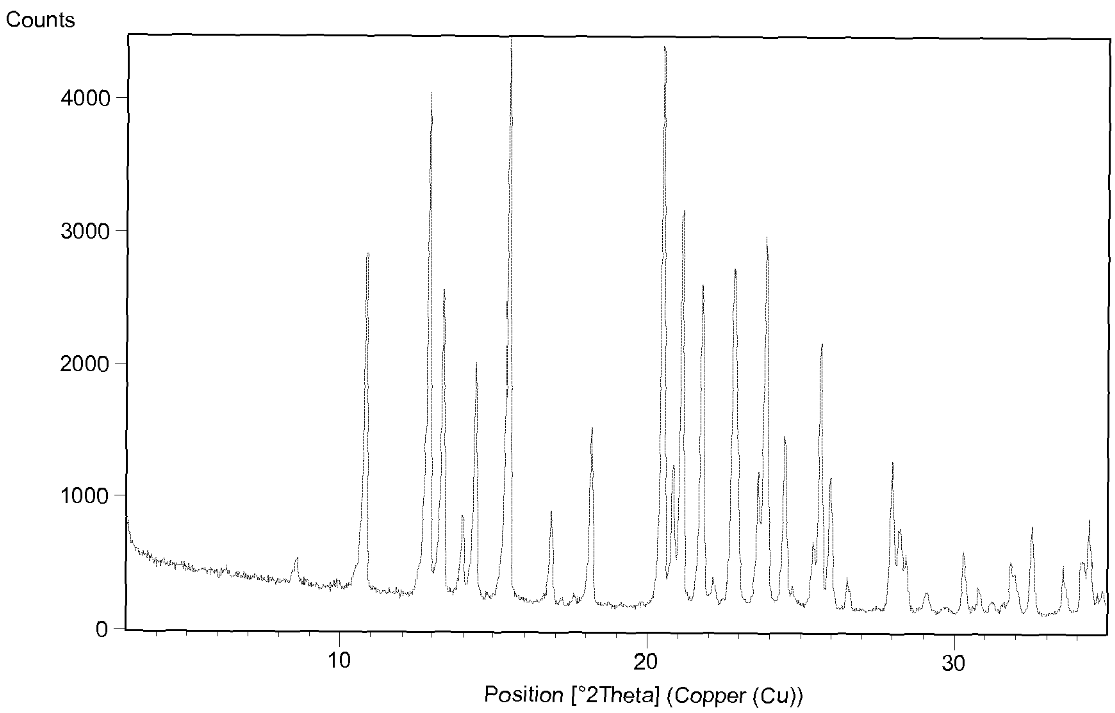

Figure 3

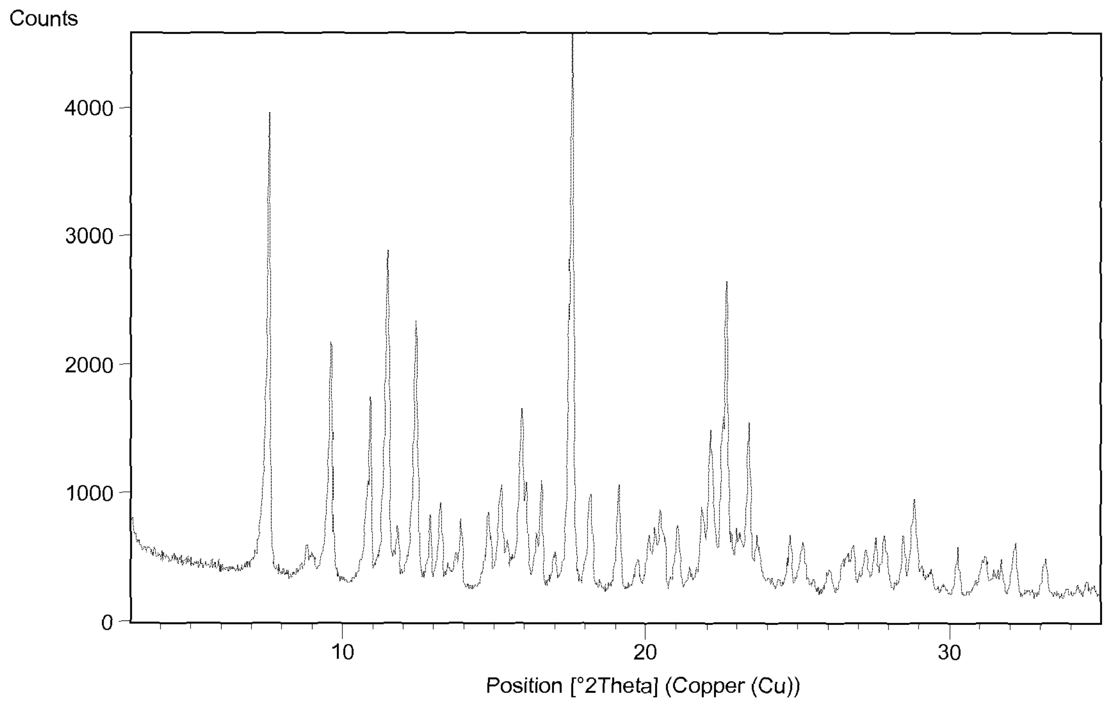
Counts
4000
3000
2000
1000
0
10
20
30
Position [°2Theta] (Copper (Cu))

Europäisches Patentamt
European Patent Office
Office européen des brevets

# EUROPEAN SEARCH REPORT

Application Number
EP 14 19 0204

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | WO 2013/174694 A1 (BASF SE [DE]) 28 November 2013 (2013-11-28)<br>* page 2, line 28 - page 3, line 30 *<br>* page 13, line 13 - line 32 *<br>* page 14, line 31 - line 36 *<br>* page 23, line 1 - line 5 *<br>----- | 1-12 | INV.<br>A01N43/84<br>C07D413/04 |
| X | WO 2010/145992 A1 (BASF SE [DE]; WITSCHEL MATTHIAS [DE]; NEWTON TREVOR WILLIAM [DE]; SEIT) 23 December 2010 (2010-12-23)<br>* page 3, line 9 - page 4, line 20 *<br>* page 11, line 1 - line 4 *<br>* examples 2.1-2.10 *<br>----- | 1-12 | |
| A | WO 2013/092858 A1 (BASF SE [DE]) 27 June 2013 (2013-06-27)<br>* page 7, line 3 - line 8 *<br>----- | 1-12 | |
| A | WO 2011/018486 A2 (BASF SE [DE]; PARRA RAPADO LILIANA [DE]; MOBERG WILLIAM KARL [DE]; NEW) 17 February 2011 (2011-02-17)<br>* page 2, line 11 - page 3, line 23 *<br>----- | 1-12 | TECHNICAL FIELDS SEARCHED (IPC)<br>A01N<br>C07D |

The present search report has been drawn up for all claims

1

| Place of search | Date of completion of the search | Examiner |
|---|---|---|
| Munich | 3 March 2015 | Staber, Brigitte |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 19 0204

This annex lists the patent family members relating to the patent documents cited in the above-mentioned European search report.
The members are as contained in the European Patent Office EDP file on
The European Patent Office is in no way liable for these particulars which are merely given for the purpose of information.

03-03-2015

| Patent document cited in search report | Publication date | Patent family member(s) | Publication date |
|---|---|---|---|
| WO 2013174694 A1 | 28-11-2013 | AU 2013265472 A1 | 04-12-2014 |
| | | CA 2871345 A1 | 28-11-2013 |
| | | CN 104334550 A | 04-02-2015 |
| | | WO 2013174694 A1 | 28-11-2013 |
| WO 2010145992 A1 | 23-12-2010 | AR 077191 A1 | 10-08-2011 |
| | | AU 2010261874 A1 | 19-01-2012 |
| | | CA 2763938 A1 | 23-12-2010 |
| | | CN 102459205 A | 16-05-2012 |
| | | CR 20110667 A | 15-06-2012 |
| | | DK 2443102 T3 | 08-07-2013 |
| | | EA 201200032 A1 | 30-07-2012 |
| | | EP 2443102 A1 | 25-04-2012 |
| | | ES 2417312 T3 | 07-08-2013 |
| | | HR P20130420 T1 | 30-06-2013 |
| | | JP 2012530098 A | 29-11-2012 |
| | | KR 20120046180 A | 09-05-2012 |
| | | NZ 597619 A | 28-06-2013 |
| | | PT 2443102 E | 05-06-2013 |
| | | SI 2443102 T1 | 30-08-2013 |
| | | TW 201111372 A | 01-04-2011 |
| | | US 2012100991 A1 | 26-04-2012 |
| | | US 2014243522 A1 | 28-08-2014 |
| | | UY 32726 A | 31-12-2010 |
| | | WO 2010145992 A1 | 23-12-2010 |
| WO 2013092858 A1 | 27-06-2013 | CN 104011044 A | 27-08-2014 |
| | | EP 2794599 A1 | 29-10-2014 |
| | | US 2014316131 A1 | 23-10-2014 |
| | | WO 2013092858 A1 | 27-06-2013 |
| WO 2011018486 A2 | 17-02-2011 | AR 077926 A1 | 05-10-2011 |
| | | TW 201109321 A | 16-03-2011 |
| | | UY 32838 A | 31-01-2011 |
| | | WO 2011018486 A2 | 17-02-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010145992 A
- WO 2006024820 A
- WO 2006037945 A
- WO 2007071900 A
- WO 2007096576 A
- WO 9626202 A
- WO 9741116 A
- WO 9741117 A
- WO 9741118 A
- WO 0183459 A
- US 6222100 B
- WO 0182685 A
- WO 0026390 A
- WO 9741218 A
- WO 9802526 A
- WO 9802527 A
- WO 04106529 A
- WO 0520673 A
- WO 0314357 A
- WO 0313225 A
- WO 0314356 A
- WO 0416073 A
- WO 00026390 A
- WO 98002526 A
- WO 03014357 A
- WO 9200377 A
- EP 242236 A
- EP 242246 A
- US 5559024 A
- WO 02015701 A
- EP 374753 A
- WO 93007278 A
- WO 9534656 A
- EP 427529 A
- EP 451878 A
- WO 0318810 A
- WO 0352073 A
- WO 03018810 A
- EP 392225 A


### Non-patent literature cited in the description


- The Compendium of Pesticide Common Names. Farm Chemicals Handbook. Meister Publishing Company, 2000, vol. 86
- **B. HOCK ; C. FEDTKE ; R. R. SCHMIDT.** Herbizide. Georg Thieme Verlag, 1995
- **W. H. AHRENS.** Herbicide Handbook. Weed Science Society of America, 1994
- **K. K. HATZIOS.** Herbicide Handbook. Weed Science Society of America, 1998
- Modern Crop Protection Compounds. Wiley VCH, 2007, vol. 1
- **G. M. SHEL-DRICK.** *SHELX-97,* 1997